# EUROPÄISCHE PATENTSCHRIFT

(11) **EP 1 852 429 B1**
(45) Veröffentlichungstag und Bekanntmachung des Hinweises auf die Patenterteilung: **15.09.2010**
(21) Anmeldenummer: 07007699.7
(22) Anmeldetag: 16.04.2007
(51) Int. Cl.: C07D 307/77, G02F 1/13357

(54) **Hexahydro-dibenzofuranderivate**
Hexahydro dibenzofuran derivatives
Dérivé d'héxahydro-dibenzofuran

(30) Priorität: 25.04.2006 DE 102006019045
(43) Veröffentlichungstag der Anmeldung: 07.11.2007
(73) Patentinhaber: Merck Patent GmbH, 64293 Darmstadt (DE)
(72) Erfinder: Jansen, Axel, Dr., 64293 Darmstadt (DE); Taugerbeck, Andreas, Dr., 64285 Darmstadt (DE); Klasen-Memmer, Melanie, Dr., 67259 Heuchelheim (DE)

(56) Entgegenhaltungen:
- WO-A-02/055463
- GRANT V H ET AL: "Iridium(III)-catalyzed tandem Claisen rearrangement-intramolecular hydroaryloxylation of aryl allyl ethers to form dihydrobenzofurans" TETRAHEDRON LETTERS, ELSEVIER, AMSTERDAM, NL, Bd. 46, Nr. 8, 21. Februar 2005 (2005-02-21), Seiten 1237-1239, XP004756223 ISSN: 0040-4039

## Beschreibung

Die vorliegende Erfindung betrifft Hexahadro-dibenzofuranderivate, ihre Herstellung, flüssigkristalline Medien enthaltend diese Derivate sowie elektrooptische Anzeigeelemente enthaltend diese flüssigkristallinen Medien. Insbesondere betrifft die Erfindung Hexahadro-dibenzofuranderivate mit negativer dielektrischer Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete sind insbesondere Anzeigedisplays für Uhren und Taschenrechner sowie große Anzeigetafeln, wie sie in Bahnhöfen, Flughäfen und Sportarenen verwendet werden. Weitere Anwendungsgebiete sind Displays von tragbaren Computern und Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante ε_{∥} parallel zu den Moleküllängsachsen größer als die Dielektrizitätskonstante ε_{⊥} senkrecht zu den Moleküllängsachsen, so ist die dielektrische Anisotropie Δε = ε_{∥} - ε_{┴} größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch den Einbau geeigneter polarer Gruppen, wie z.B. von Nitrilgruppen oder Fluor, in die Flüssigkristallmoleküle läßt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Bei den am weitesten verbreiteten TN-Zellen (abgeleitet aus dem Englischen: "twisted nematic", verdrillt nematisch) ist eine nur etwa 5 bis 10 µm dicke flüssigkristalline Schicht zwischen zwei planparallelen Glasplatten angeordnet, auf die jeweils eine elektrisch leitende, transparente Schicht aus Zinnoxid oder Indium-Zinnoxid (ITO) als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so dass sie im spannungsfreien Zustand einheitlich mit der gleichen Orientierung flach oder mit demselben kleinen Anstellwinkel (englisch: "tilt angle") auf der Innenseite der Displayfläche aufliegen. Auf der Außenseite des Displays sind zwei Polarisationsfolien, die nur linear polarisiertes Licht ein- und austreten lassen, in einer bestimmten Anordnung aufgebracht.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen zu erreichen. Schwierigkeiten bereitet jedoch noch die starke Blickwinkelabhängigkeit bei Flüssigkristallanzeigen, wie sie beispielsweise für Laptops verwendet werden. Die beste Abbildungsqualität läßt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In der WO 02/055463 A1 werden unter anderem 3-monosubstituierte und 3,7-disubstituierte 4,6-Difluordibenzofurane und -thiophene offenbart, ohne dass physikalische oder elektrooptische Eigenschaften präzisiert werden.

Eine Aufgabe der vorliegenden Erfindung ist es daher, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem zu nennen ein hoher Klärpunkt, eine geringe Rotationsviskosität, eine optische Anisotropie im Anwendungsintervall, sowie die Eigenschaften, die zur Erzielung von Mischungen mit den gewünschten flüssigkristallinen Phasen über einen breiten Temperaturbereich dienen.

Diese Aufgabe wird erfindungsgemäß gelöst durch Verbindungen der allgemeinen Formel I 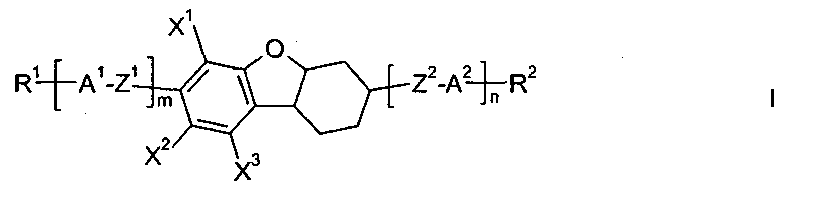 worin
- m und n: jeweils unabhängig voneinander 0, 1 oder 2 sind,
- X¹, X² und X³: jeweils unabhängig voneinander H, Halogen, CN oder CF₃ bedeuten, bevorzugt H, F, Cl, CN oder CF₃,
- A¹ und A²: jeweils unabhängig voneinander 1,4-Phenylen, worin =CH- ein- oder zweimal durch =N- ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann,
1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F und/oder -Cl substituiert sein können,
Bicyclo[1.1.1]pentan-1,3-diyl, Bicyclo[2.2.2]octan-1,4-diyl, Spiro[3.3]heptan-2,6-diyl, Tetrahydropyran-2,5-diyl oder 1,3-Dioxan-2,5-diyl,
bedeutet;
- Z¹ und Z²: jeweils unabhängig voneinander eine Einfachbindung, -CF₂O-, -OCF₂-, -CH₂CH₂-, -CF₂CF₂-, -CF₂CH₂-, -CH₂CF₂-, -CHF-CHF-, -(CO)O-, -O(CO)-, -CH₂O-, -OCH₂-, -CF=CH-, -CH=CF-, -CF=CF-, -CH=CH- oder -C≡C- bedeuten;
- R¹ und R²: unabhängig voneinander, Wasserstoff, einen unsubstituierten, einen einfach mit -CN oder -CF₃ oder einen einfach oder mehrfach mit -F, -Cl, -Br und/oder -I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -(CO)O-, -O(CO)- oder -O(CO)O- so ersetzt sein können, dass Heteroatome nicht direkt verknüpft sind, -F, -Cl, -Br, -I, -CN, -SCN, -NCS oder -SF₅ bedeuten;
wobei
- A¹,A²,Z¹,Z²: jeweils die gleiche oder unterschiedliche Bedeutungen haben können, wenn m beziehungsweise n größer als 1 sind, und,
wobei
für den Fall, dass zugleich n = 0, m = 0 und X¹, X² und X³ ungleich F sind, R¹ und R² dann nicht gleichzeitig H bedeuten.

Eine derartige Verbindung ist in Tetrahedron Letters, 46(8), 2005, Seiten 1237-1239 als Verbindung 19 offenbart.

Die Verbindungen besitzen überwiegend ein negatives Δε und eignen sich daher insbesondere für eine Verwendung in VA-TFT-Displays. Vorzugsweise besitzen die erfindungsgemäßen Verbindungen ein Δε < -2 und besonders bevorzugt ein Δε < -4. Sie zeigen eine sehr gute Verträglichkeit mit den üblichen, in Flüssigkristallmischungen für Displays verwendeten Substanzen.

Ferner weisen die erfindungsgemäßen Verbindungen der Formel I insbesondere für die Verwendung in VA-TFT-Displays geeignete Werte der optischen Anisotropie Δn auf. Bevorzugt besitzen die erfindungsgemäßen Verbindungen ein Δn von größer als 0,05 und kleiner als 0,40.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die Verbindungen bzw. flüssigkristalline Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die Rotationsviskositäten der Verbindungen sind vorteilhafterweise klein, besonders für m+n = 0.

Weiterhin ist es bevorzugt, dass ein oder zwei der Reste X¹, X² und X³ Cl oder F, insbesondere Fluor, bedeutet. Besonders bevorzugt bedeuten X² und X³ H und X¹ keinen Wasserstoff. Alternativ ist X¹ bevorzugt H und wenigstens einer der Substituenten X² und X³ kein Wasserstoff. Besonders bevorzugt sind X¹, X² und X³ unabhängig voneinander H oder F. In einer besonders bevorzugten Ausführungsform ist daher X¹ Fluor und X² und X³ Wasserstoff. Alternativ dazu ist besonders bevorzugt X¹ H, X² F und X³ H oder F.

Für den Fall, dass der Rest R¹ ein Fluoratom oder fluoriertes Alkyl bedeutet, insbesondere wenn gleichzeitig m 0 bedeutet, dann sind unter der Formel I auch Verbindungen umfasst, die eine insgesamt positive dielektrische Anisotropie besitzen können. Solche Verbindungen eignen sich dann für dielektrisch positive Flüssigkristallmischungen, die in Displays wie z. B. vom Typ des TN-TFT oder IPS ('in-plane-switching') verwendet werden. Die Anforderungen an die übrigen physikalischen Parameter, wie z.B. der Viskosität, sind über die meisten Anwendungen hinweg weitgehend kongruent. Die genannten Verbindungen sind also gleichermaßen für diese Zwecke geeignet, weil sie günstige Werte für die Parameter wie Rotationsviskosität, Δn, etc, haben und sich für den Aufbau flüssigkristalliner Mischungen eingnen.

A¹ und A² sind bevorzugt und unabhängig voneinander ein gegebenenfalls substituiertes 1,4-Phenylen, ein gegebenenfalls substituiertes 1,4-Cyclohexylen, worin -CH₂- ein- oder zweimal durch -O- ersetzt sein kann, oder ein gegebenenfalls substituiertes 1,4-Cyclohexenylen. Wenn n oder m 2 sind, können die Ringe A¹ beziehungsweise A² die gleichen oder unterschiedliche Bedeutungen annehmen.

Besonders bevorzugt sind A¹ und A² unabhängig voneinander 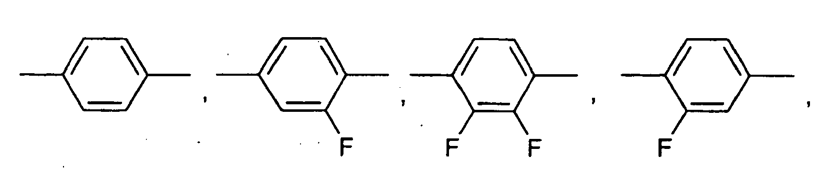 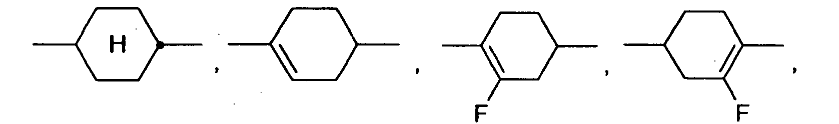 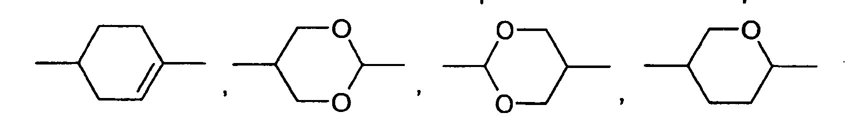 oder

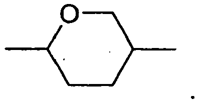

Ganz besonders bevorzugt sind A¹ und A² 1,4-Cyclohexylenringe und/oder gegebenenfalls mit Fluor substituierte 1,4-Phenylenringe.

Bevorzugt sind Z¹ und Z² unabhängig voneinander eine Einfachbindung, -CH₂O- -OCH₂-, -CF₂O-, -OCF₂-, -CF₂CF₂-, -CH=CH-, -CF=CH-, -CH=CF- oder -CF=CF-, besonders bevorzugt unabhängig voneinander eine Einfachbindung, -CF₂O-, -OCF₂-, -CF₂CF₂-, -CH=CH-, -CF=CH-, -CH=CF-, -CH₂O-, -OCH₂- oder -CF=CF-. Ganz besonders bevorzugt sind Z¹ und Z² unabhängig voneinander eine Einfachbindung, -CF₂O-, -OCF₂-, -CH₂O-, -OCH₂- oder -CF=CF-, insbesondere eine Einfachbindung.

Sofern R¹ und R² in Formel I jeweils unabhängig voneinander einen Alkanylrest und/oder einen Alkoxyrest (Alkyloxyrest) mit 1 bis 15 C-Atomen darstellen, sind diese geradkettig oder verzweigt. Vorzugsweise ist jeder dieser Reste geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R¹ und R² in Formel I können jeweils unabhängig voneinander auch ein Oxaalkylrest sein, d.h. ein Alkanylrest, in dem mindestens eine der nichtterminalen CH₂-Gruppen des Alkanylrests durch -O- ersetzt ist, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl. In entsprechender Weise können R¹ und R² in Formel I auch unabhängig voneinander Thioalkanyl- bzw. Sulfonalkanylreste sein, d.h. Alkanylreste, in denen eine CH₂-Gruppe durch -S- beziehungsweise -SO₂- ersetzt ist.

R¹ und R² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im allgemeinen sind die jeweiligen E-Isomeren bevorzugt.

In gleicher Weise wie bei einem Alkanylrest kann auch bei einem Alkenylrest wenigstens eine der CH₂-Gruppen durch Sauerstoff, Schwefel oder -SO₂- ersetzt sein. Bei dem Ersatz durch -O- liegt dann ein Alkenyloxyrest (mit entständigem Sauerstoff) beziehungsweise ein Oxaalkenylrest (mit nicht-terminalem Sauerstoff) vor.

R¹ und R² in Formel I können unabhängig voneinander auch ein Alkinylrest mit 2 bis 15 C-Atomen sein, der geradkettig oder verzweigt ist und wenigstens eine C-C-Dreifachbindung aufweist.

R¹ und R² in Formel I können jeweils unabhängig voneinander ein Alkanylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser Rest geradkettig und hat 2 bis 6 C-Atome. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxy-methyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)-propyl oder 4-(Methoxycarbonyl)butyl. Ferner kann ein Alkanylrest auch eine -O-CO-O-Einheit aufweisen. Auch der Ersatz einer CH₂-Gruppe durch lediglich eine -CO-Gruppe (Carbonylfunktion) ist möglich.

R¹ und R² in Formel I können jeweils unabhängig voneinander ein Alkenylrest mit 2 bis 15 C-Atomen sein, in dem eine CH₂-Gruppe bevorzugt in Nachbarschaft zu einer unsubstituierten oder substituierten -C=C-Einheit durch -CO-, -CO-O-, -O-CO- oder -O-CO-O- ersetzt ist, wobei dieser Rest geradkettig oder verzweigt sein kann. Vorzugsweise ist der Rest geradkettig und hat 4 bis 13 C-Atome. Besonders bevorzugt sind dabei Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl oder 8-Methacryloyloxyoctyl. Entsprechend kann auch in einem Alkinylrest in Nachbarschaft zu einer substituierten -C≡C-Einheit eine CH₂-Gruppe durch -CO-, -CO-O-, -O-CO- oder -O-CO-O- ersetzt sein.

R¹ und R² in Formel I können jeweils unabhängig voneinander ein einfach durch -CN oder -CF₃ substituierter Alkanylrest oder Alkoxyrest mit 1 bis 15 C-Atomen oder ein Alkenylrest oder Alkinylrest mit 2 bis 15 C-Atomen sein, wobei diese vorzugsweise geradkettig sind. Die Substitution durch -CN oder -CF₃ ist in beliebiger Position möglich.

R¹ und R² in Formel I können jeweils unabhängig voneinander ein Alkanylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R¹ und R² in Formel I können jeweils unabhängig voneinander ein einfach oder mehrfach mit F, Cl, Br und/oder I substituierter Alkanylrest oder Alkoxyrest mit 1 bis 15 C-Atomen oder Alkenylrest oder Alkinylrest mit 2 bis 15 C-Atomen sein, wobei diese Reste vorzugsweise geradkettig sind und Halogen vorzugsweise -F und/oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor- oder Chlorsubstituent in beliebiger Position sein, vorzugsweise ist er in ω-Position.

R¹ und R² in Formel I können auch jeweils unabhängig voneinander -F, -Cl, -Br, -I, -CN, -SCN, -NCS oder -SF₅ sein. In diesem Fall erhöht sich die dielekrische Anisotropie hin zu positiveren Werten. Für ganz besonders stark negative dielektrische Anisotropien sind diese Substituenten nicht auszuwählen. Für hohes Δε sind sie allerdings bevorzugt.

Besonders bevorzugt sind R¹ und R² unabhängig voneinander in der allgemeinen Formel I Wasserstoff oder ein Alkanylrest, Alkoxyrest oder Alkenylrest mit 1 bis 7 bzw. 2 bis 7 C-Atomen, wobei jeder dieser Reste bevorzugt unsubstituiert oder mit Halogen einfach oder mehrfach substituiert ist.
Ganz besonders bevorzugt sind R¹ und R² unabhängig voneinander Wasserstoff oder ein Alkanylrest, Alkoxyrest oder Alkenylrest mit 1 bis 7 bzw. 2 bis 7 C-Atomen, wobei jeder dieser Reste bevorzugt unsubstituiert ist.

Für den Fall, dass m = 0 ist, bedeutet R¹ vorzugsweise eine Alkyl- oder Alkoxygruppe, H oder F, besonders bevorzugt eine Alkoxygruppe mit 1-6 C-Atomen.

Für die genannten Substituenten R¹ und R² gilt die anfangs genannte Einschränkung für den Fall, dass m und n 0 sind und X¹, X², X³ ungleich F sind. Weiterhin gilt besonders für den Fall, dass m und n 0 sind, dass R¹ und R² bevorzugt nicht gleichzeitig H bedeuten.

Halogen bedeutet im Zusammenhang der vorliegenden Erfindung Fluor, Chlor, Brom und Iod, insbesondere Fluor oder Chlor.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen. Dieser Rest ist unsubstituiert oder einfach oder mehrfach mit Fluor, Chlor, Brom, Iod, Carboxy, Nitro, -NH₂, -N(Alkanyl)₂ und/oder Cyano substituiert, wobei die Mehrfachsubstitution mit dem gleichen oder mit verschiedenen Substituenten erfolgen kann. Sofern es sich bei diesem Alkylrest um einen gesättigten Rest handelt, wird er auch als "Alkanyl" bezeichnet. Ferner umfasst der Ausdruck "Alkyl" auch unsubstituierte oder entsprechend insbesondere mit -F, -Cl, -Br, -I und/oder -CN oder -CF₃ ein- oder mehrfach gleich oder verschieden substituierte Kohlenwasserstoffreste, in denen eine oder mehrere CH₂-Gruppen derart durch -O- ("Alkoxy", "Oxaalkyl"), -S- ("Thioalkyl"), -SO₂-, -CH=CH- ("Alkenyl"), -C≡C- ("Alkinyl"), -CO-O-, -O-CO- oder -O-CO-O- ersetzt sein können, dass Heteroatome (O, S) in der Kette nicht direkt miteinander verknüpft sind.

Bevorzugte Verbindungen der allgemeinen Formel I weisen insgesamt keine, eine, zwei oder drei Einheiten -Z¹-A¹- und/oder -Z²-A²- auf, das heißt m + n = 0, 1, 2 oder 3 mit m und n jeweils 0, 1, 2 oder 3. Sind zwei oder drei Einheiten -Z¹-A¹- und/oder -Z²-A²- vorhanden, können sie an nur eine Molekülseite (d.h. m = 2 oder 3 und n = 0 oder n = 2 oder 3 und m = 0) oder auch an beide Molekülseiten gebunden sein. Bevorzugt ist m oder n 0. Besonders bevorzugt ist m + n = 0, 1 oder 2 und ganz besonders 0 oder 1.

Bevorzugte Verbindungen der Formel I, für die m + n = 0 gilt, werden durch die folgende Formel dargestellt: 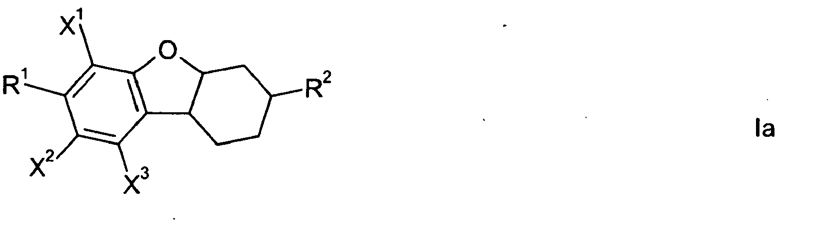 worin
R¹, R², X¹, X² und X³ die gleichen sowie die gleichen bevorzugten Bedeutungen, wie für Formel I oben definiert, besitzen.
Für den Fall, dass R¹ Wasserstoff ist, ist bevorzugt wenigstens eine Gruppe aus X¹, X² und X³ ein Fluorsubstituent, insbesondere X¹.

Bevorzugte Verbindungen der Formel I, für die gilt: m + n = 1, werden durch die folgenden Formeln dargestellt: 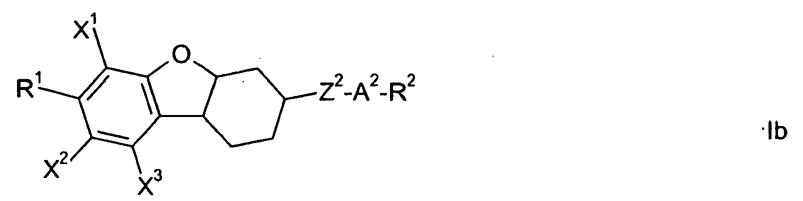 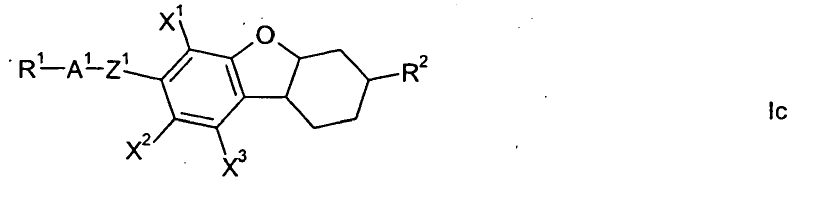 worin
R¹, R², A¹, A², X¹, X², X³, Z¹ und Z² die gleichen sowie die gleichen bevorzugten Bedeutungen, wie für Formel I oben definiert, besitzen.

Bevorzugte Verbindungen der Formel I, für die gilt: m + n = 2, werden durch die folgenden Formeln dargestellt: 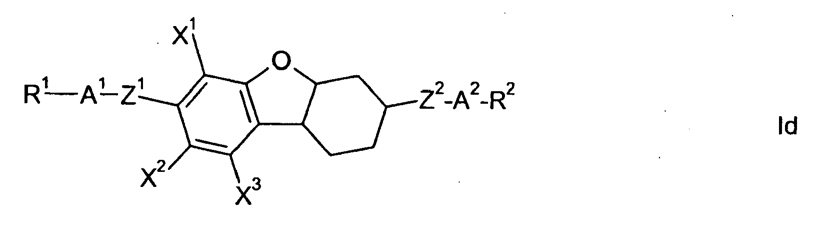 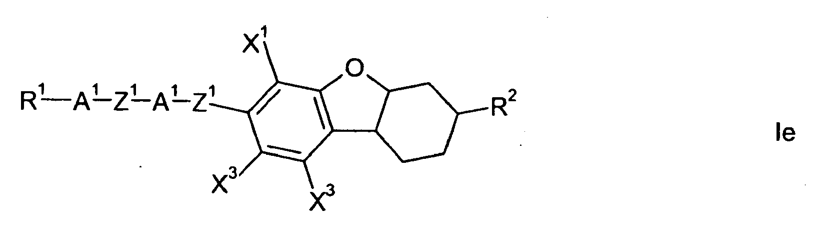 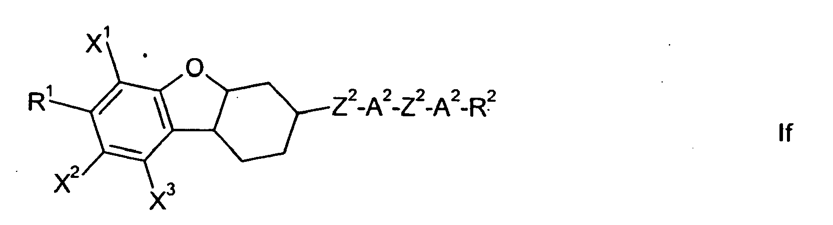 worin
R¹, R², A¹, A², X¹, X², X³, Z¹ und Z² die gleichen sowie die gleichen bevorzugten Bedeutungen, wie für Formel I oben definiert, besitzen.

Soweit A¹, A², Z¹ beziehungsweise Z² in den Formeln Ie und If zweifach vorkommen, können sie jeweils die gleiche oder verschiedene Bedeutungen aufweisen.

Bevorzugte Verbindungen der Formel I, für die gilt: m + n = 3, werden durch die folgenden Formeln dargestellt: 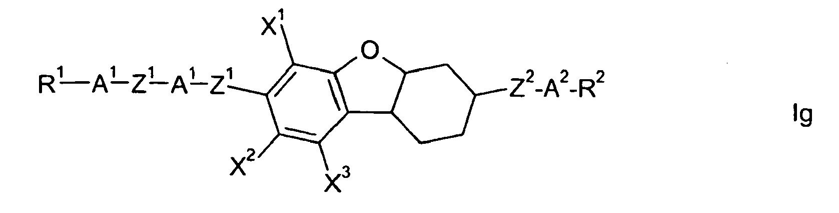 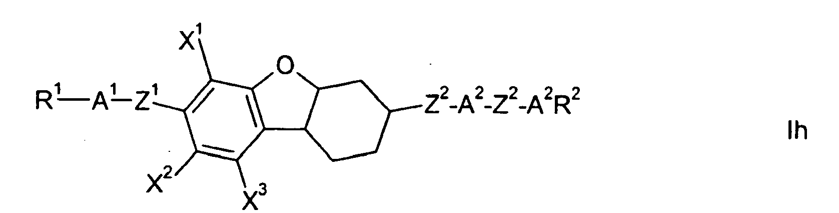 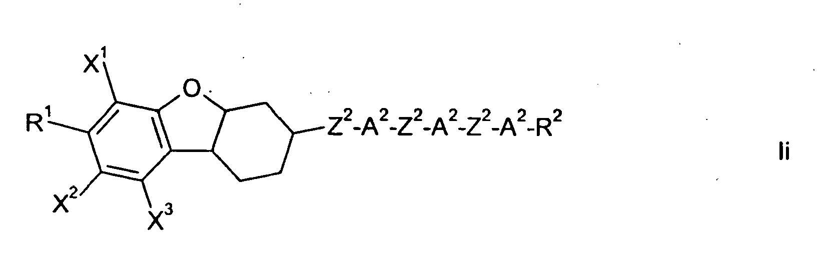 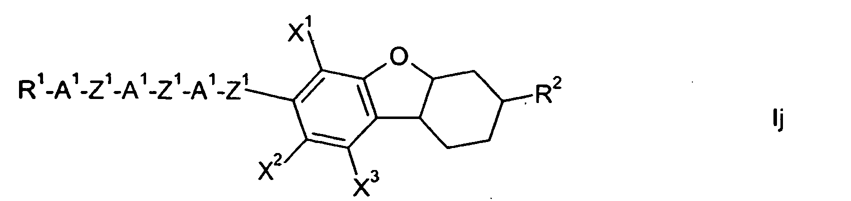 worin
R¹, R², A¹, A², X¹, X², X³, Z¹, Z² und Y die gleichen sowie die gleichen bevorzugten Bedeutungen, wie für Formel I oben definiert, besitzen. Soweit A¹, A², Z¹ beziehungsweise Z² in den Formeln Ig bis Ij mehr als einmal vorkommen, können sie jeweils die gleiche oder verschiedene Bedeutungen aufweisen.
Besonders bevorzugt sind erfindungsgemäße Verbindungen der Formeln Ia, Ib, Ic, Id, Ie und If, insbesondere der Formeln Ia, Ib und Ic.

Ganz besonders bevorzugte Verbindungen der Formel Ia sind die Folgenden: 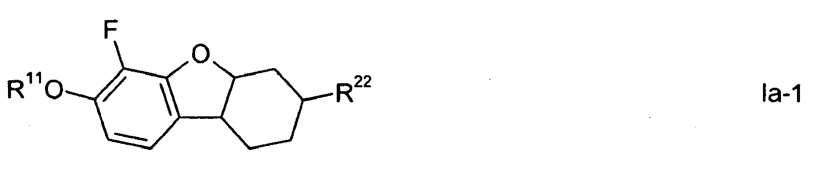 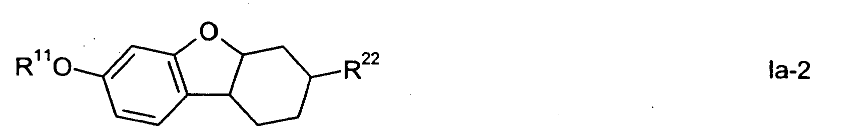 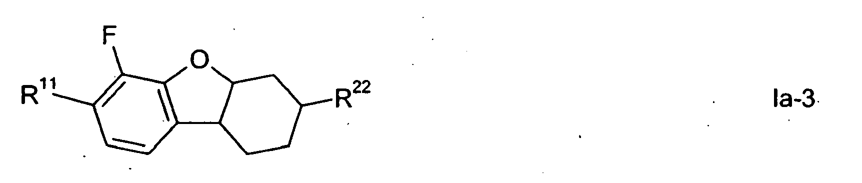 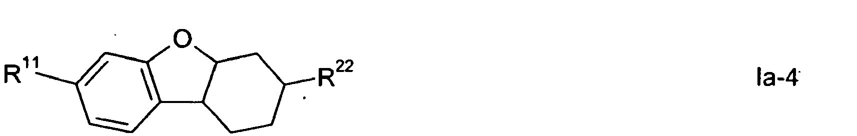 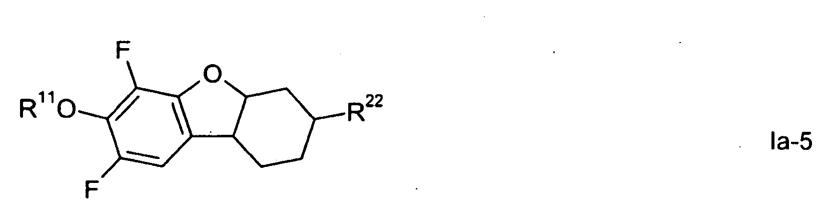 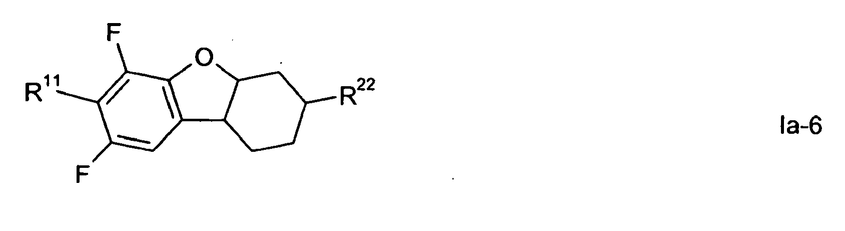 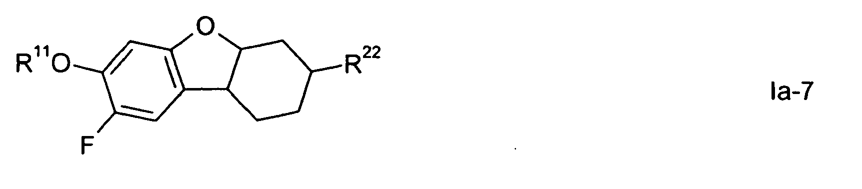 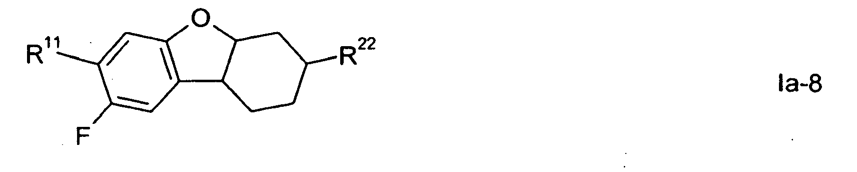 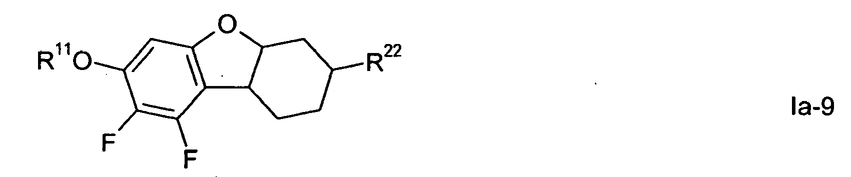 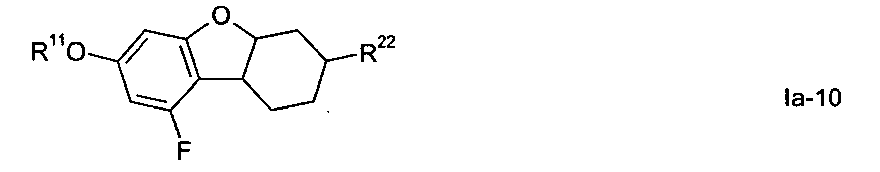 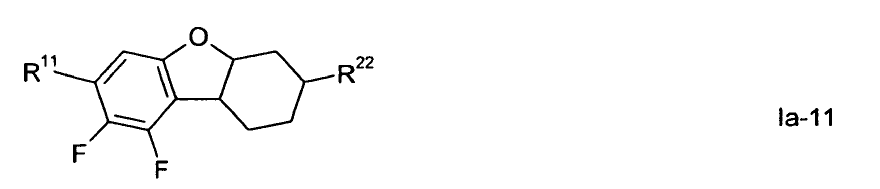 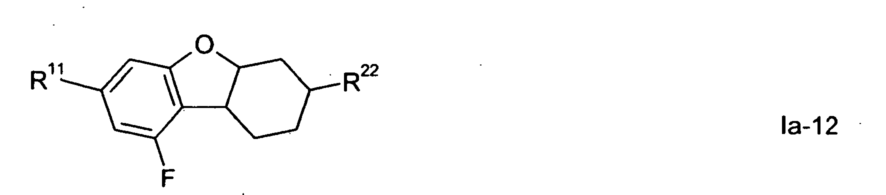 worin
R¹¹, R²² einen Alkylrest mit bis zu 8 C-Atomen bedeutet.

Für den Fall, dass R¹¹ direkt an den Ring gebunden ist, kann R¹¹ bevorzugt auch F bedeuten, wie in den Verbindungen der folgenden Formeln:

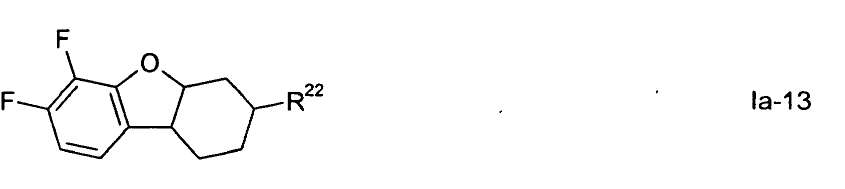

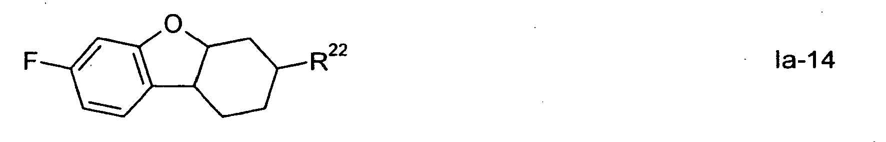

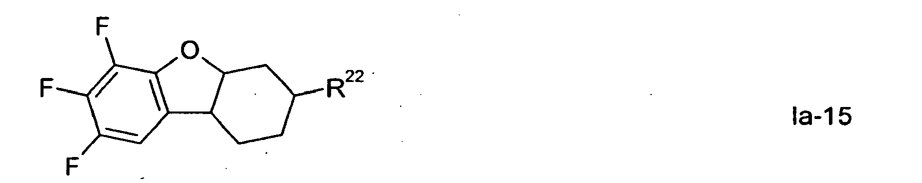

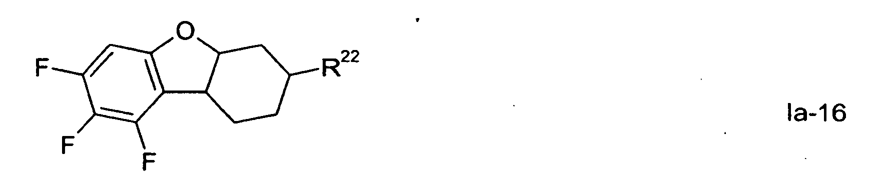

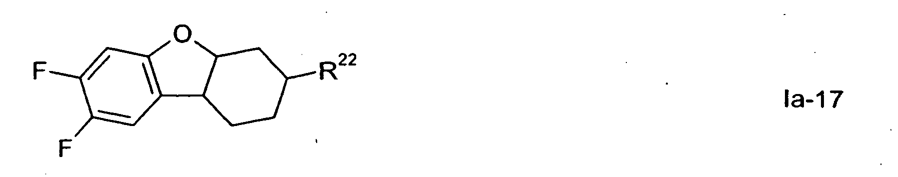

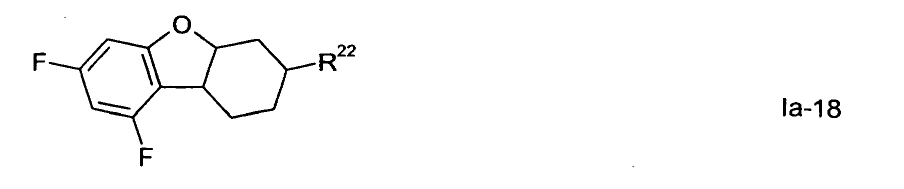

Unter den Verbindungen der Formeln Ia-1 bis Ia-12 sind jene der Formeln Ia-1 bis Ia-4 noch weiter bevorzugt, insbesondere Verbindungen der Formel Ia-1 und Ia-3.

Unter den bevorzugten erfindungsgemäßen Verbindungen der Formel Ib sind die Folgenden besonders bevorzugt: 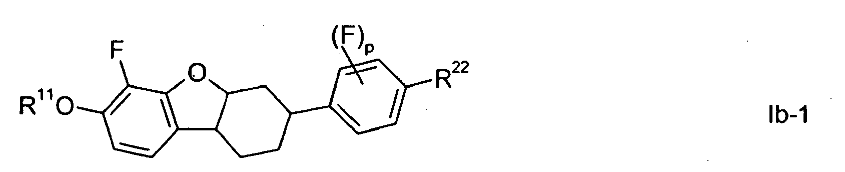 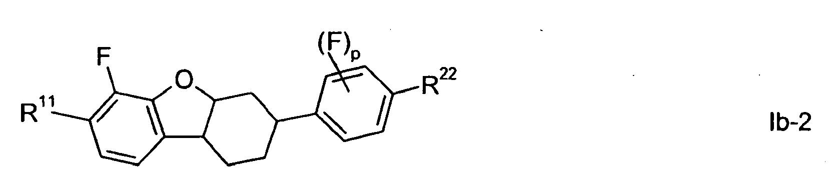 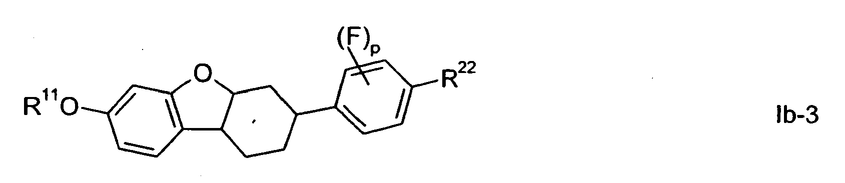 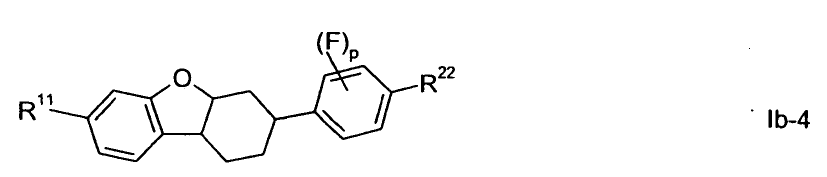 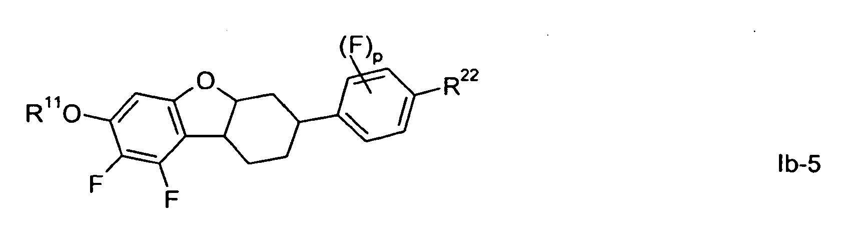 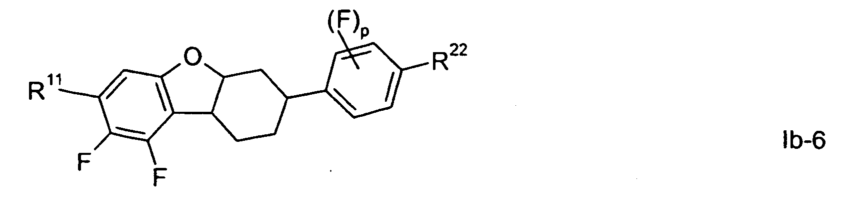 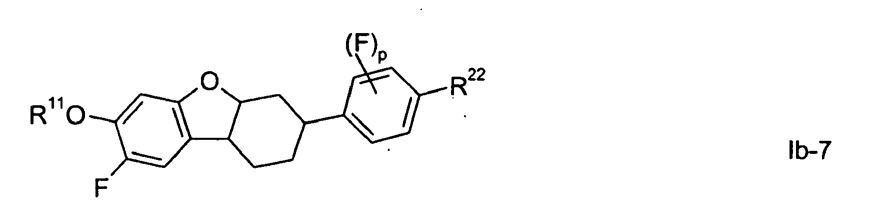 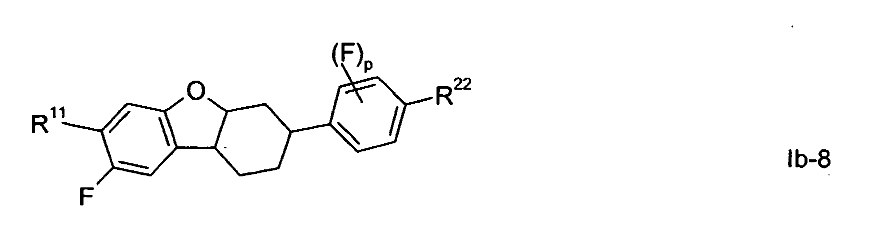 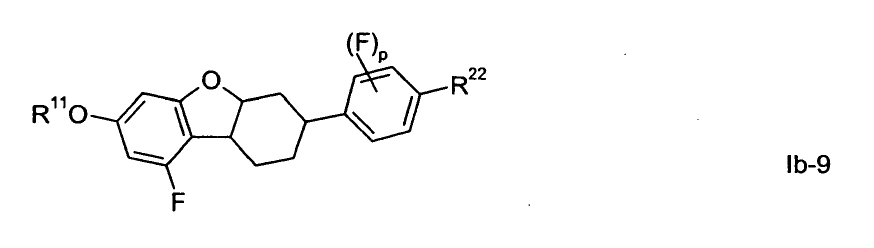 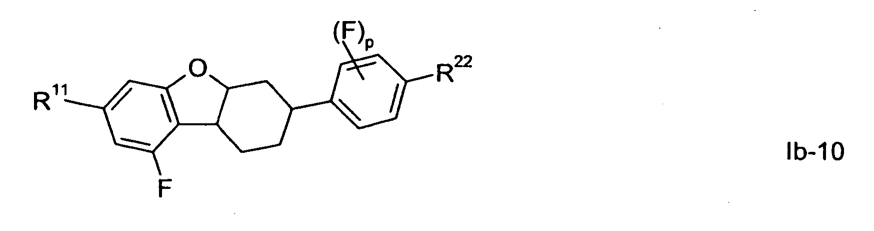 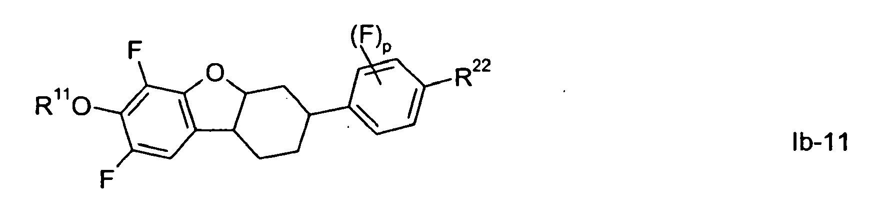 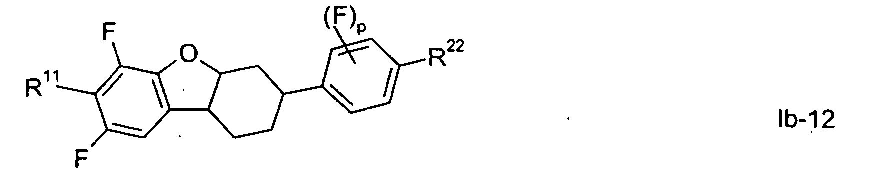 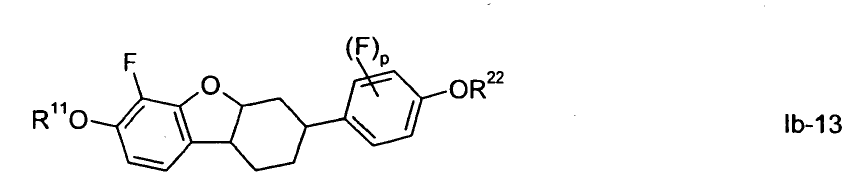 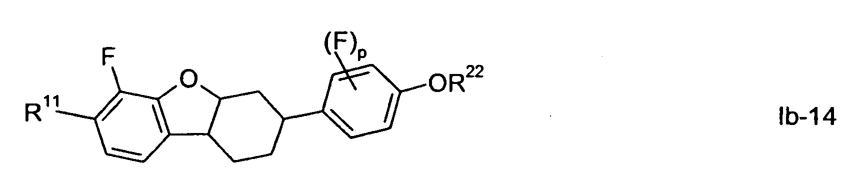 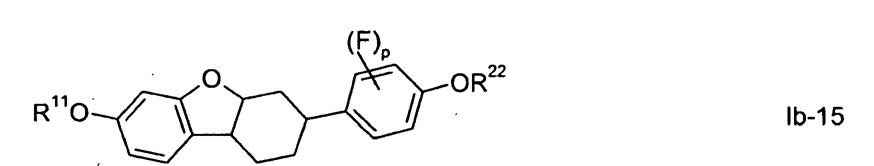 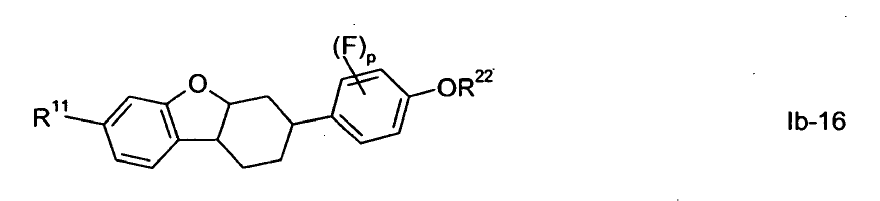 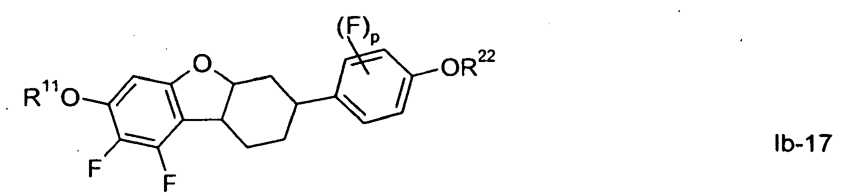 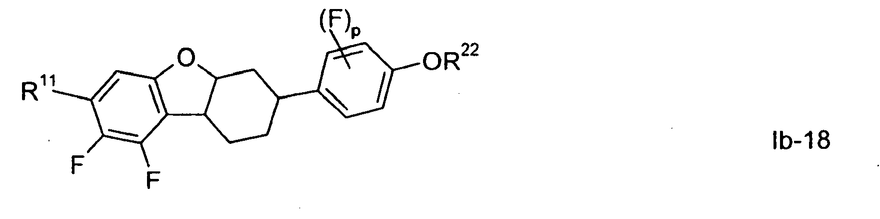 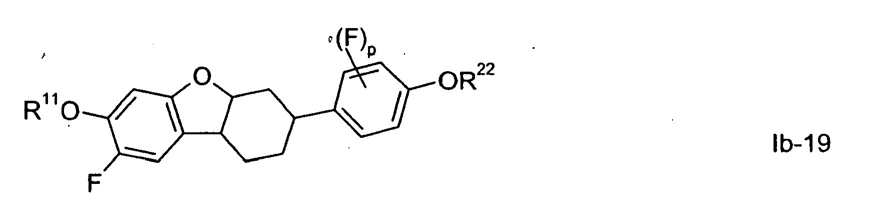 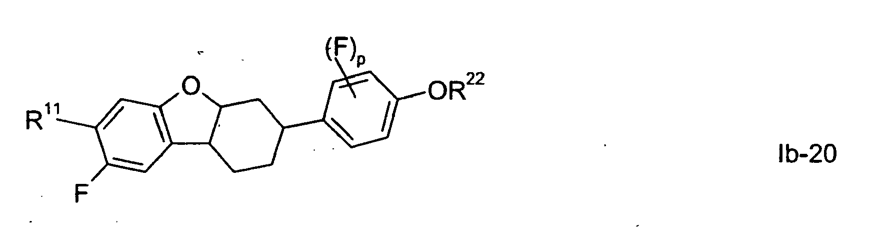 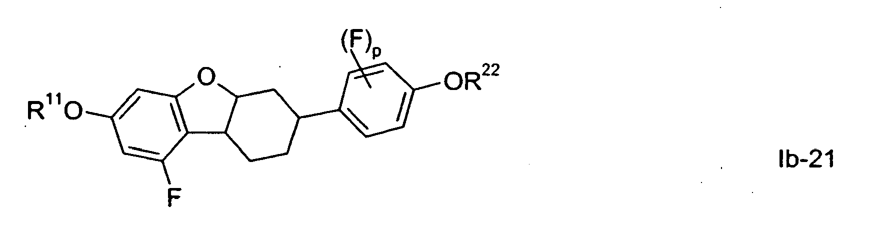 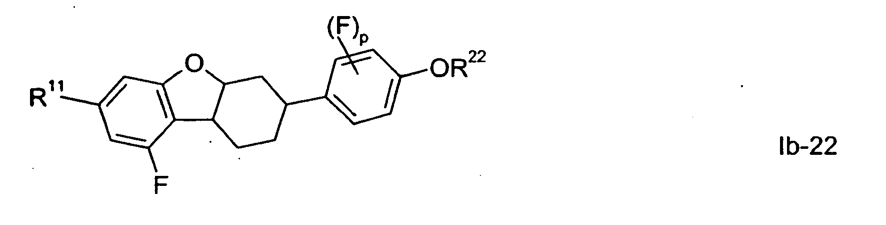 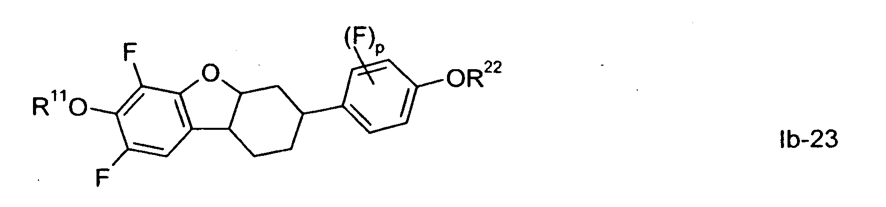 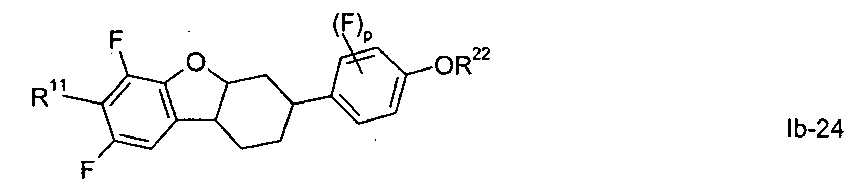 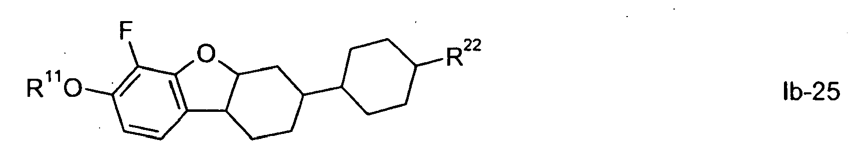 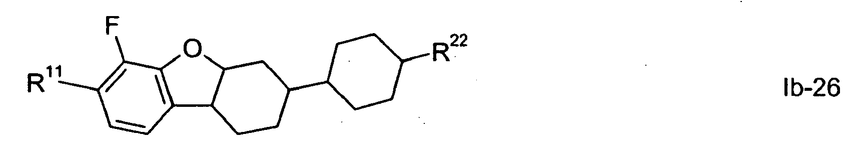 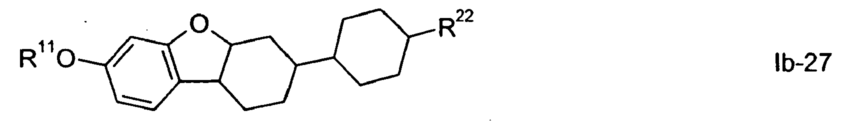 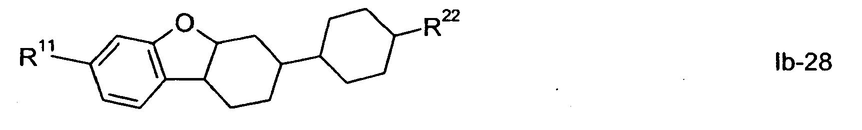 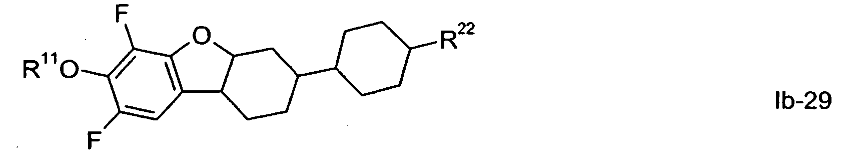 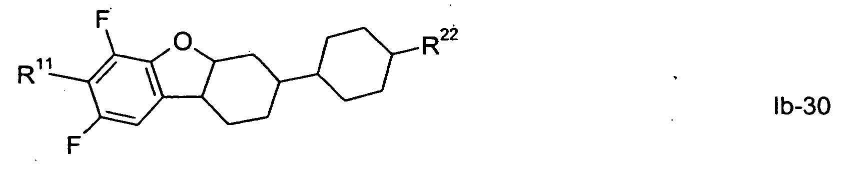 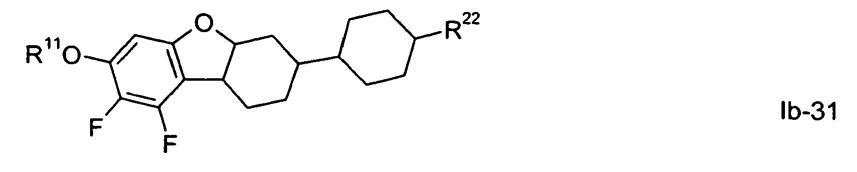 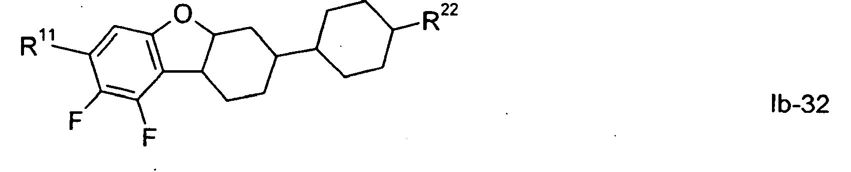 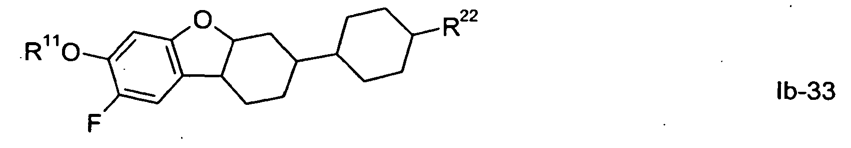 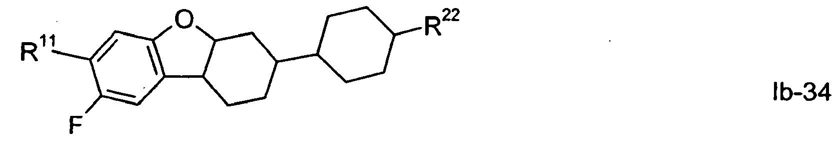 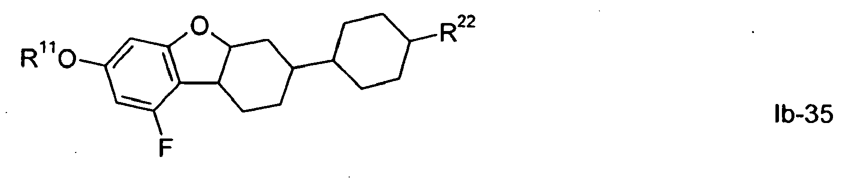 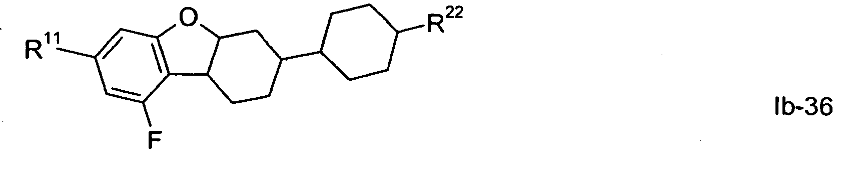 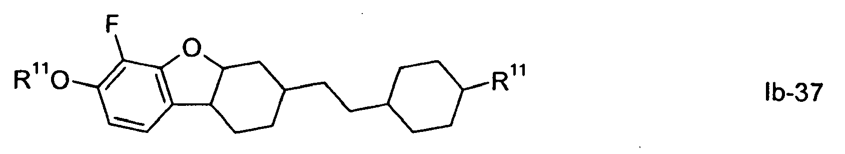 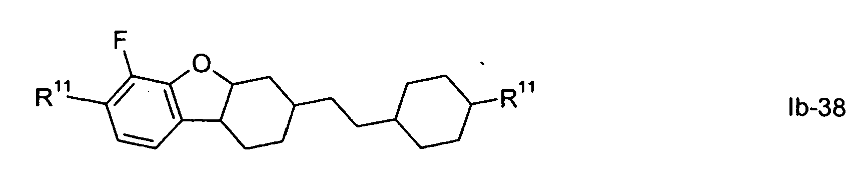 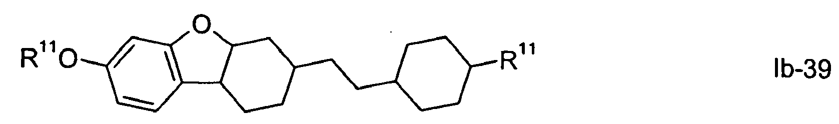 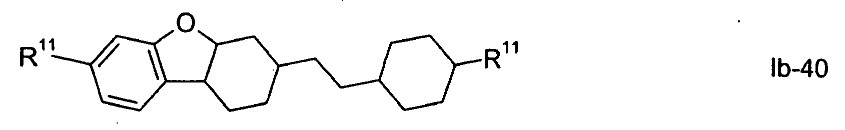 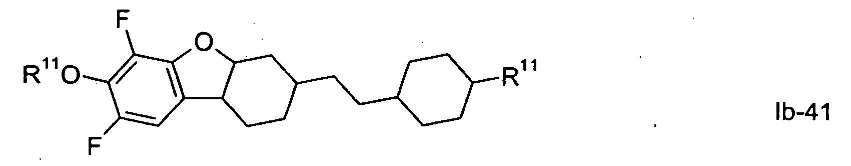 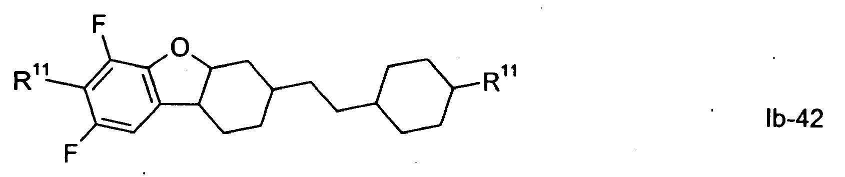 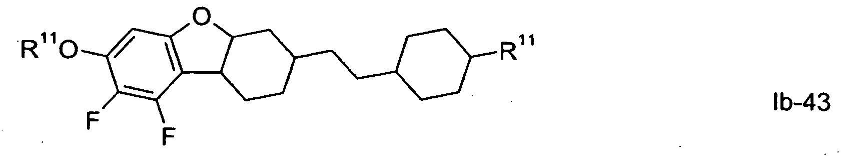 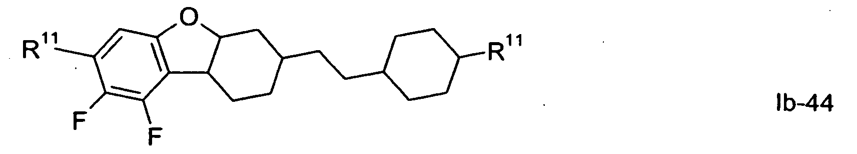 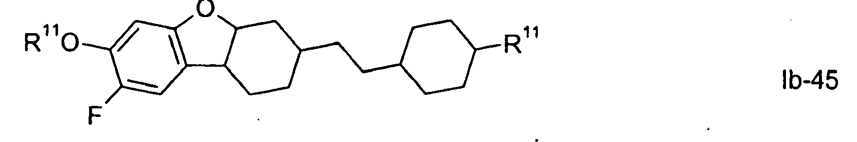 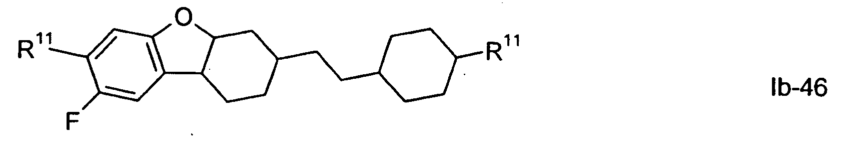 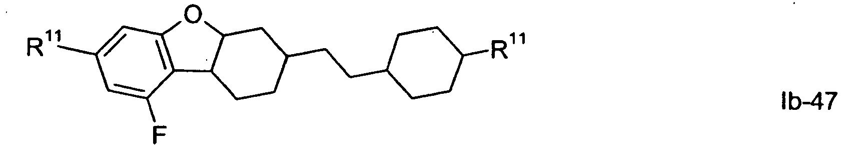 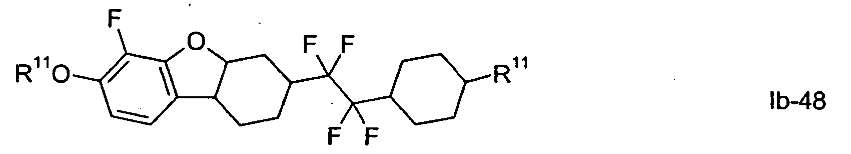 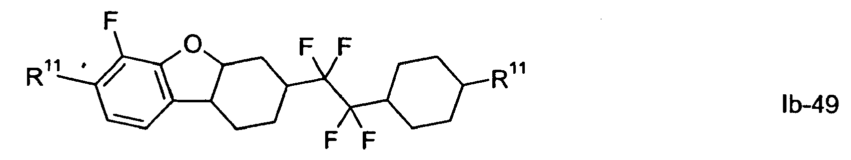 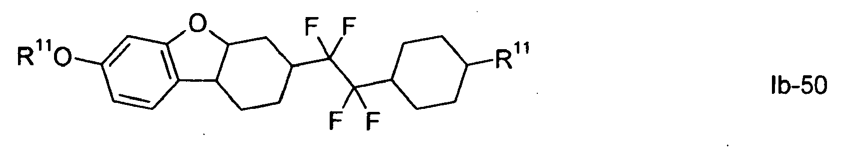 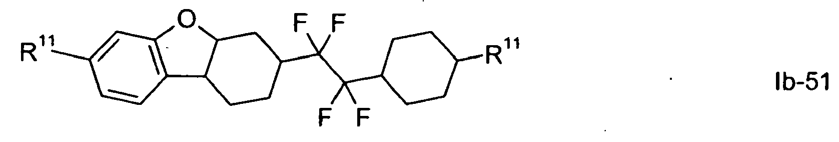 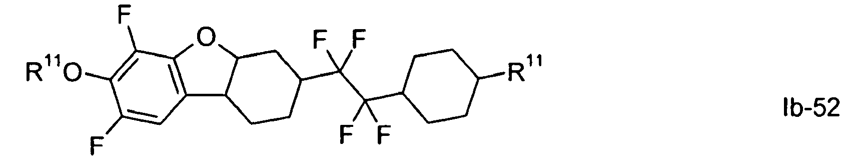 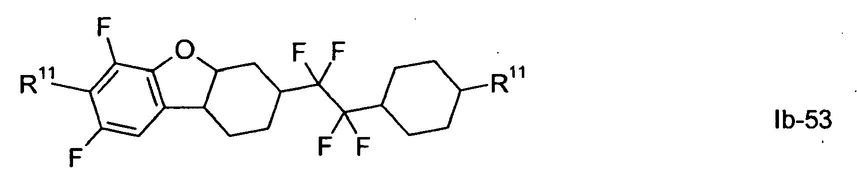 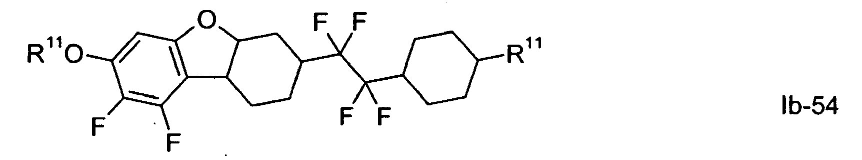 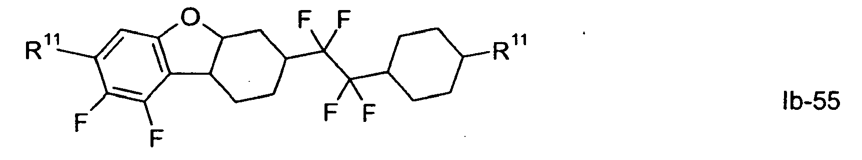 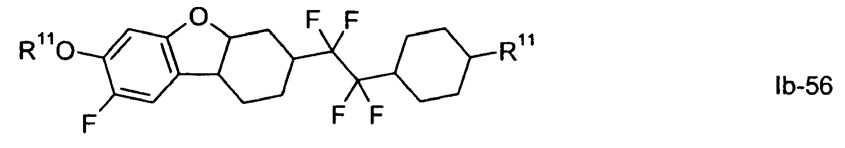 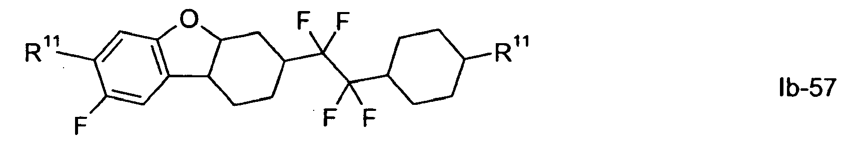 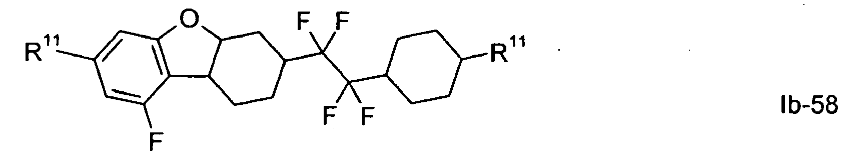 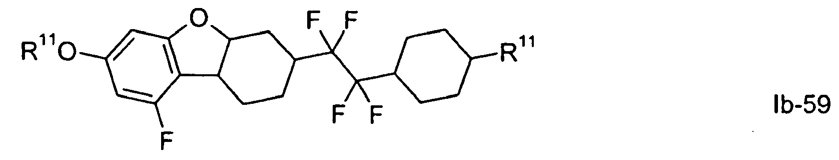 worin
p jeweils unabhängig voneinander 0 bis 4, bevorzugt 0, 1 oder 2, ist und
R¹¹, R²² die gleichen Bedeutungen wie oben definiert besitzen.

Besonders bevorzugt unter den Verbindungen Ib sind solche mit einem Cyclohexylring.

Vor- und nachstehend bedeutet in den Unterformel der Molekülteil 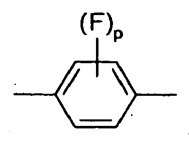 bevorzugt einen Molekülteil der Formeln

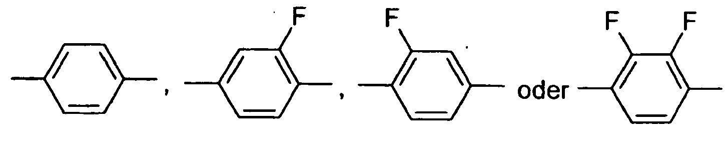

Soweit p in den Formeln mehrmals vorkommt, kann es jeweils die gleiche oder verschiedene Bedeutung aufweisen.

Unter den bevorzugten erfindungsgemäßen Verbindungen der Formel Ic sind die Folgenden besonders bevorzugt: 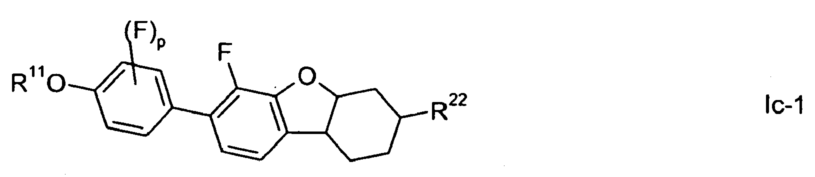 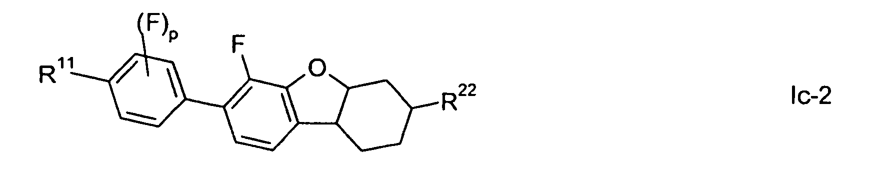 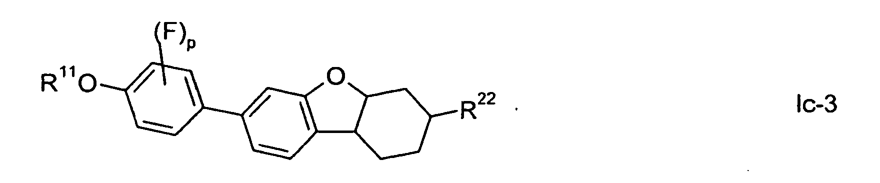 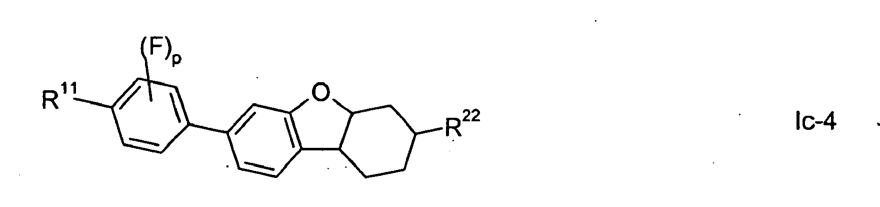 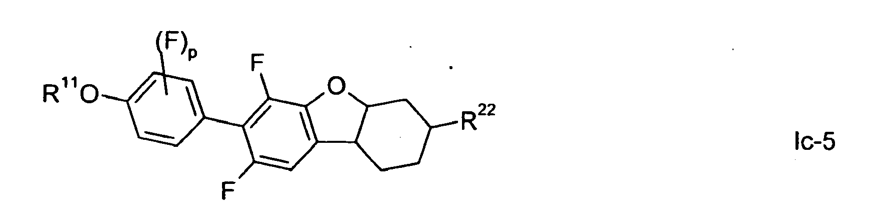 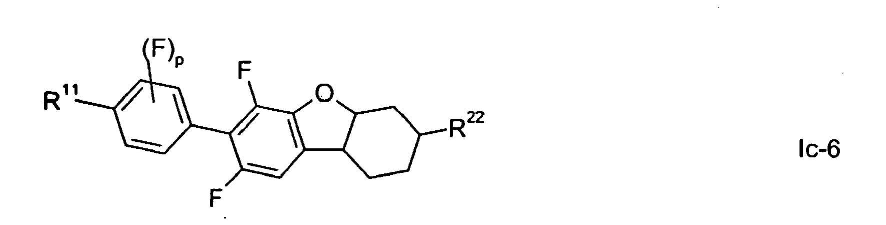 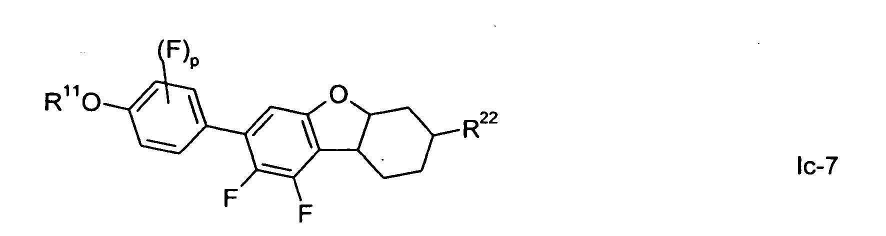 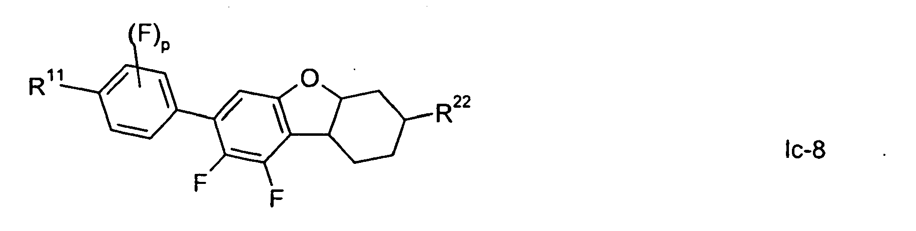 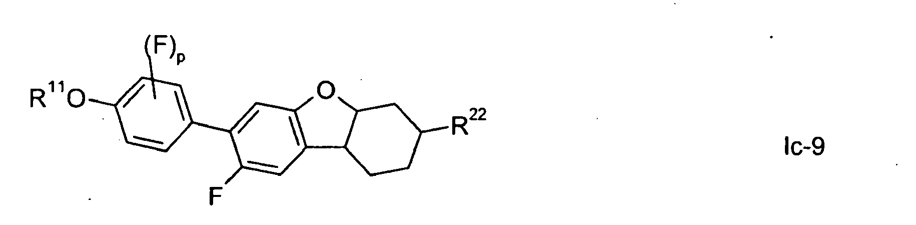 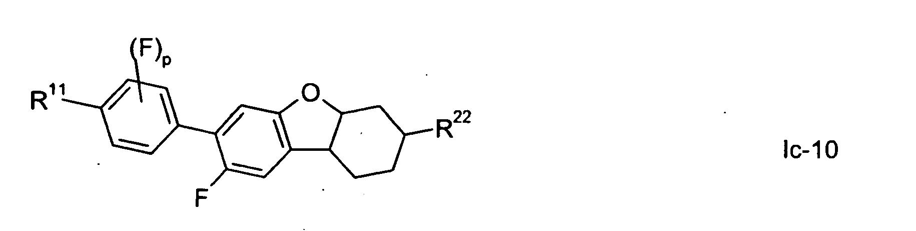 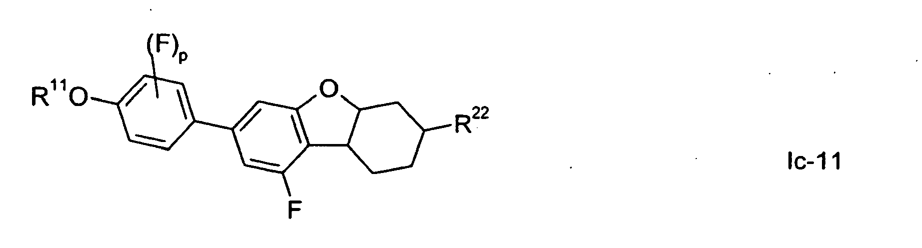 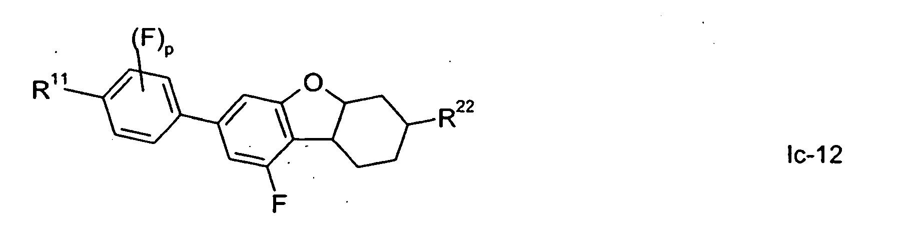 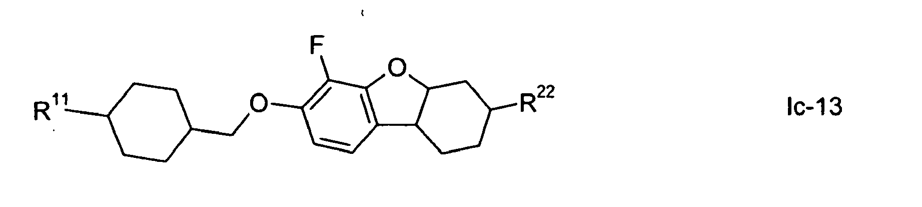 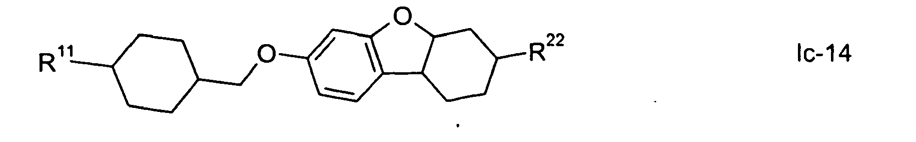 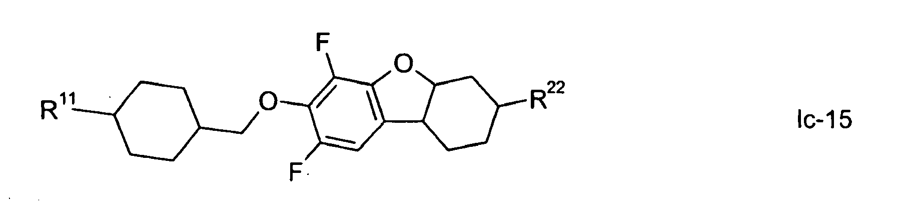 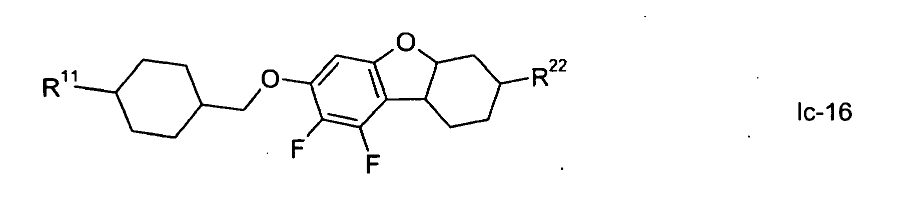 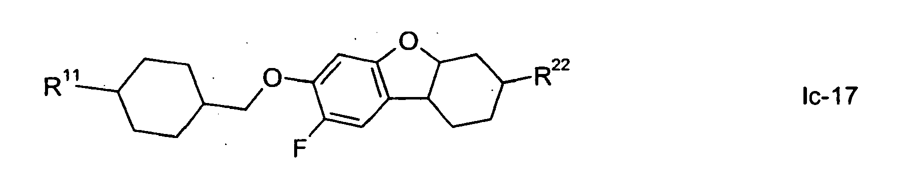 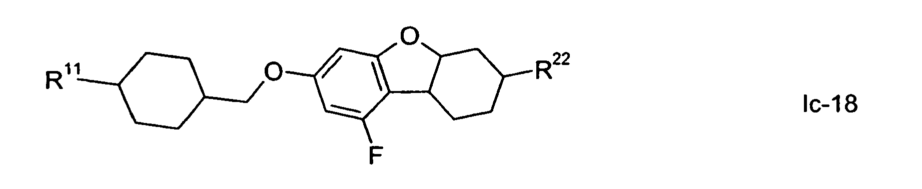 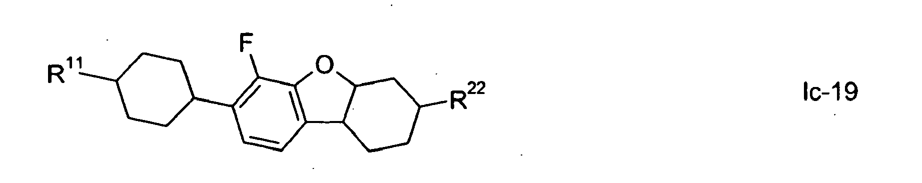 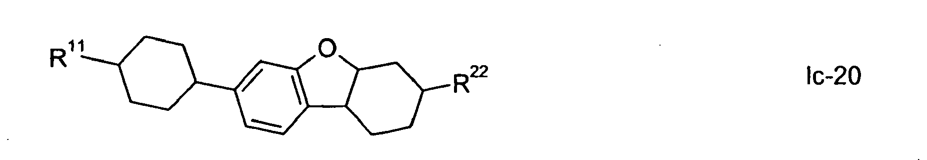 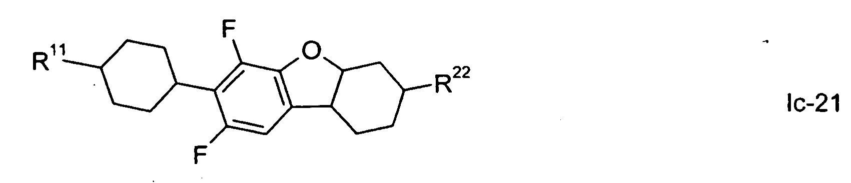 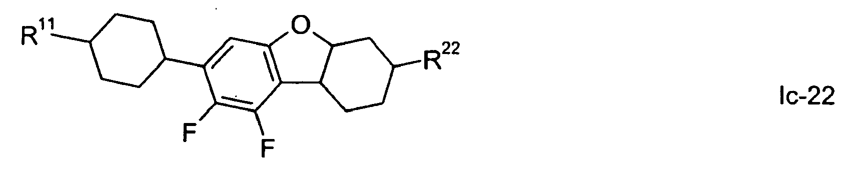 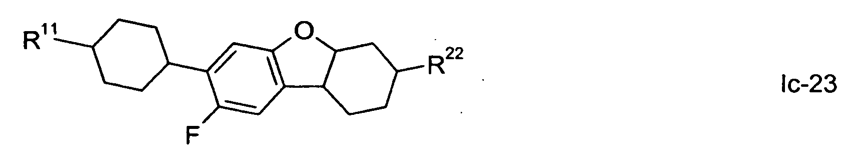 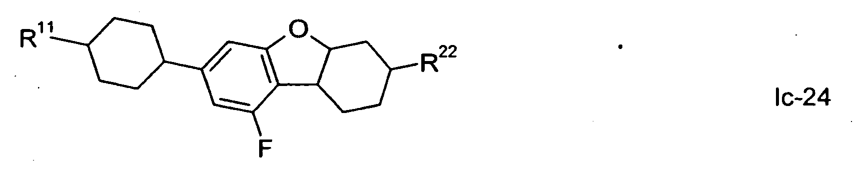 worin
R¹¹, R²² und p die gleichen Bedeutungen wie oben definiert besitzen. Unter den Verbindungen der Formeln Ic1-Ic24 sind Verbindungen der Formeln mit einem unsubstituierten oder substituierten 1,4-Phenylen-Ring und die Verbindung Ic-13 am meisten bevorzugt.

Unter den bevorzugten erfindungsgemäßen Verbindungen der Formel Id sind die Folgenden besonders bevorzugt: 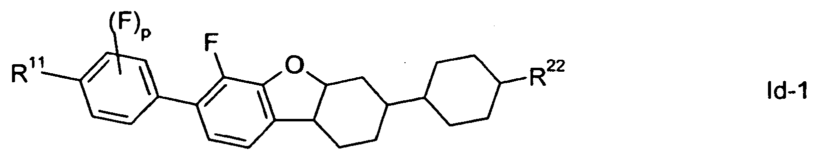 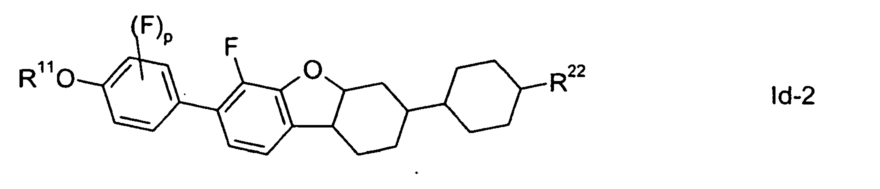 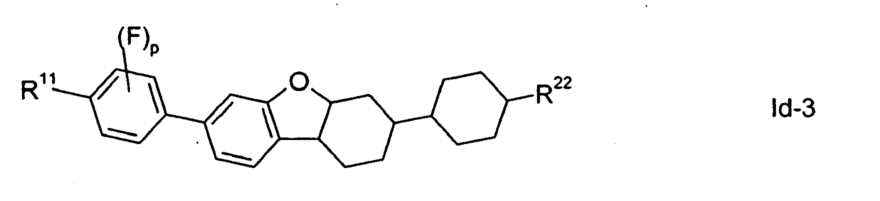 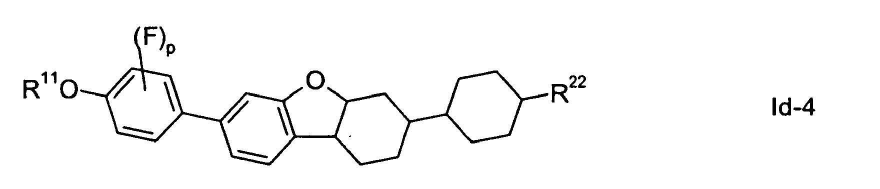 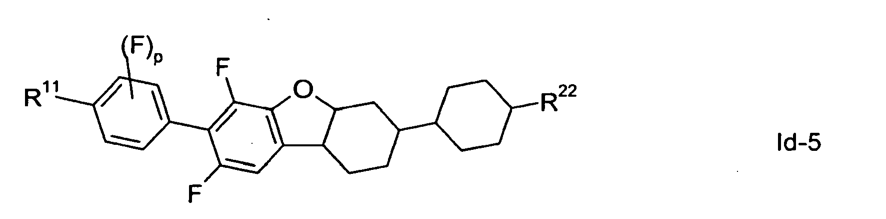 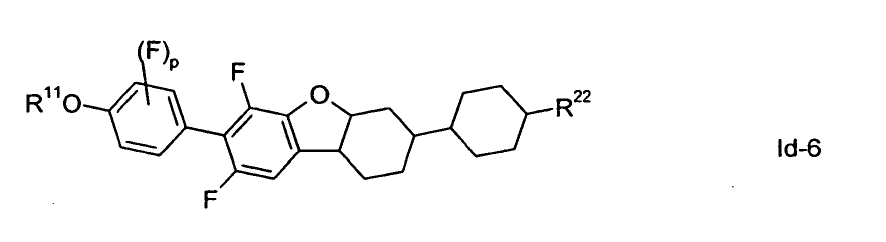 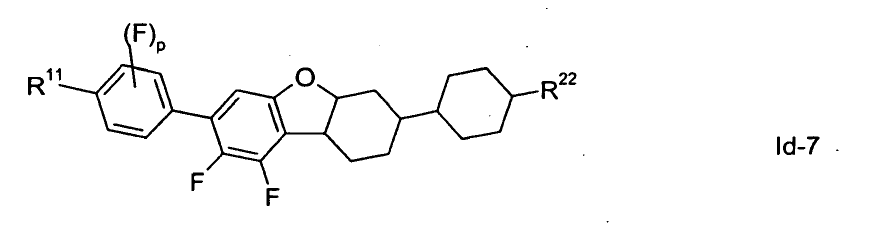 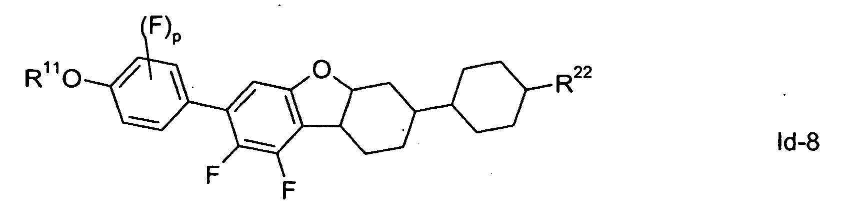 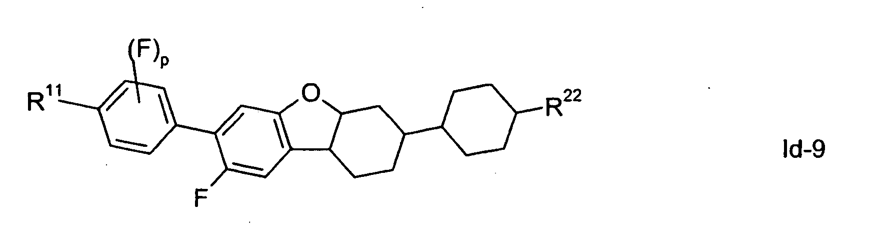 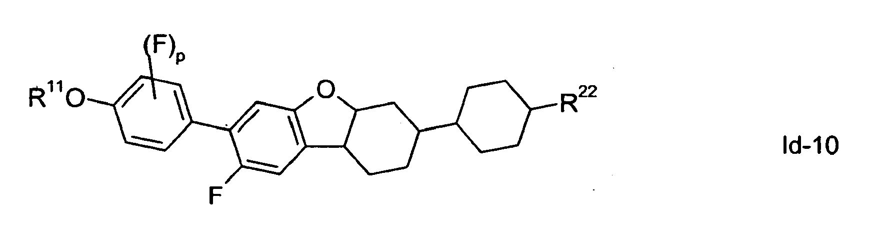 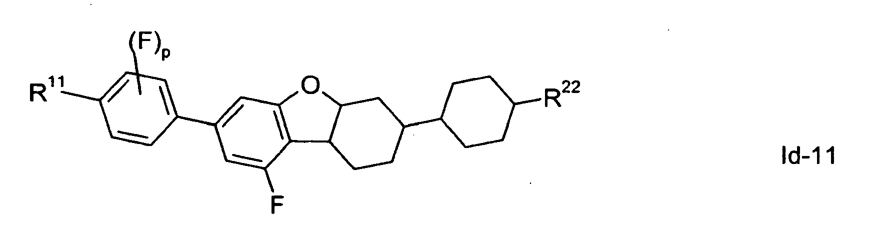 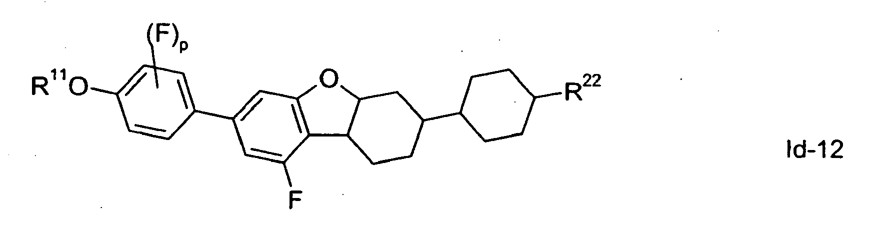 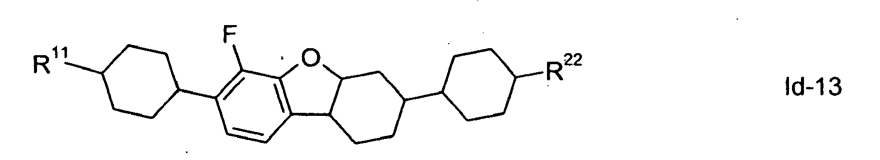 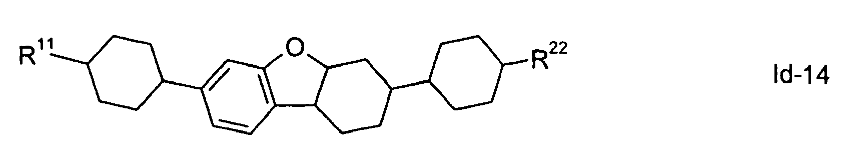 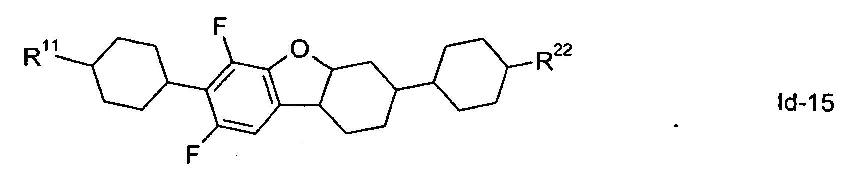 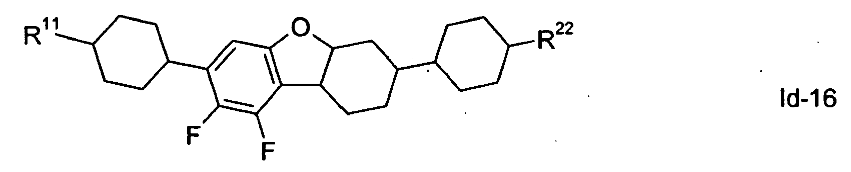 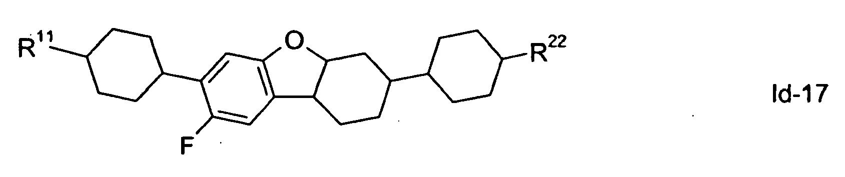 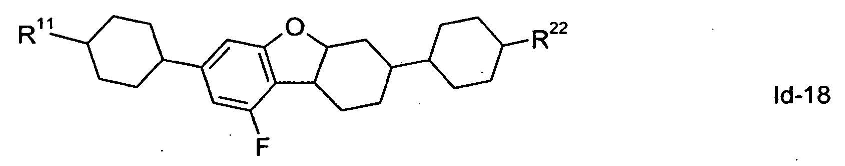 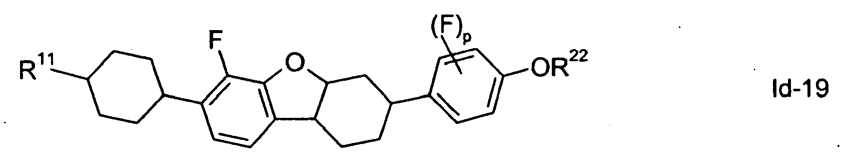 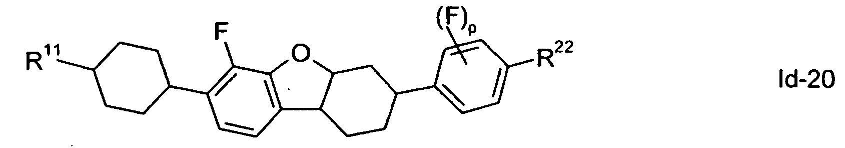 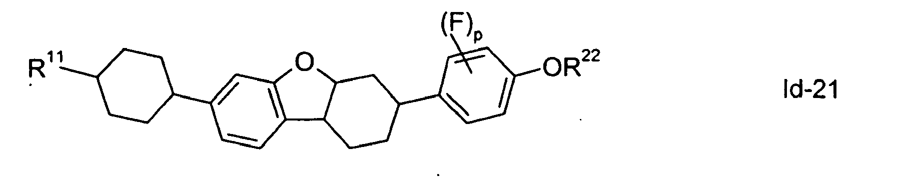 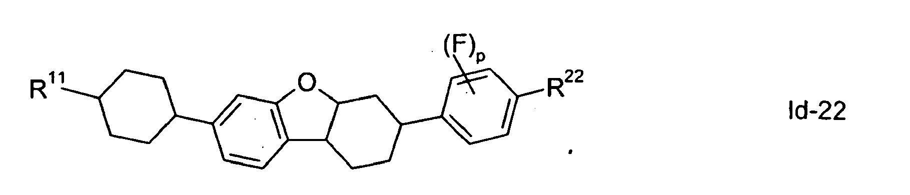 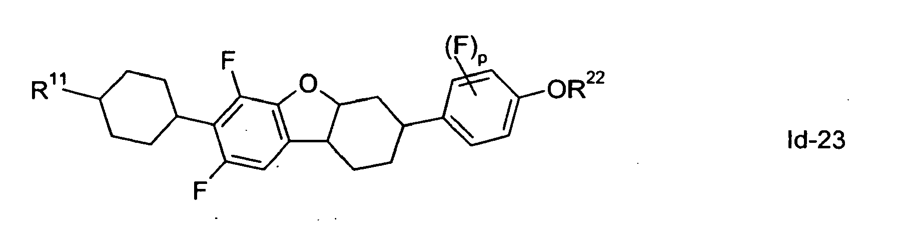 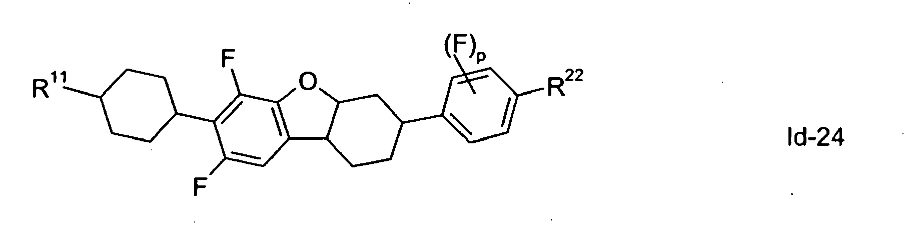 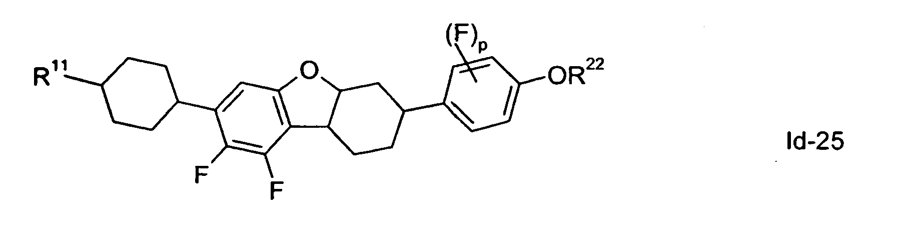 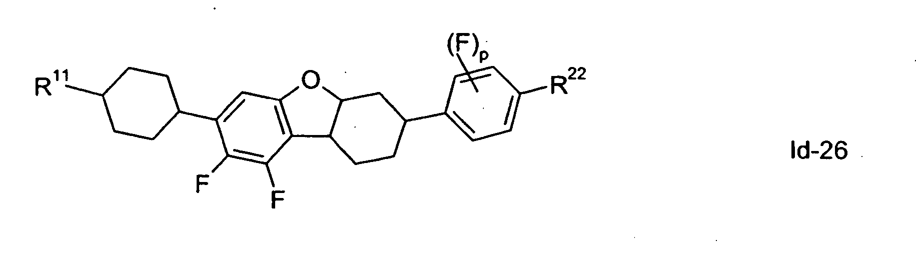 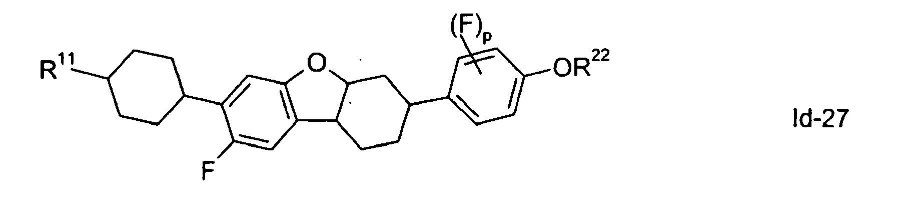 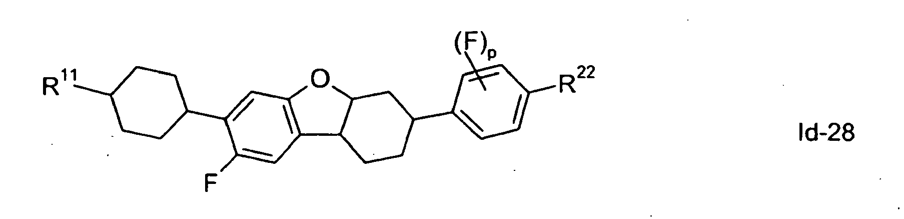 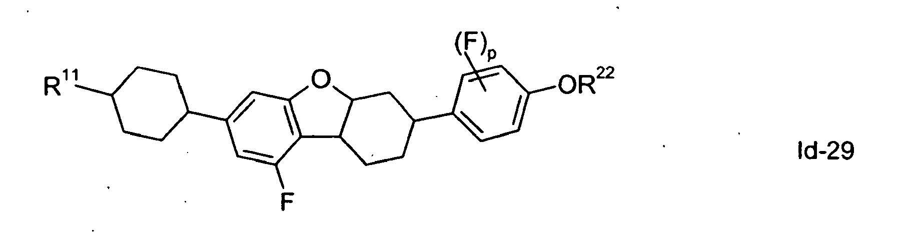 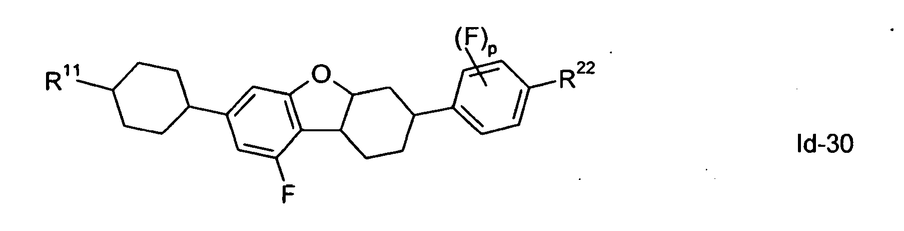 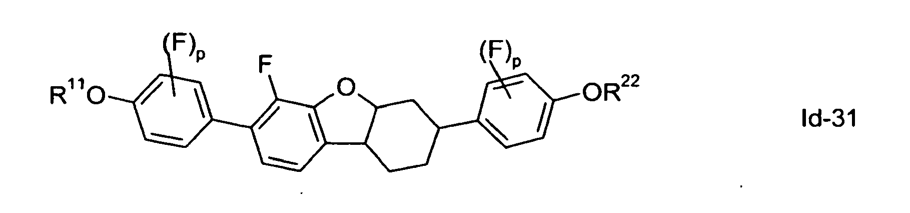 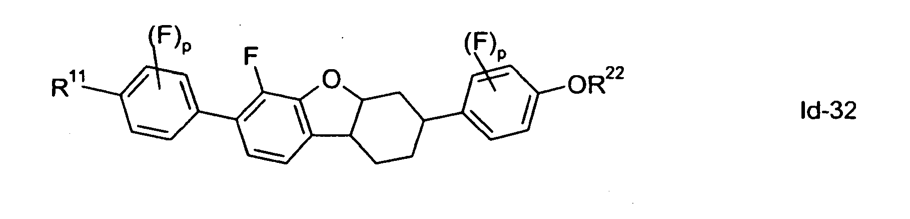 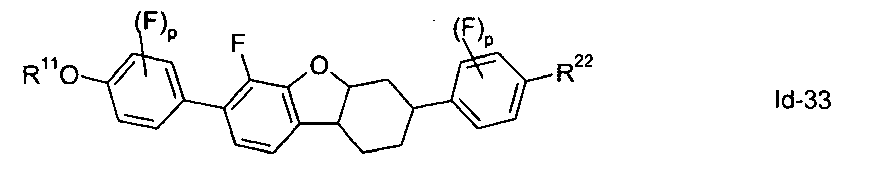 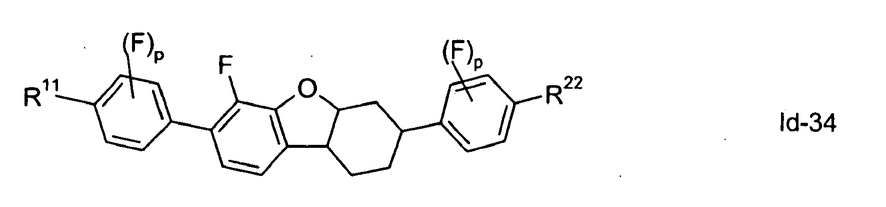 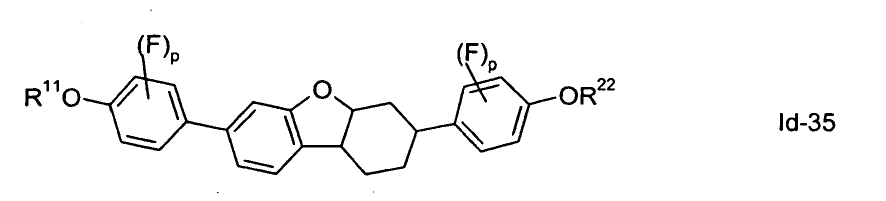 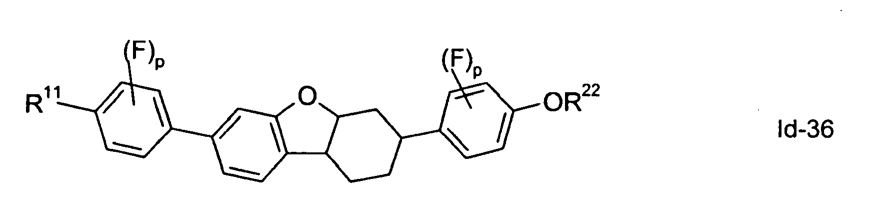 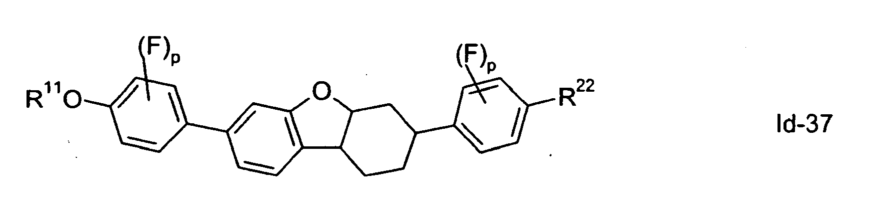 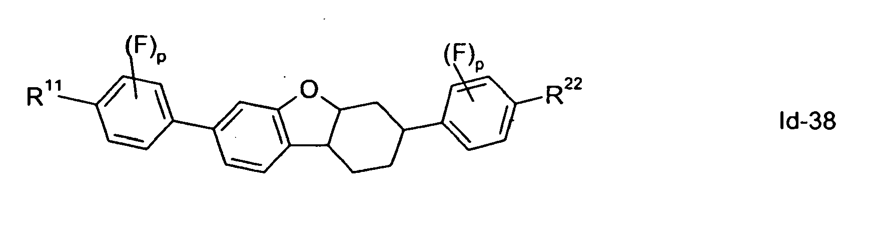 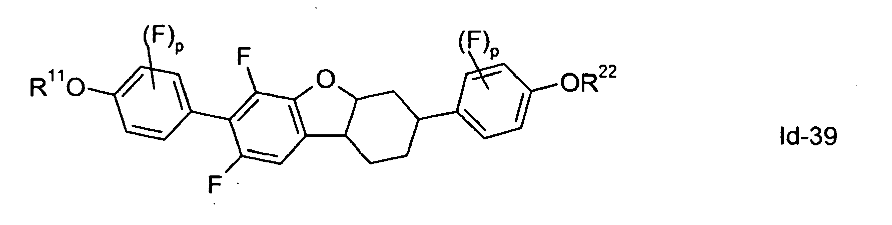 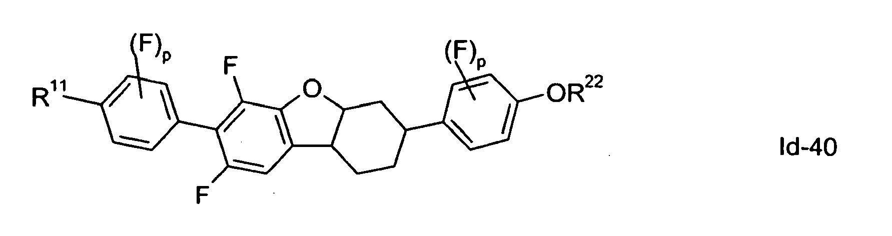 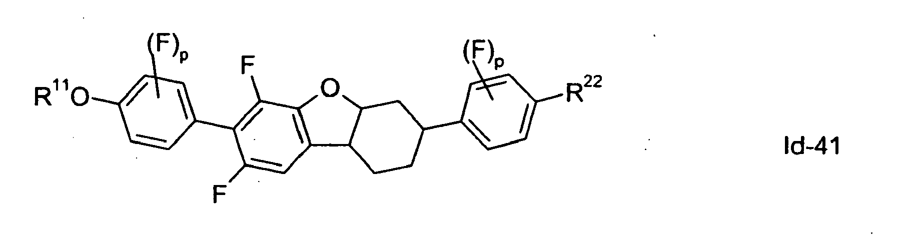 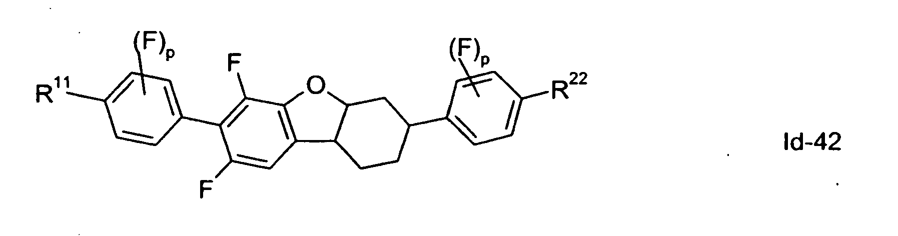 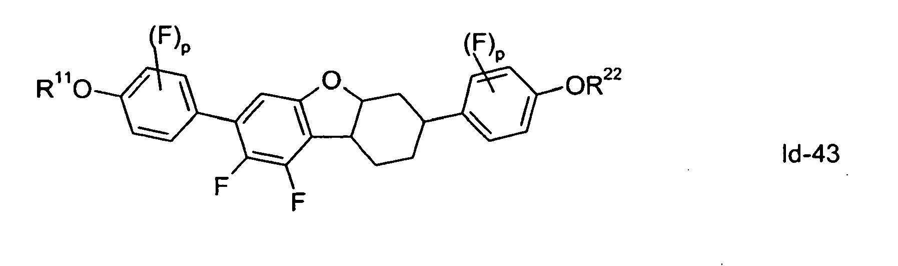 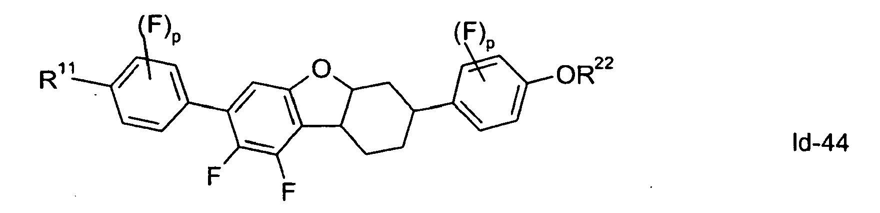 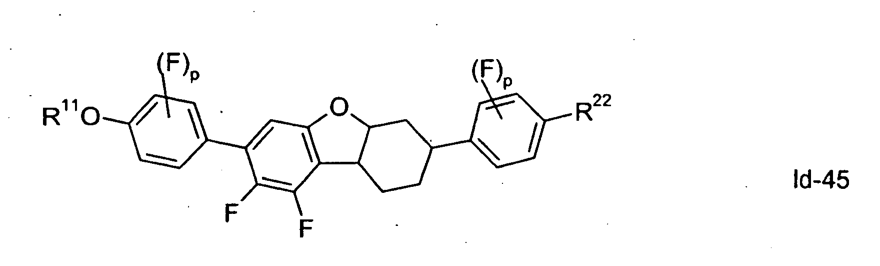 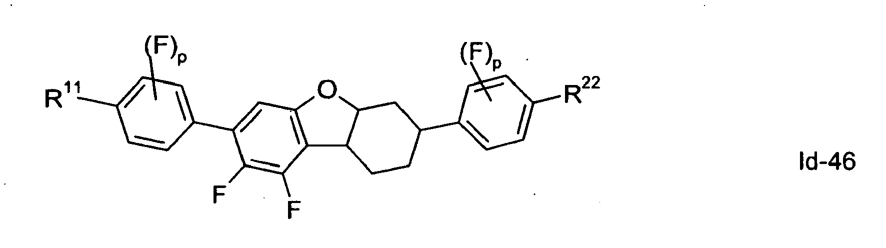 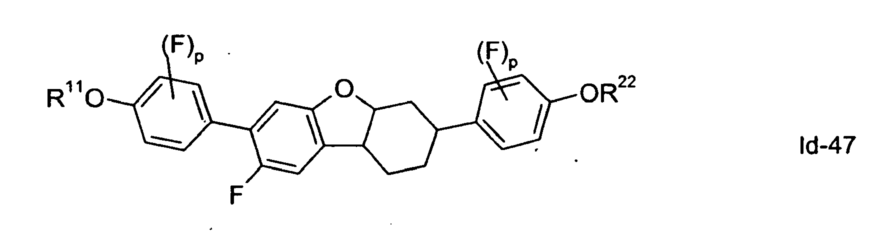 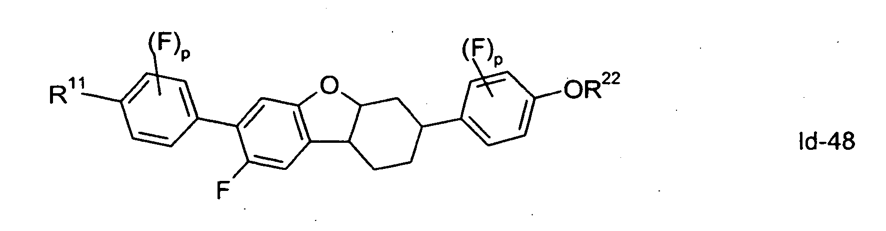 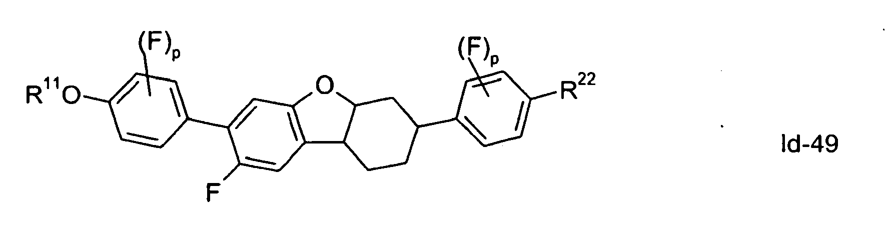 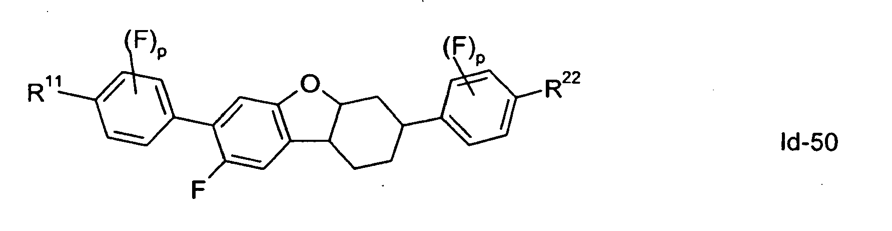 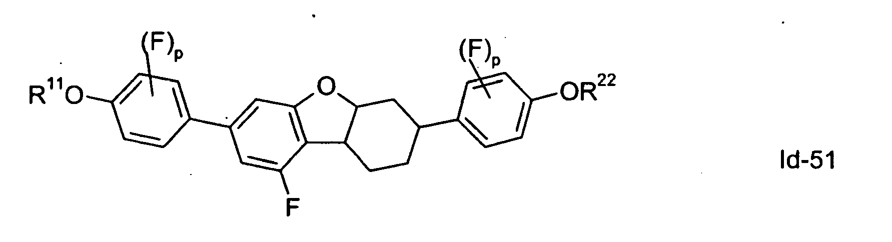 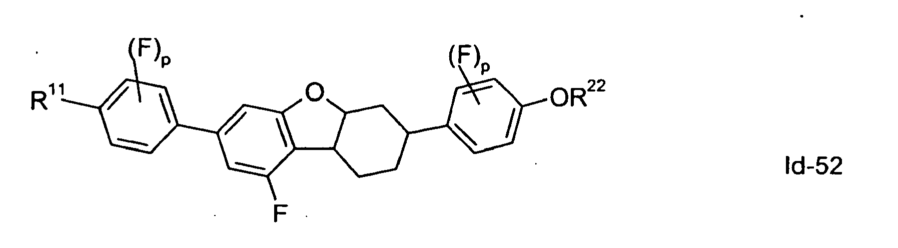 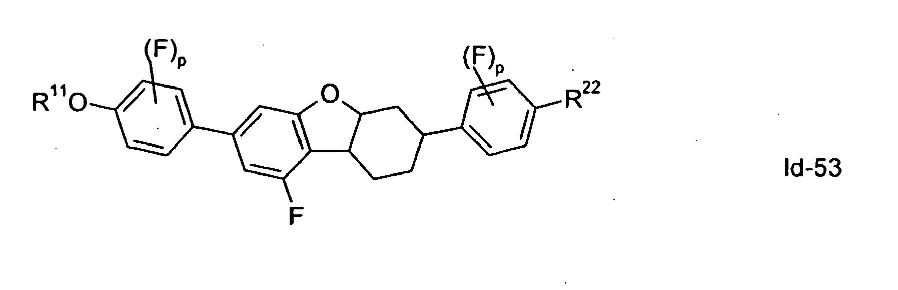 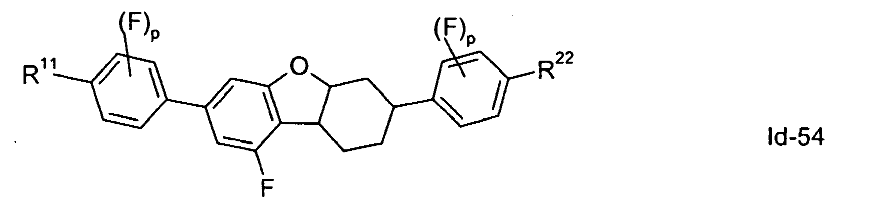 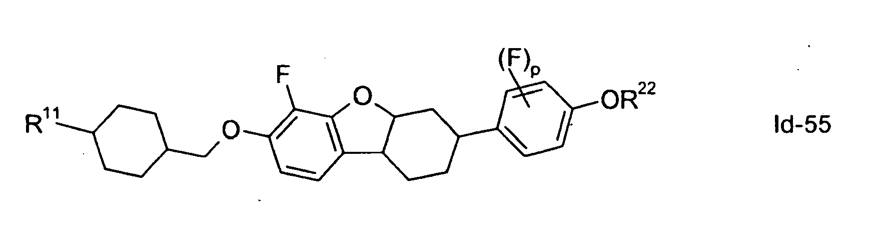 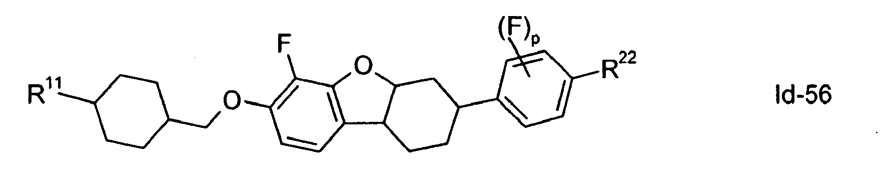 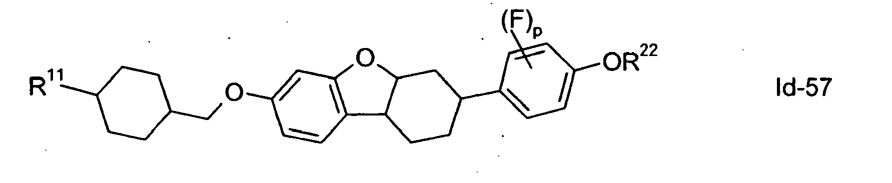 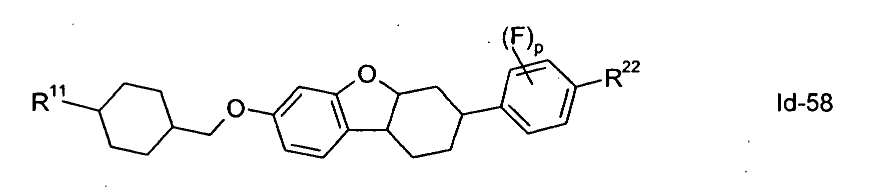 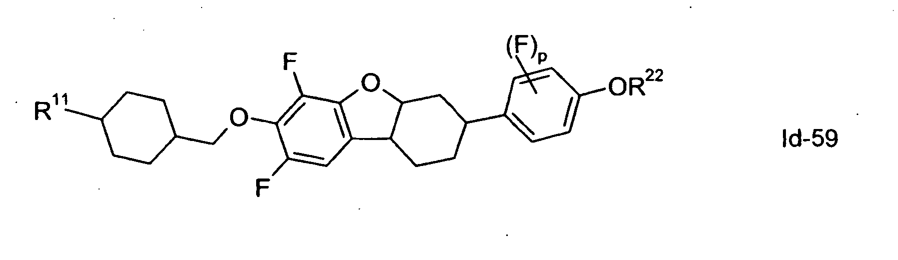 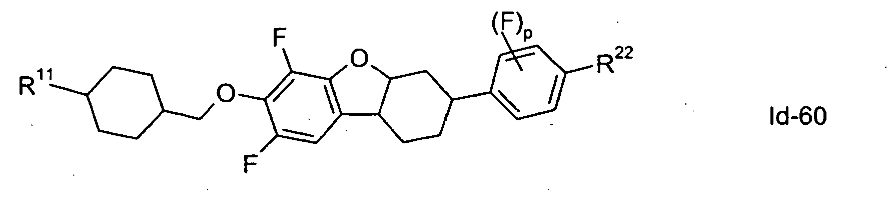 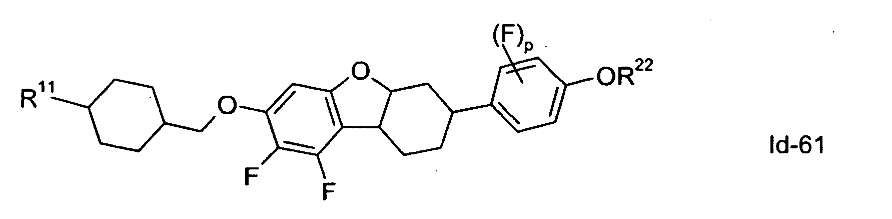 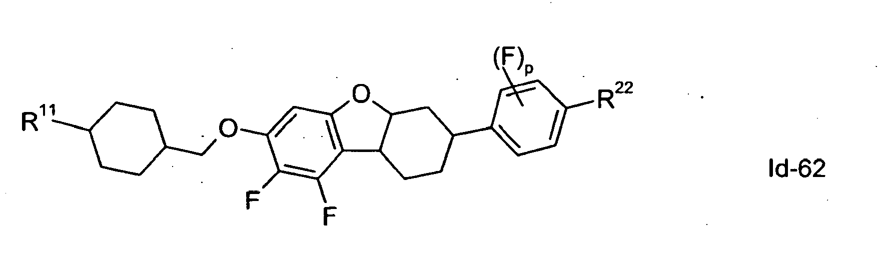 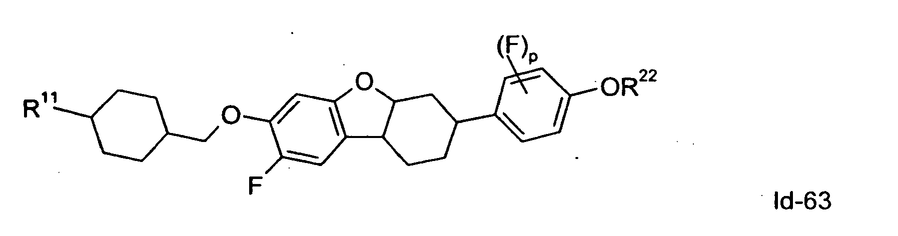 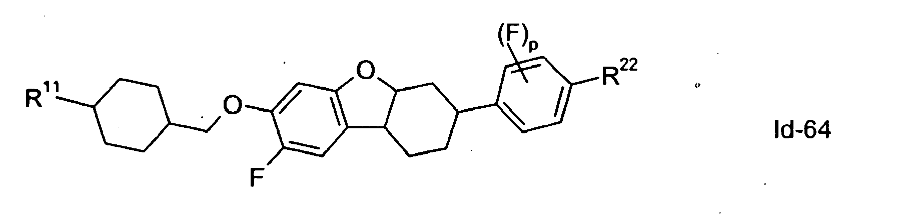 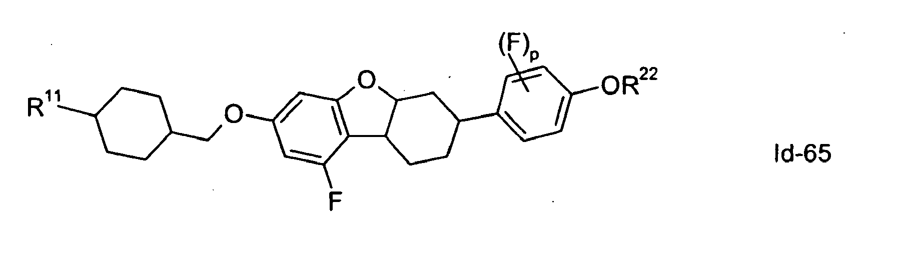 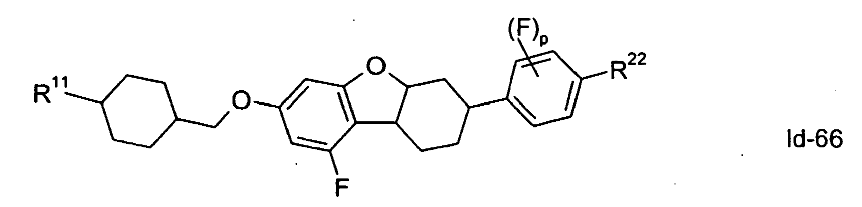 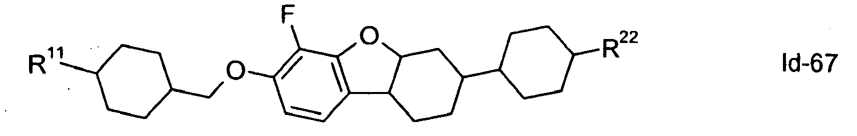 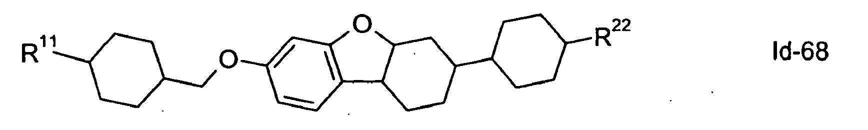 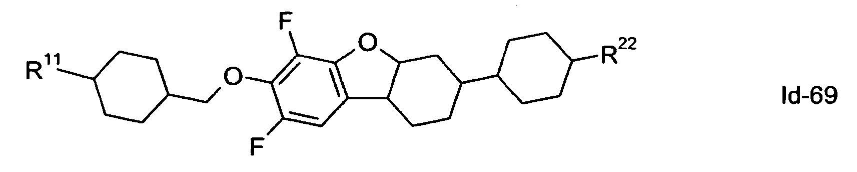 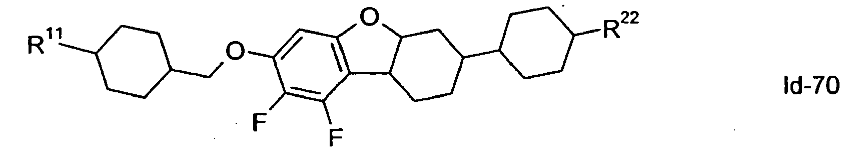 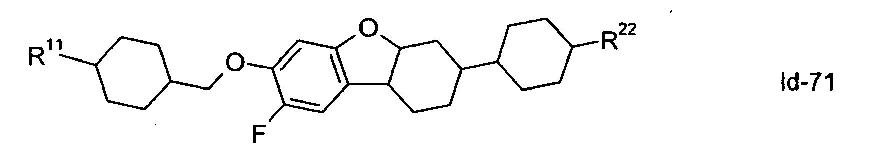 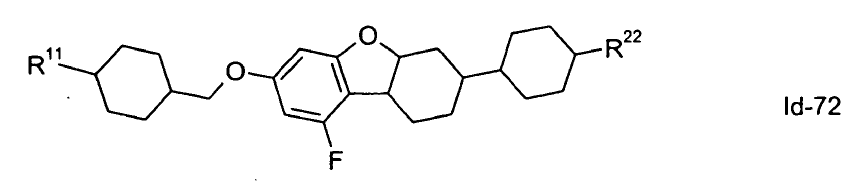 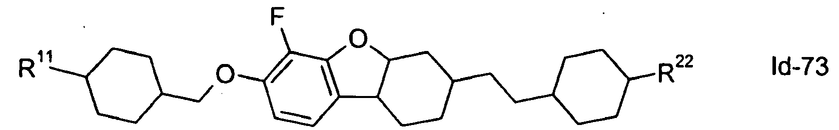 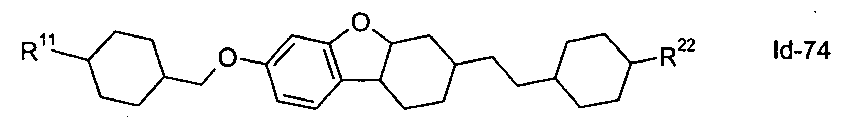 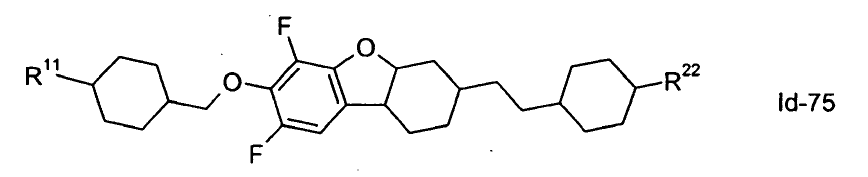 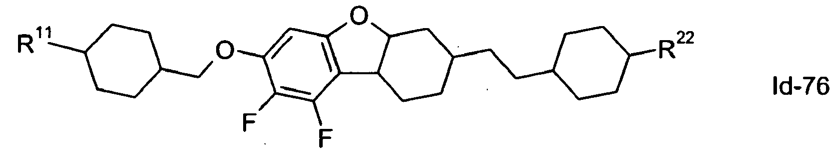 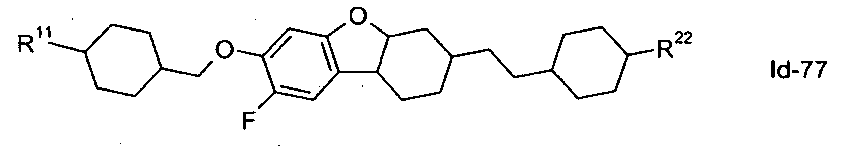 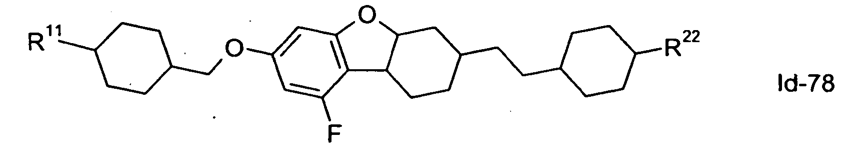 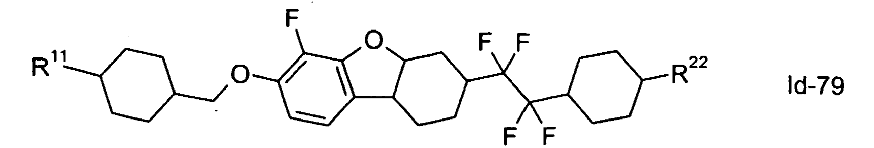 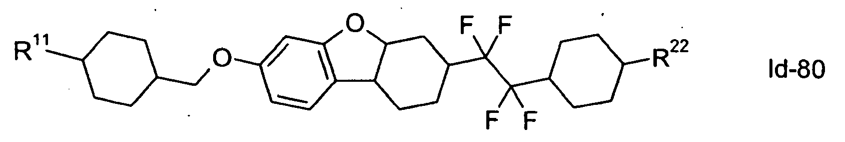 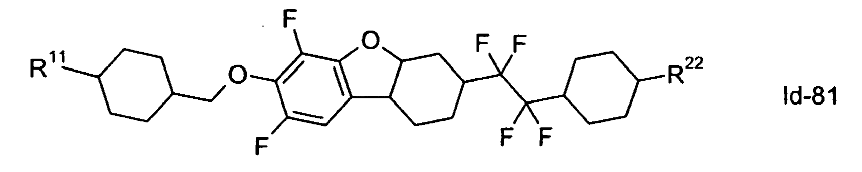 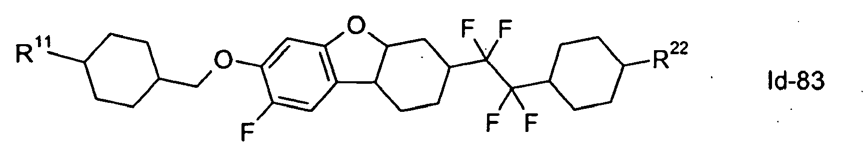 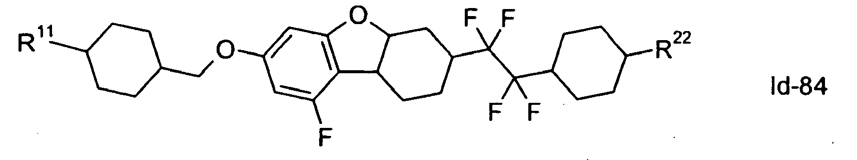 worin
R¹¹, R²² und p die gleichen Bedeutungen wie oben definiert besitzen.
Soweit p in den Formeln mehrmals vorkommt, 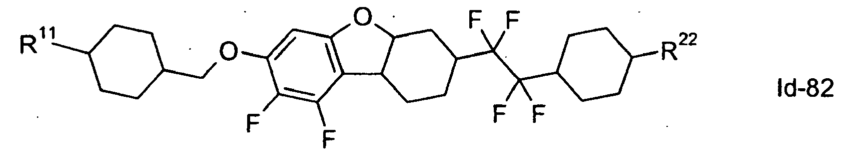 kann es jeweils die gleiche oder verschiedene Bedeutung aufweisen.

Unter den Verbindungen der Formeln Id-1 bis Id-84 sind Verbindungen der Formeln bevorzugt, in denen der Grundkörper linksseitig mit einem Arylrest substituiert ist und / oder der Grundkörper rechtsseitig mit einem Cyclohexylrest verknüpft ist, also die Verbindungen der Formeln Id-1 bis Id-18, Id-31 bis Id-54 und Id-67 bis Id-72. Besonders bevorzugt sind insgesamt die Verbindungen der Formeln Id-1 bis Id-13 und Id-67 bis Id-72, ferner Id-13 bis Id-18, Id-31 bis Id-38 und Id-73 bis Id-84; ganz besonders die Formeln Id-1, Id-2, Id-3, Id-4, Id-67 und Id-68.

Unter den bevorzugten erfindungsgemäßen Verbindungen der Formel le sind die Folgenden besonders bevorzugt: 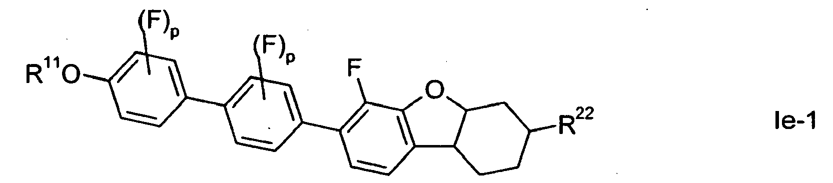 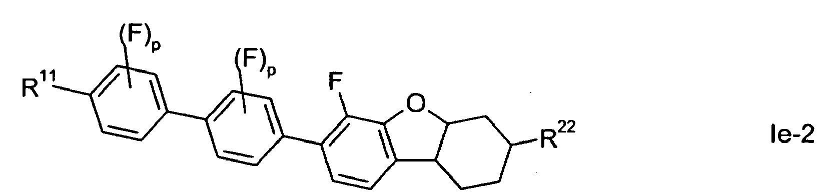 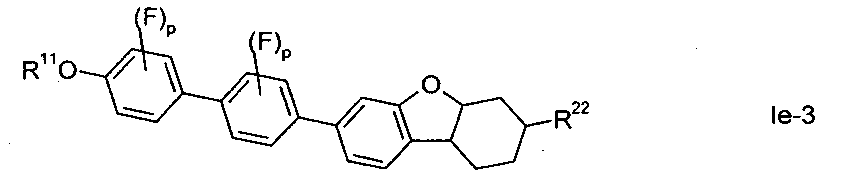 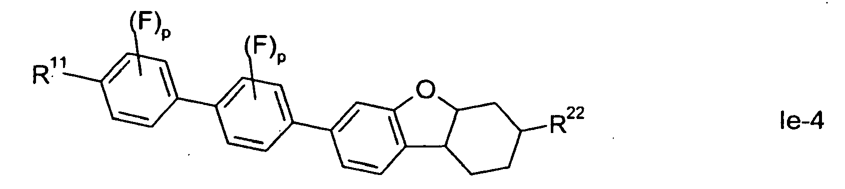 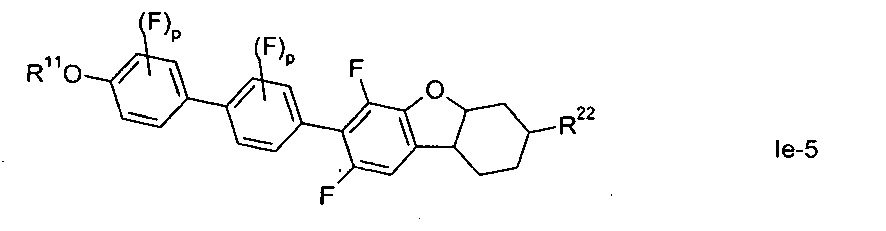 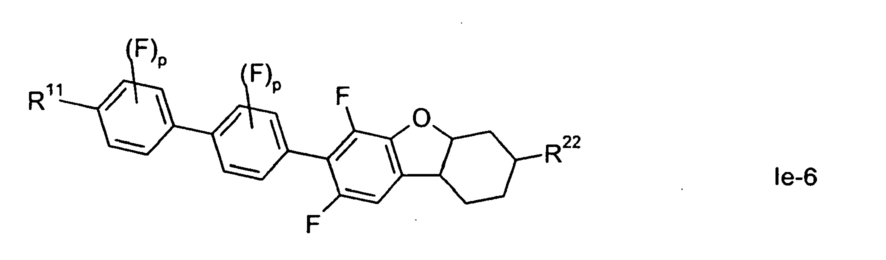 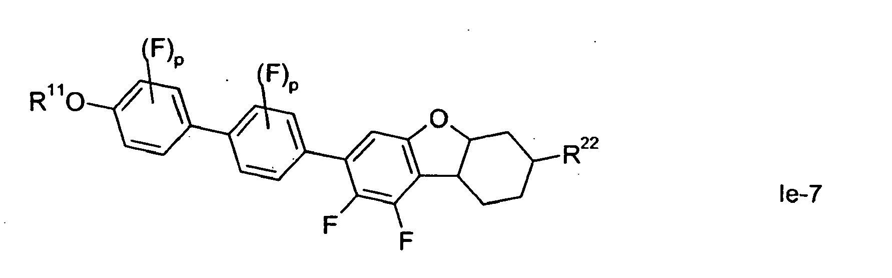 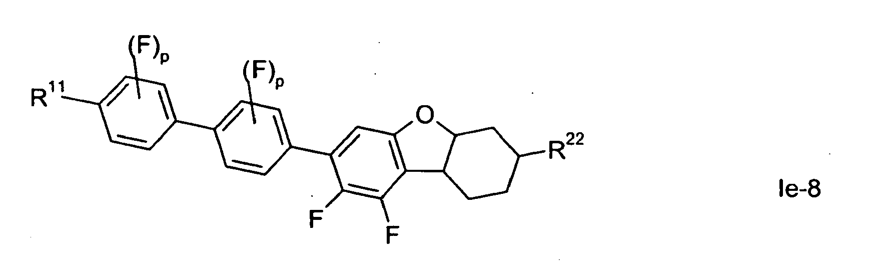 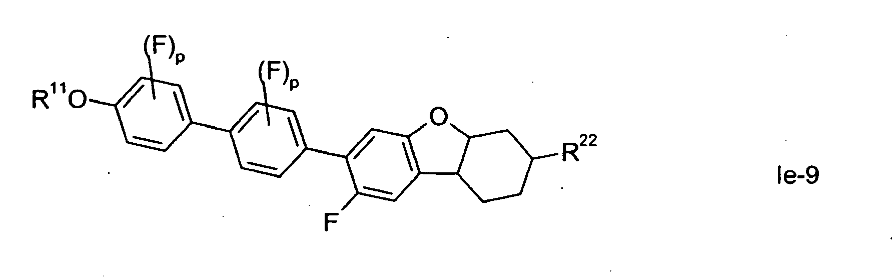 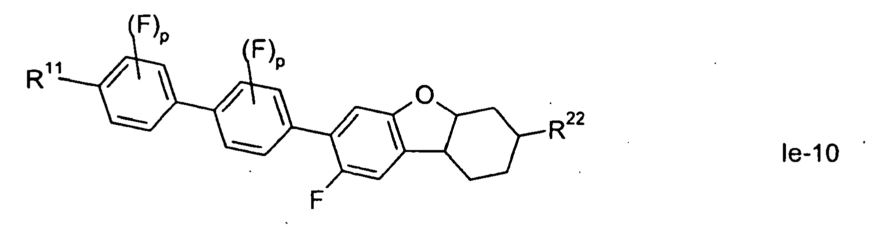 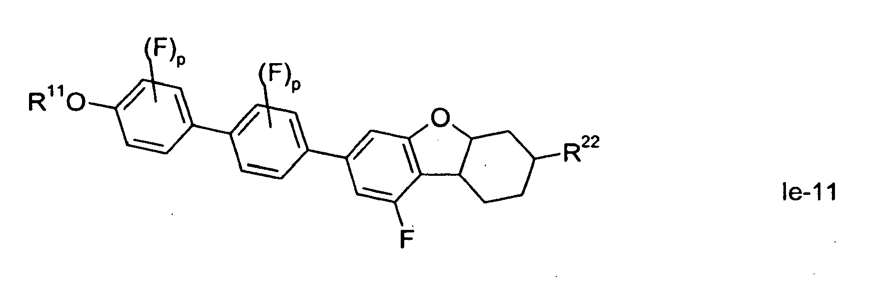 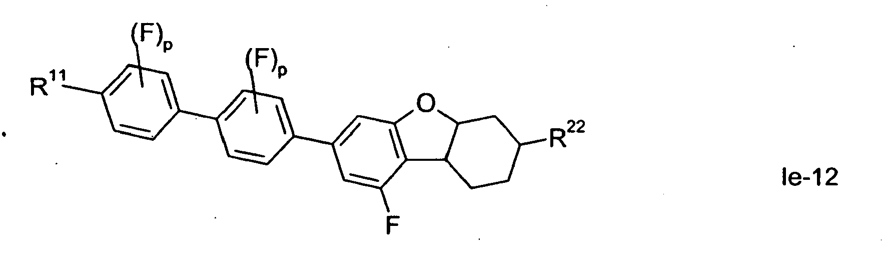 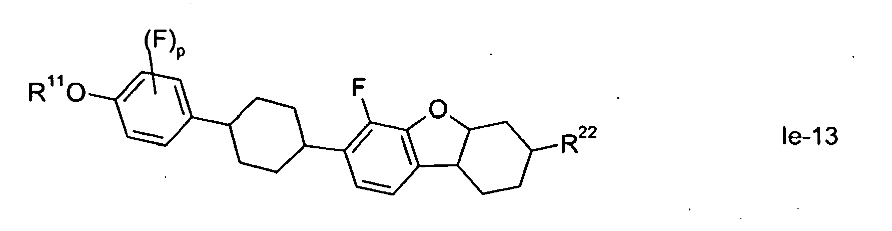 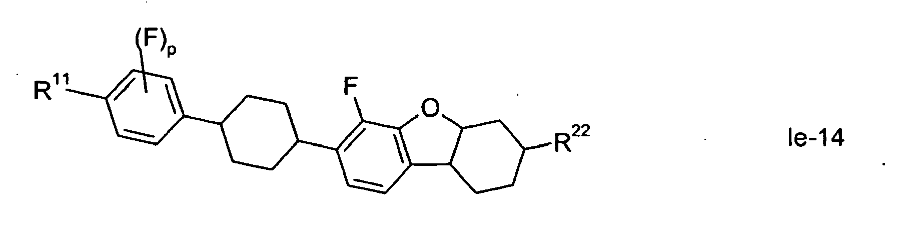 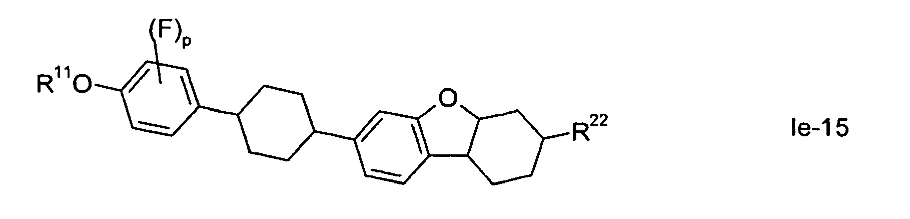 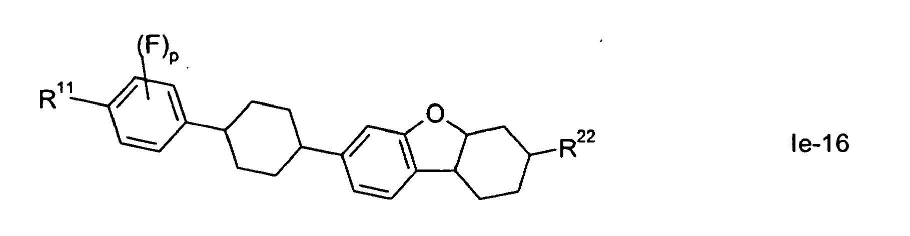 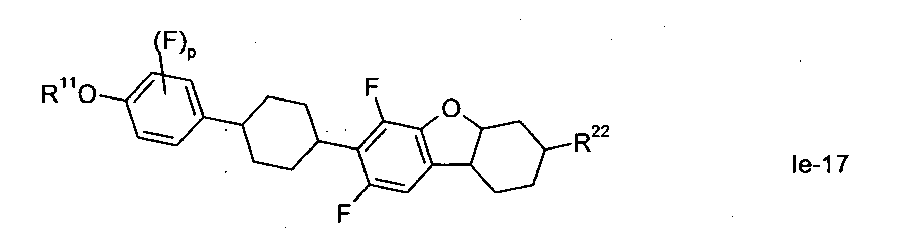 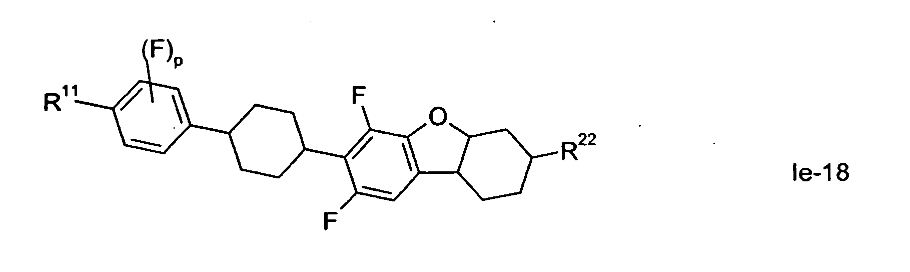 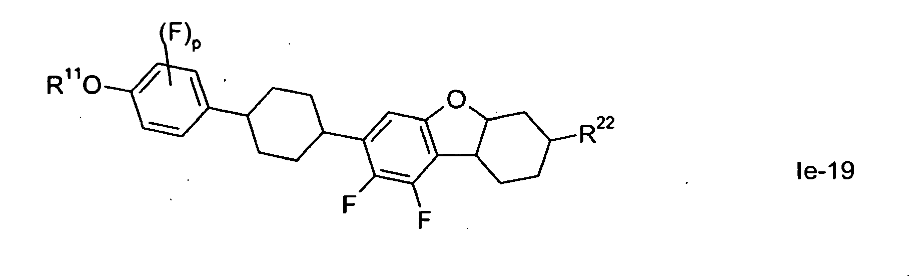 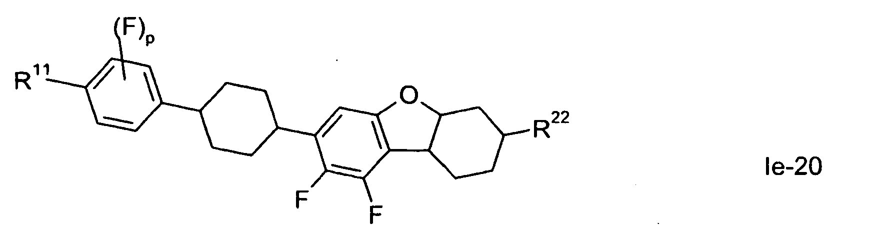 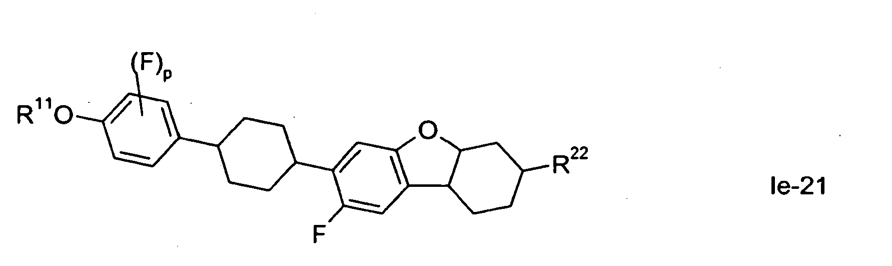 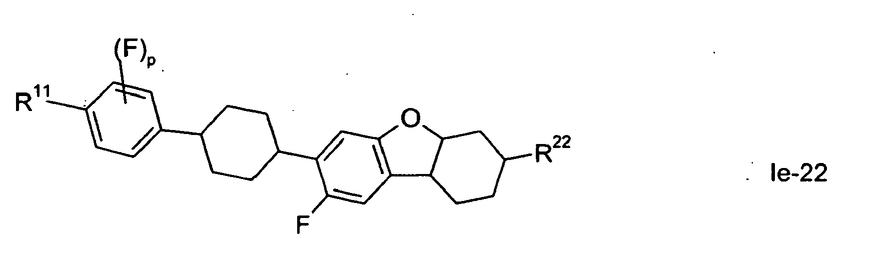 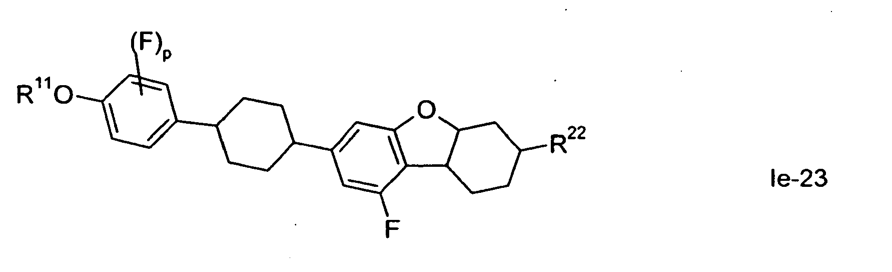 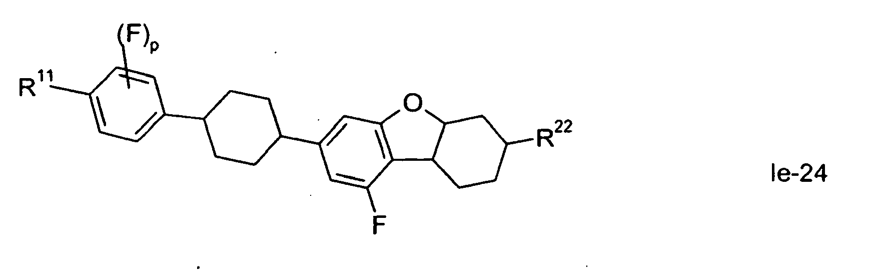 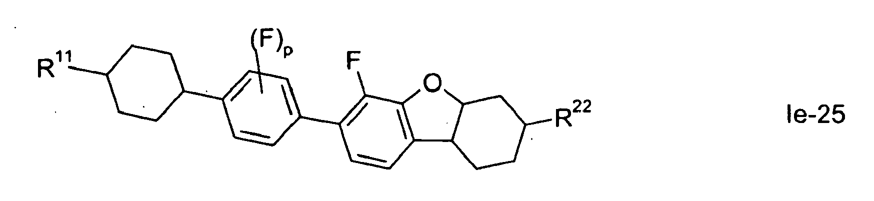 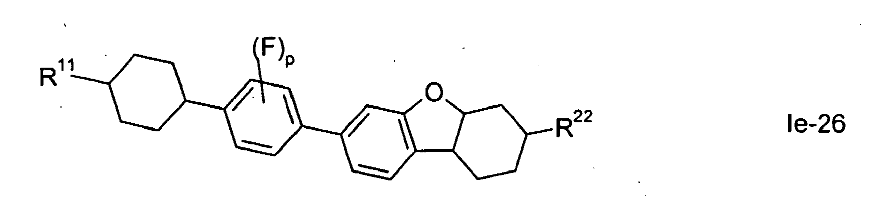 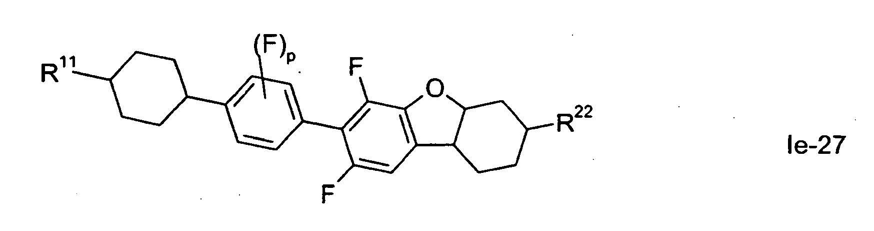 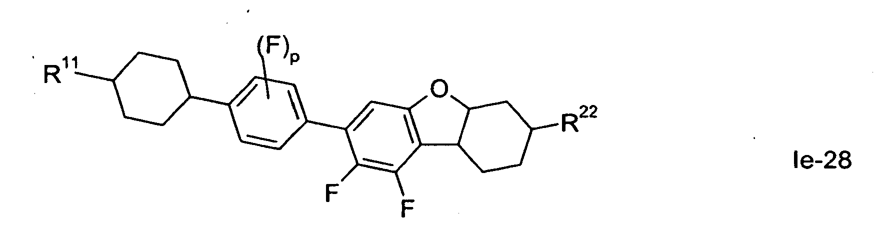 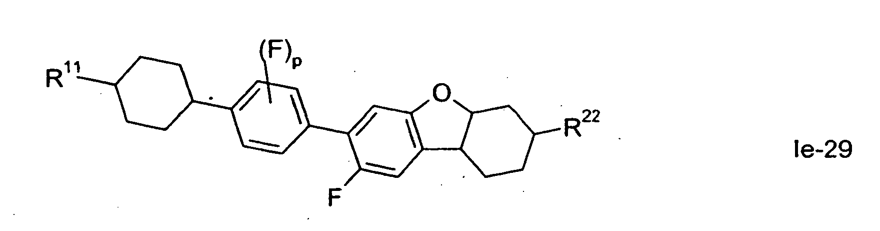 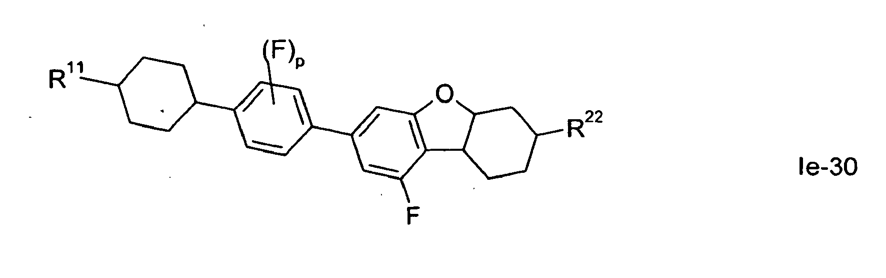 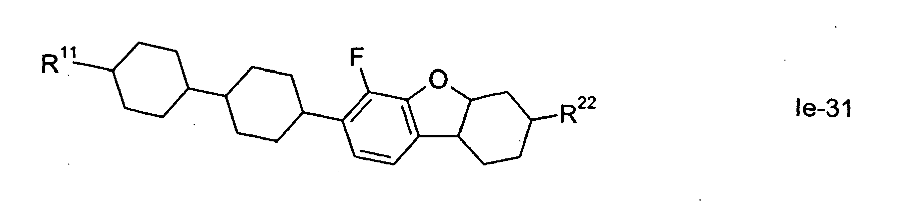 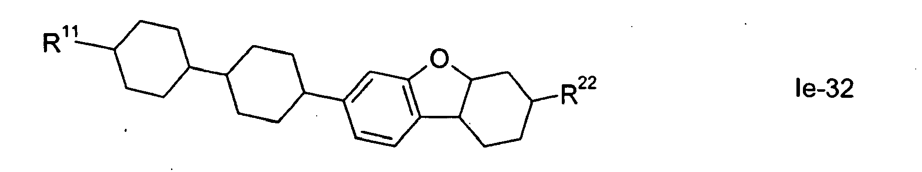 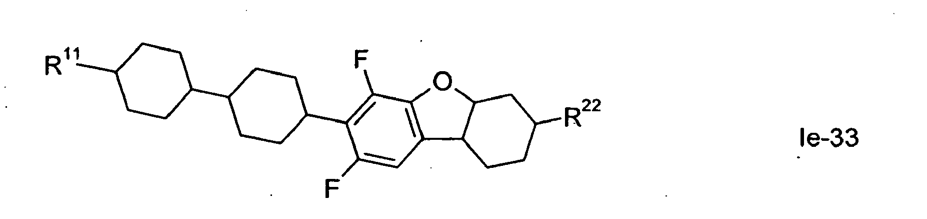 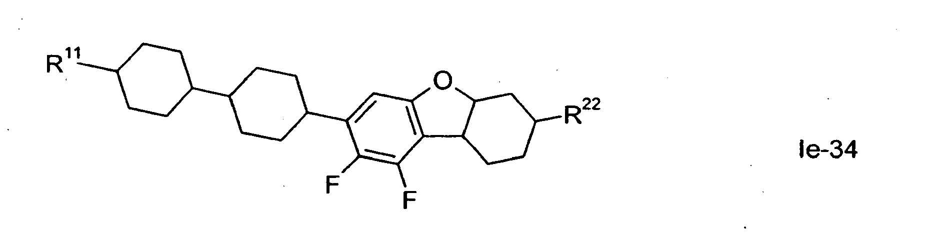 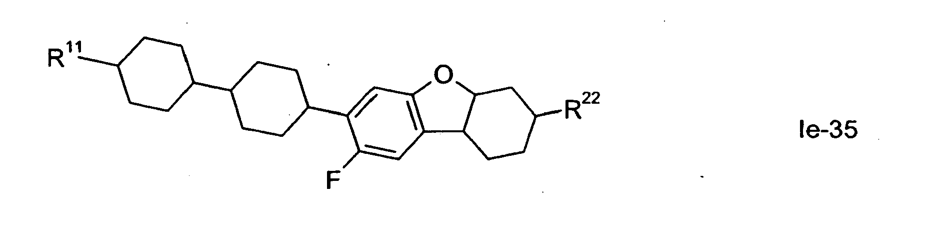 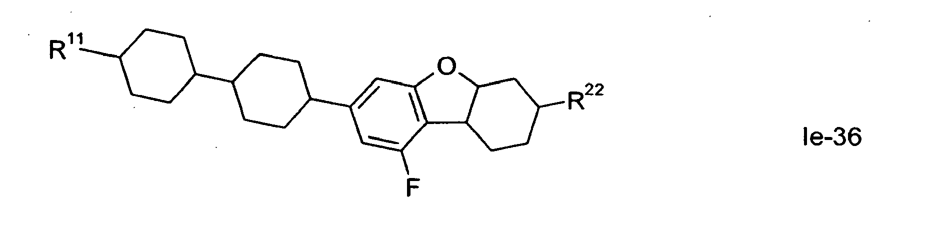 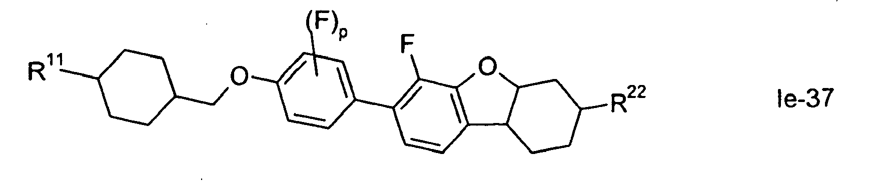 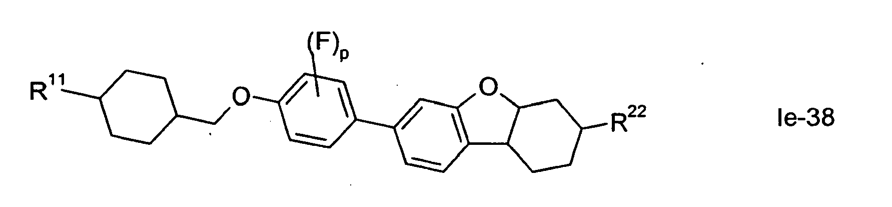 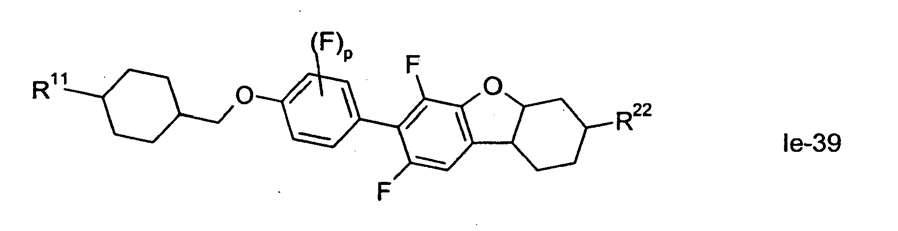 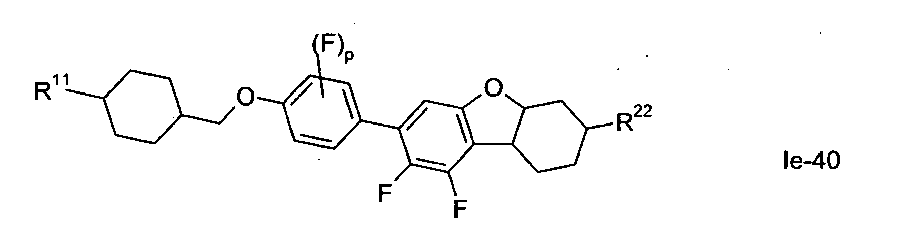 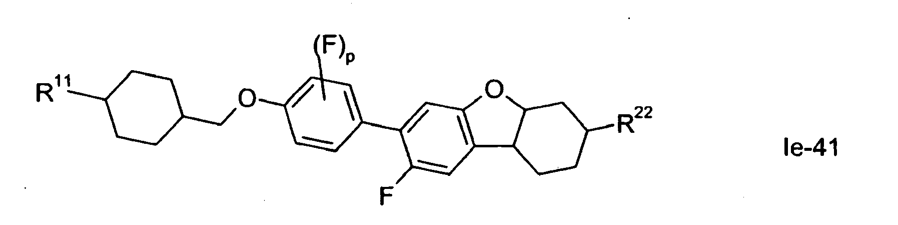 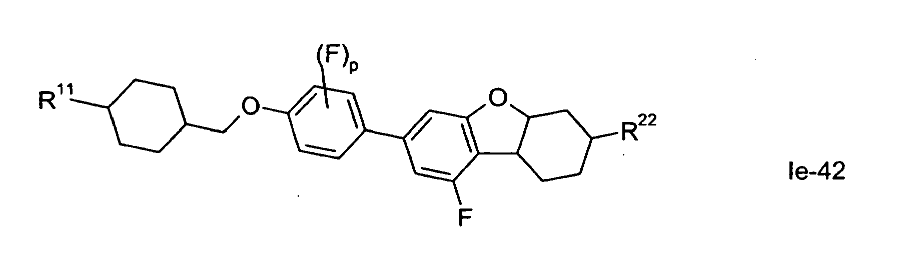 worin
R¹¹, R²² und p die gleichen Bedeutungen wie oben definiert besitzen. Soweit p in den Formeln mehrmals vorkommt, kann es jeweils die gleiche oder verschiedene Bedeutung aufweisen.

Unter den Verbindungen der Formeln Ie-1 bis Ie-42 sind Verbindungen der Formeln mit einem unsubstituierten oder substituerten 1,4-Phenylen-Ring am meisten bevorzugt.

Unter den bevorzugten erfindungsgemäßen Verbindungen der Formel If sind die Folgenden besonders bevorzugt: 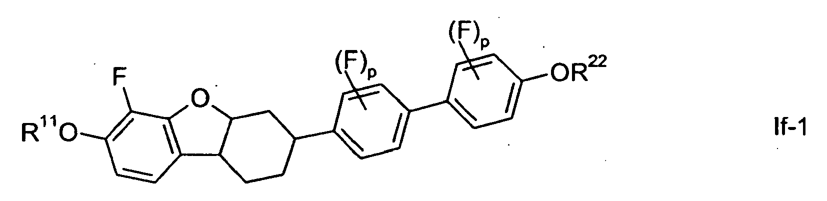 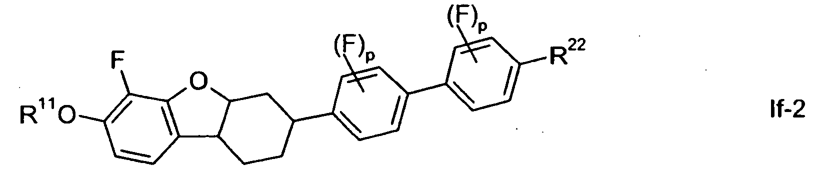 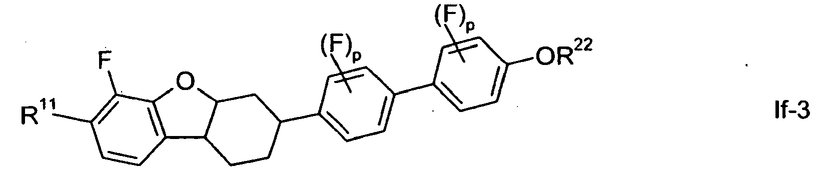 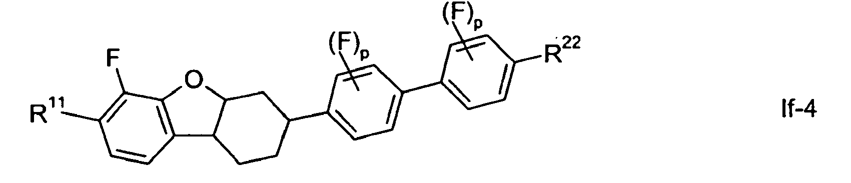 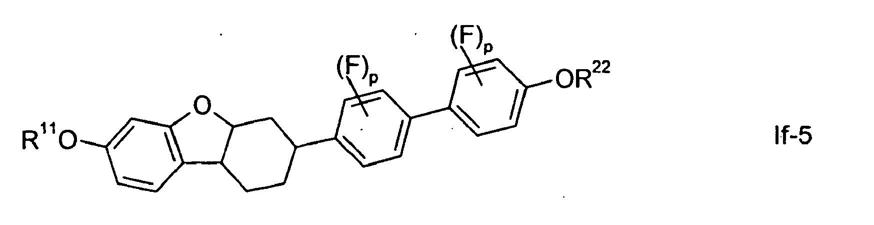 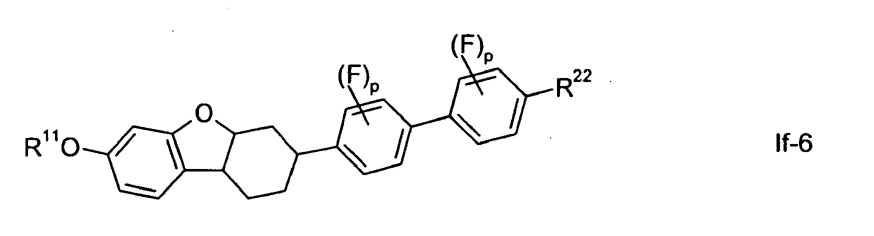 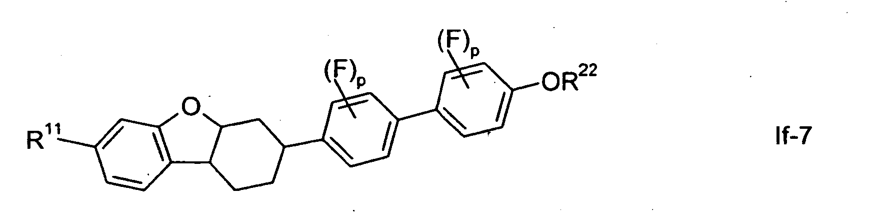 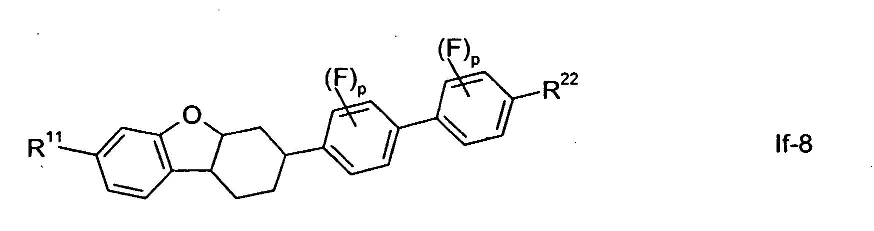 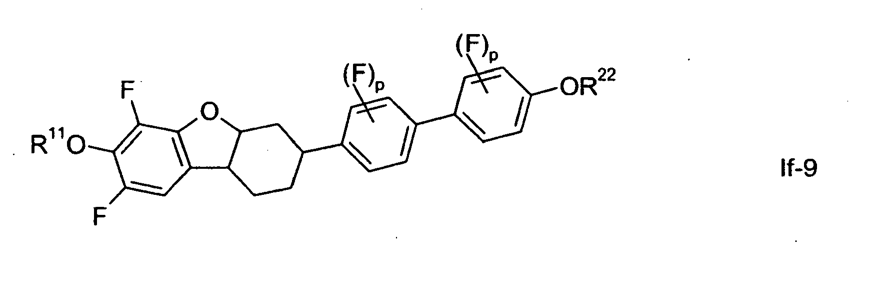 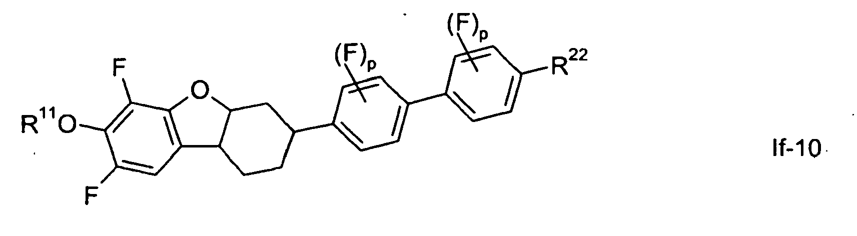 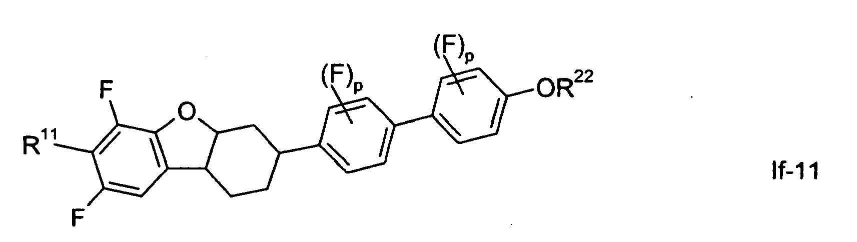 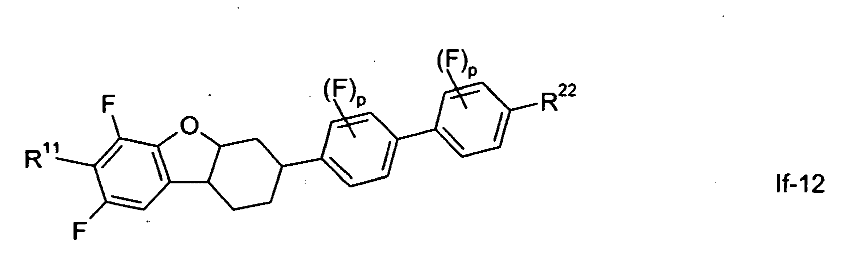 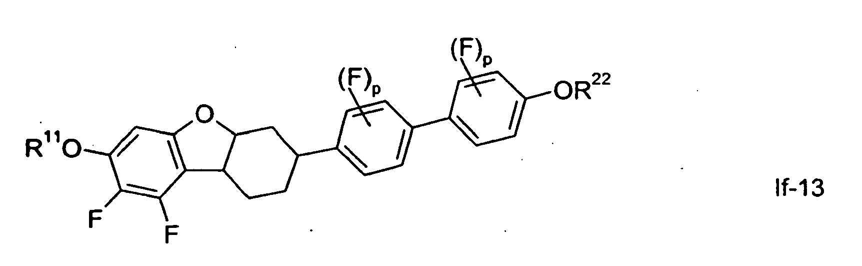 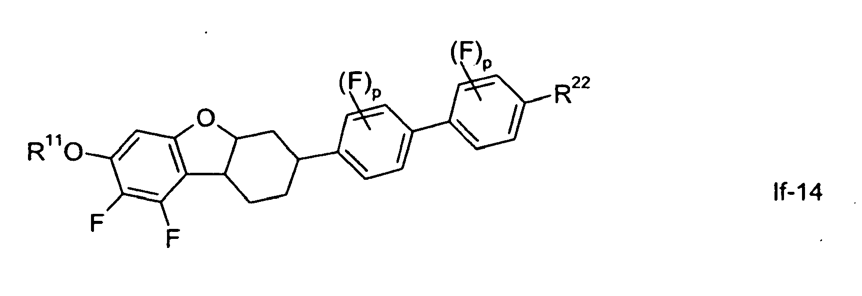 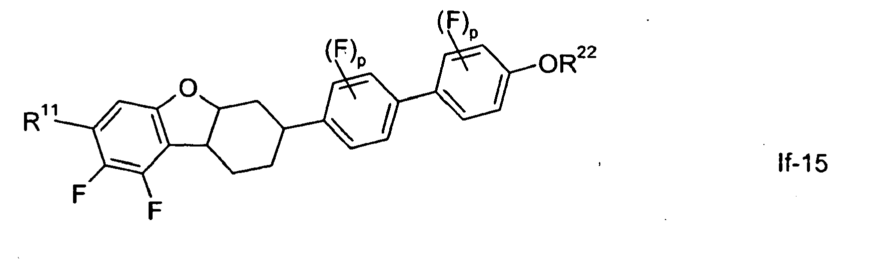 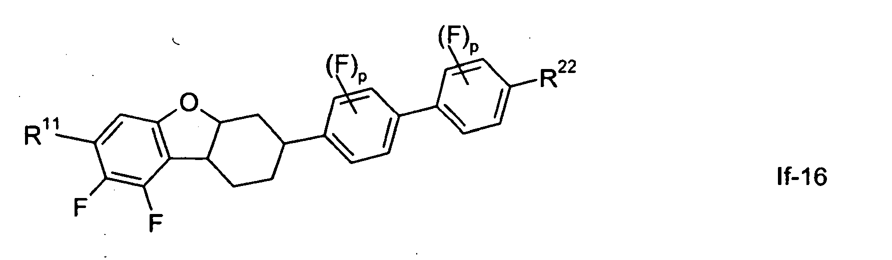 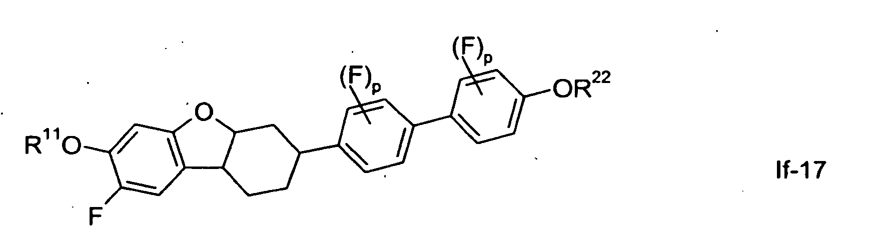 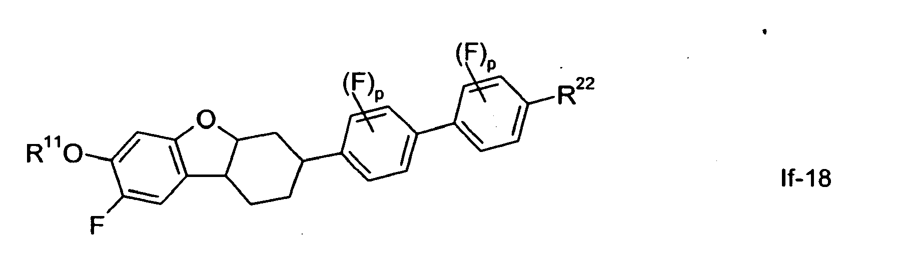 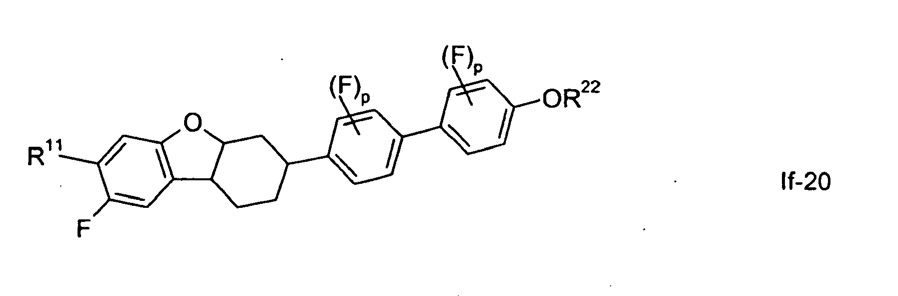 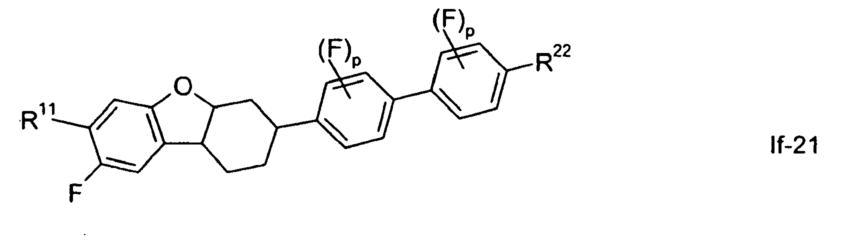 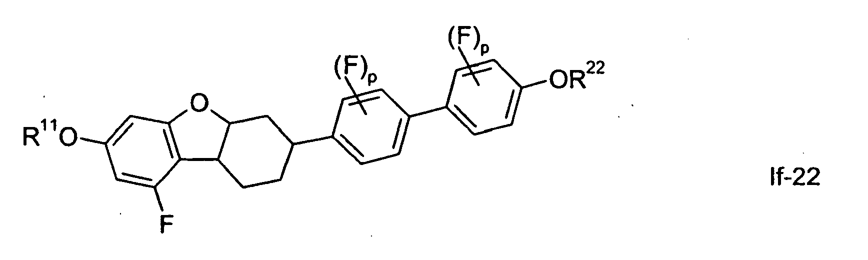 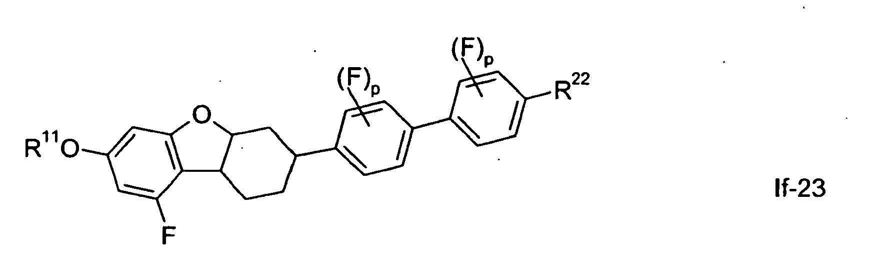 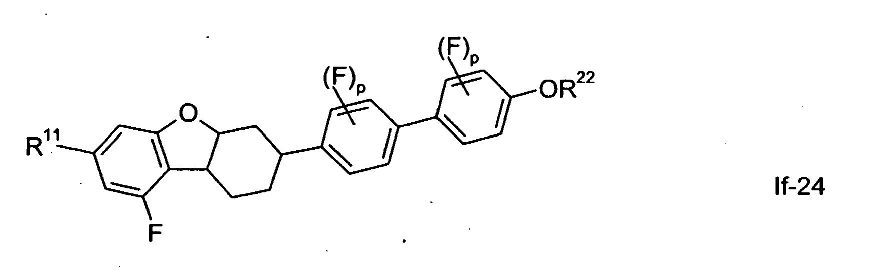 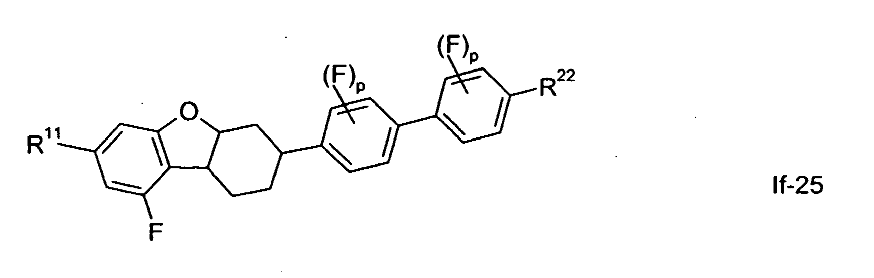 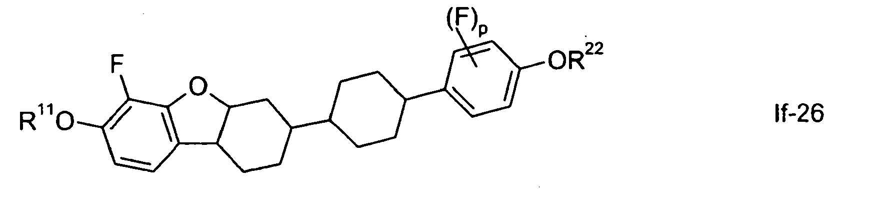 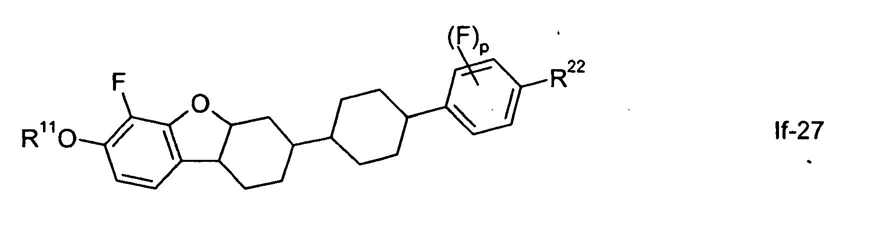 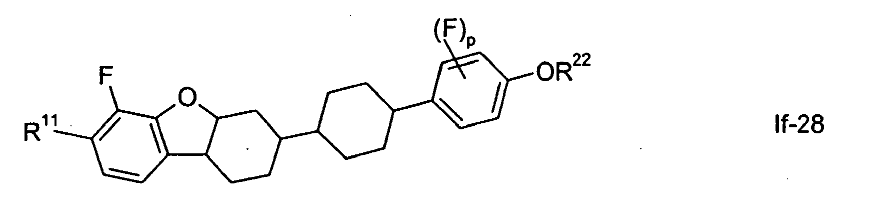 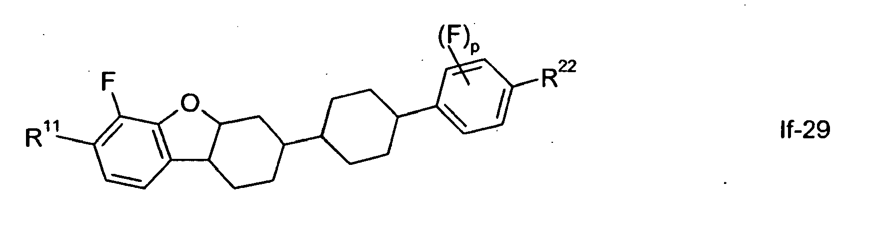 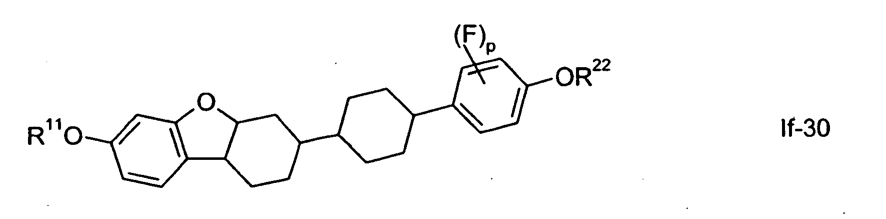 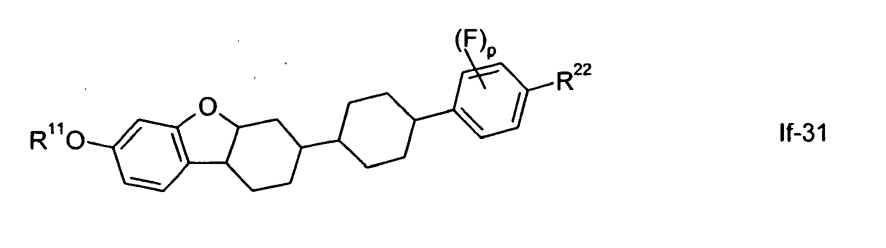 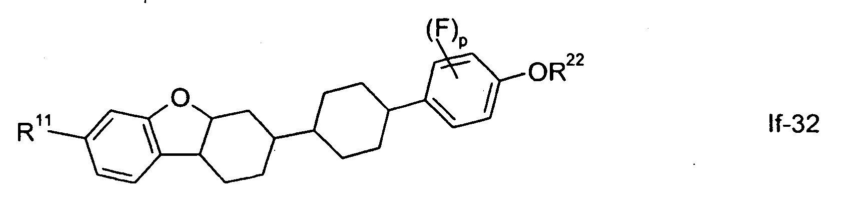 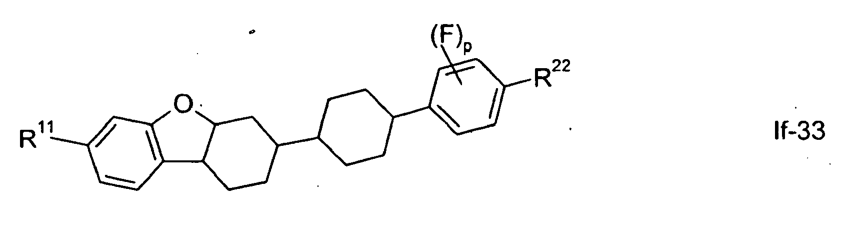 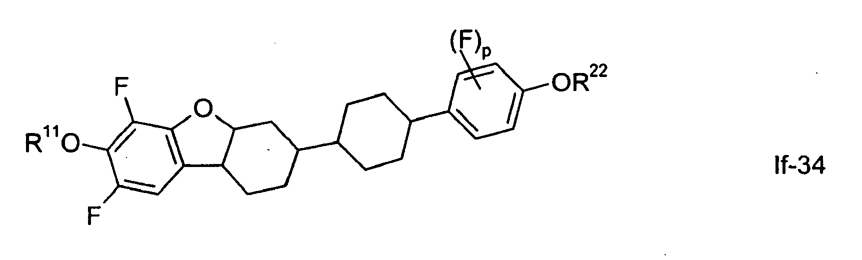 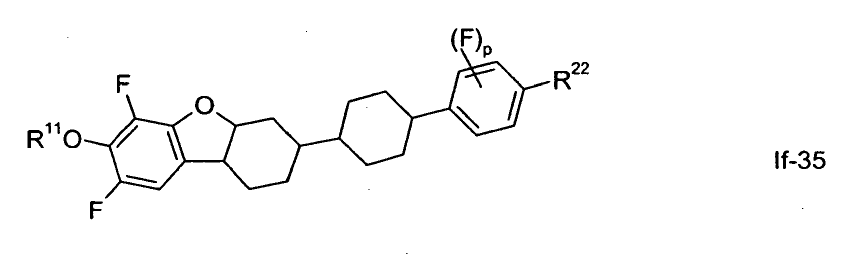 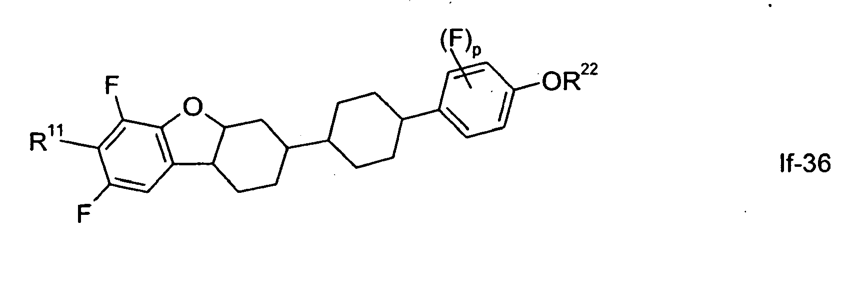 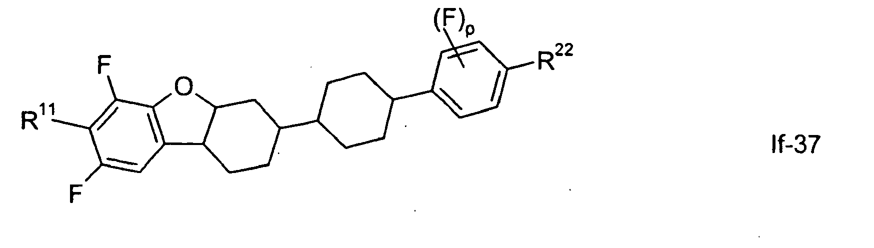 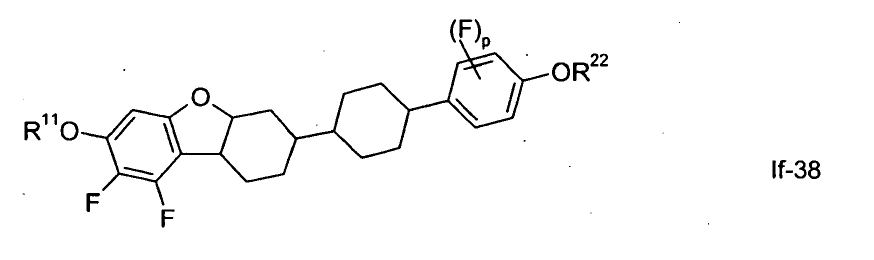 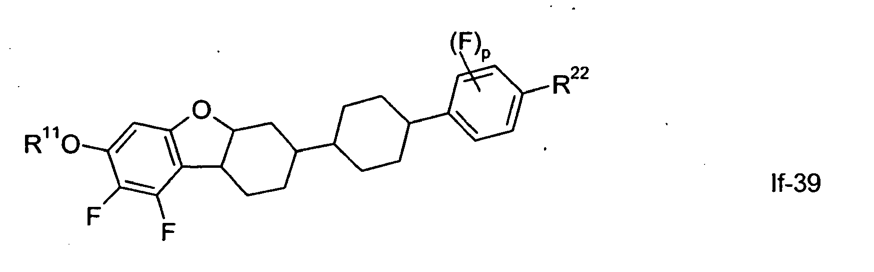 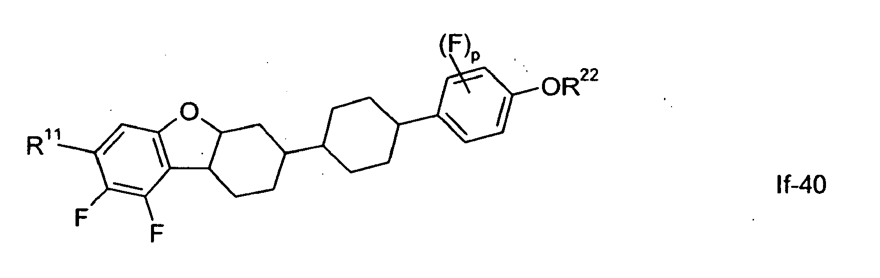 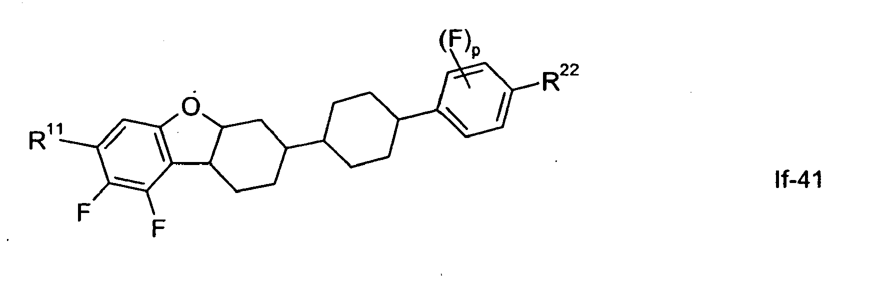 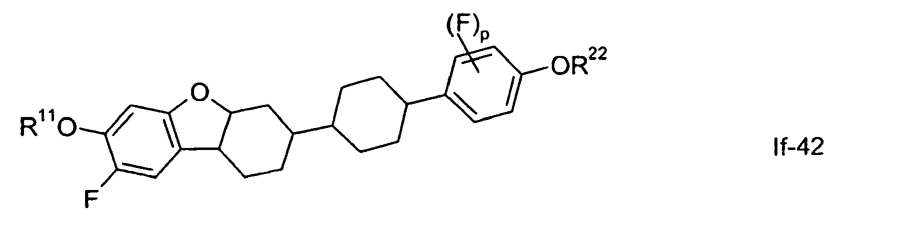 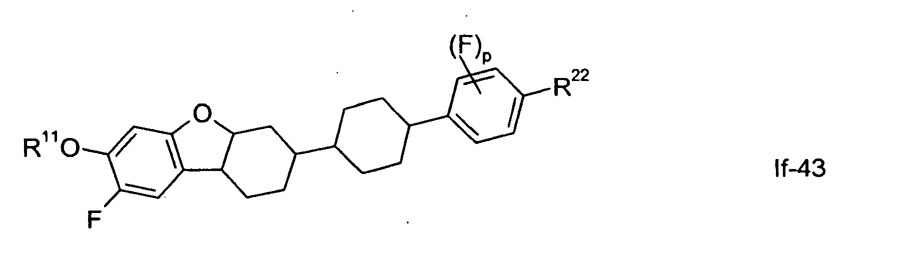 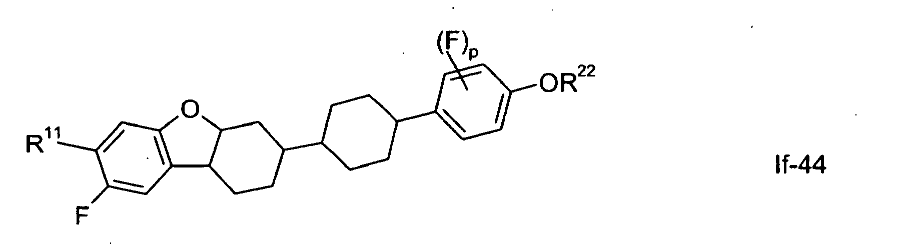 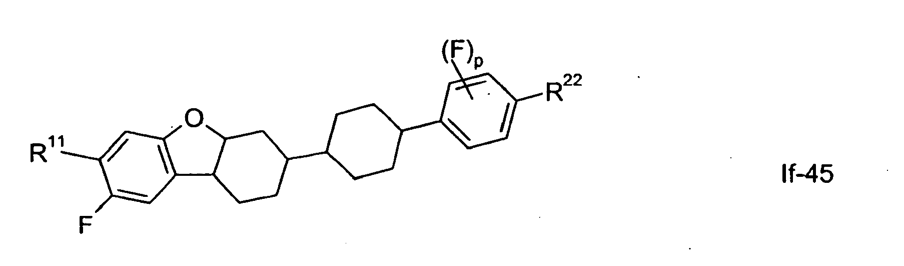 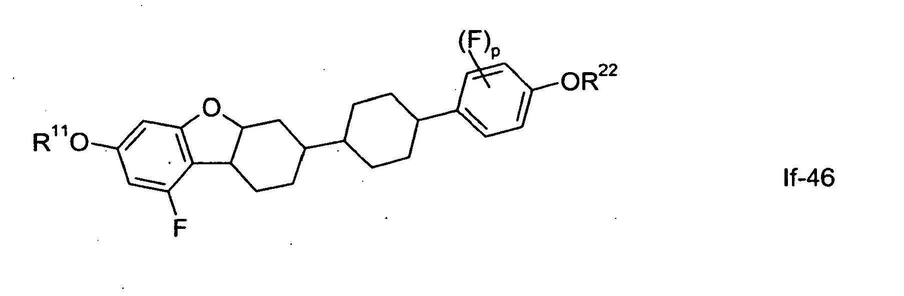 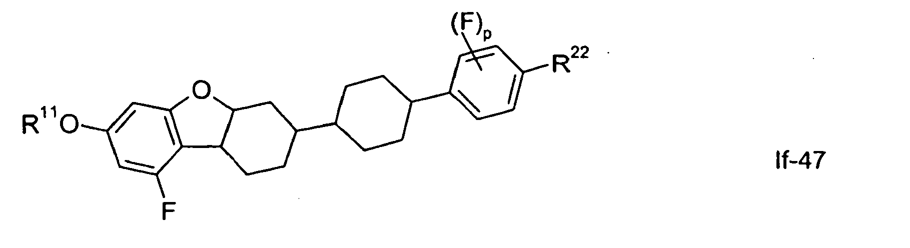 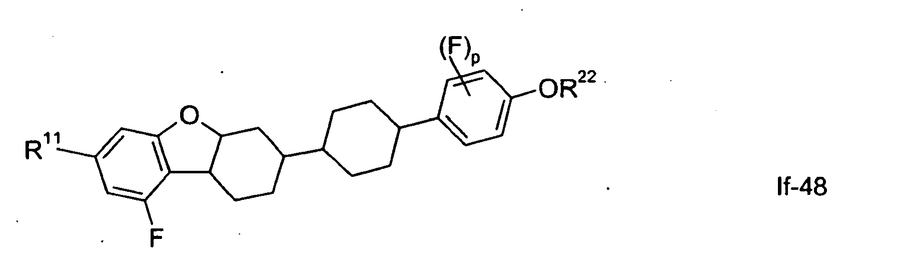 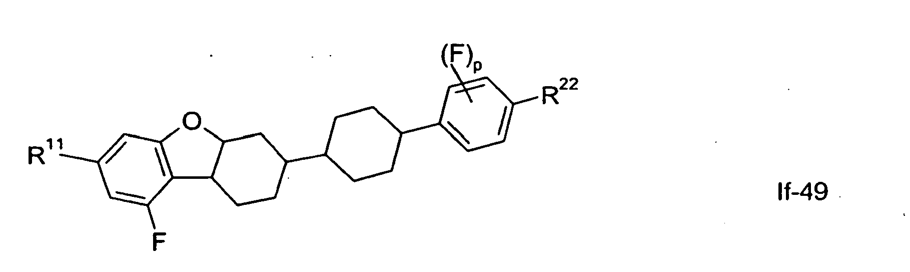 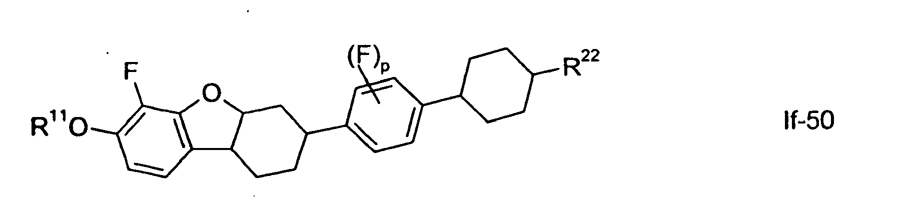 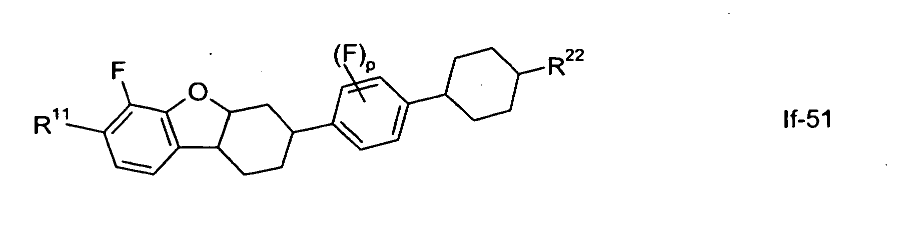 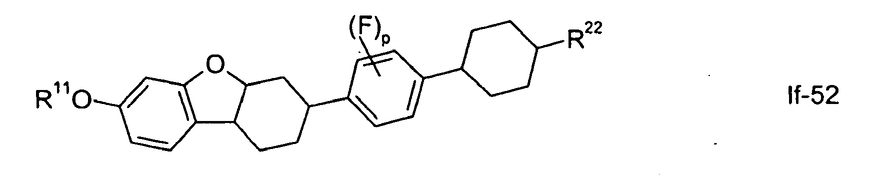 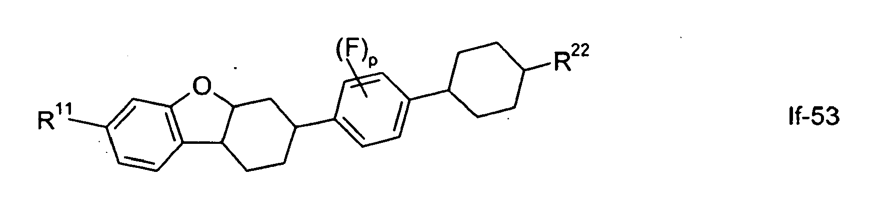 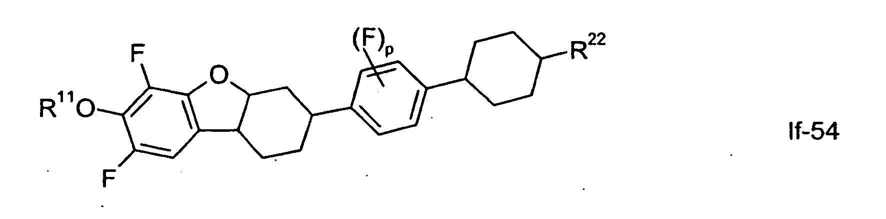 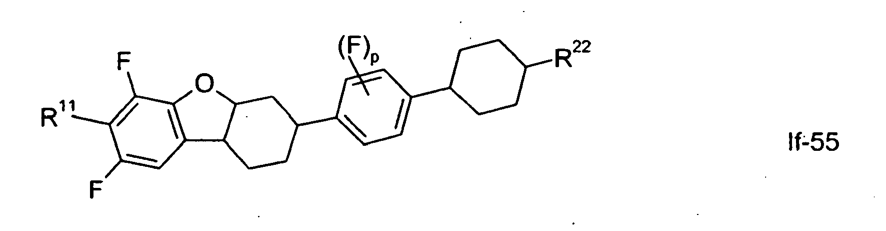 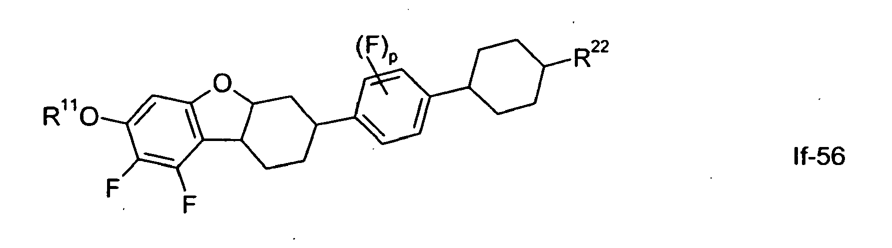 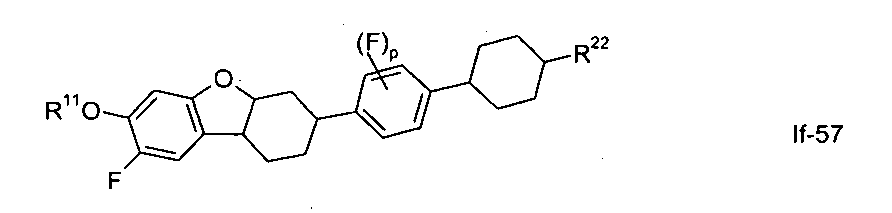 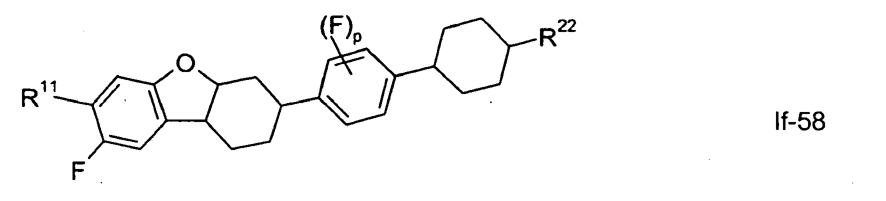 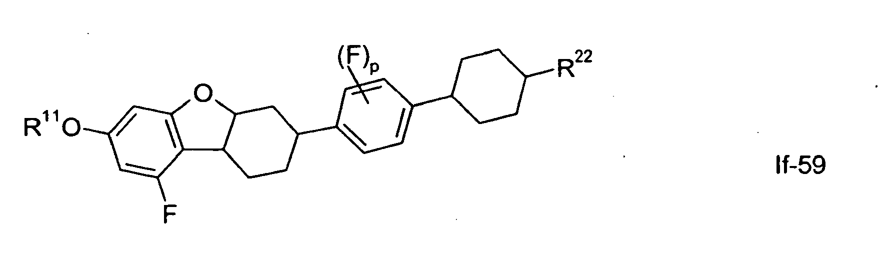 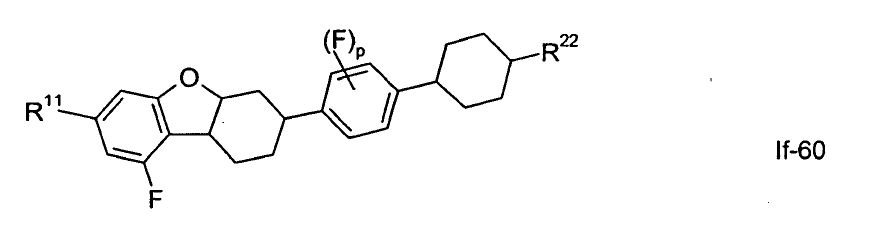 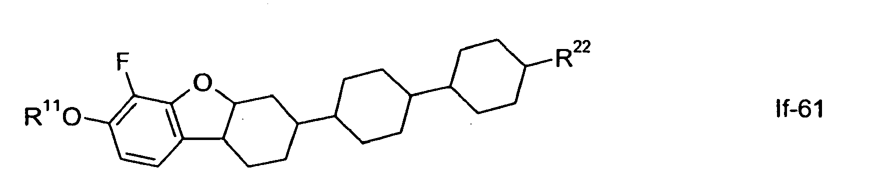 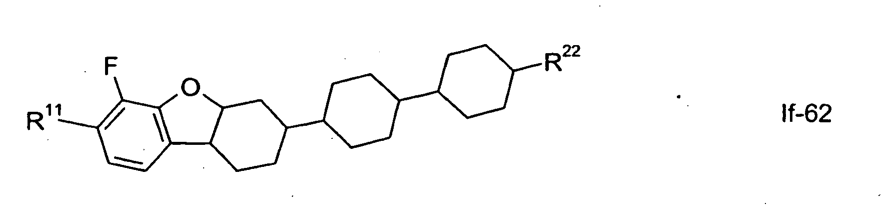 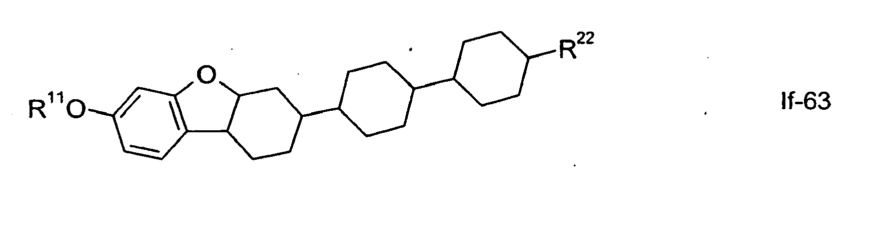 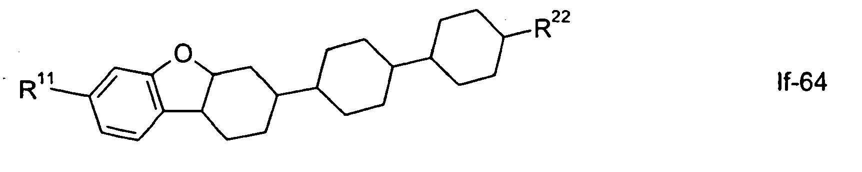 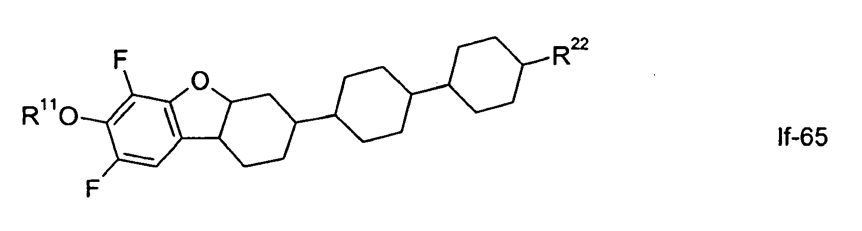 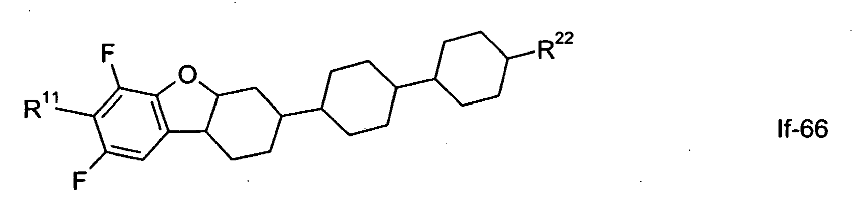 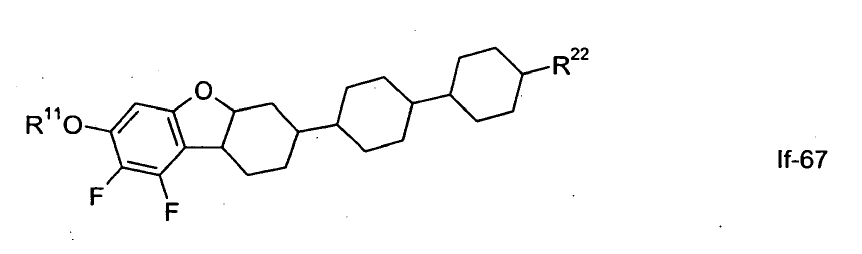 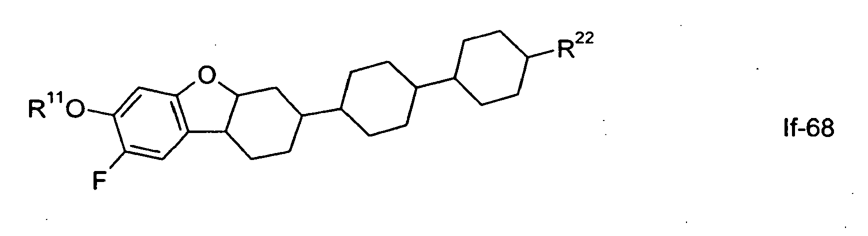 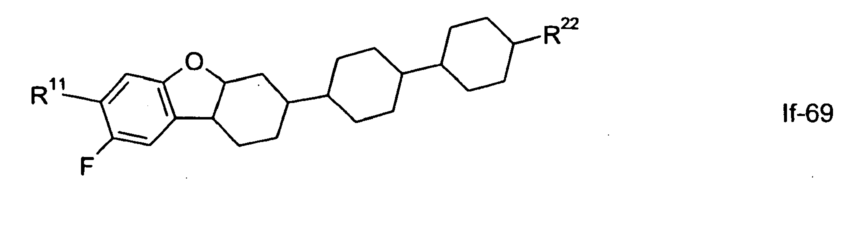 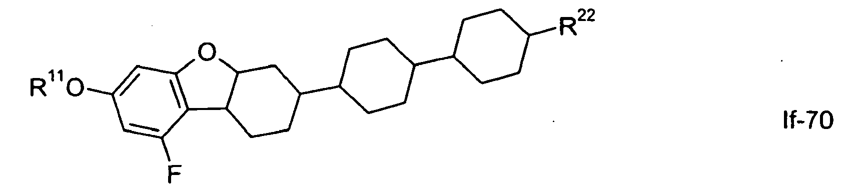 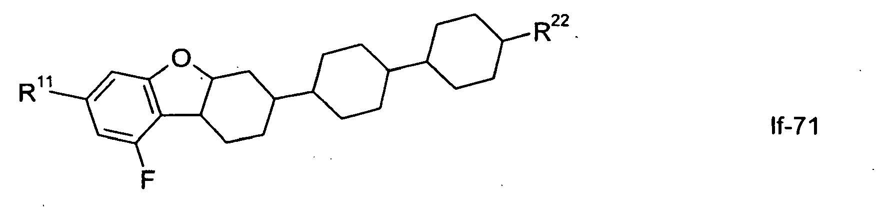 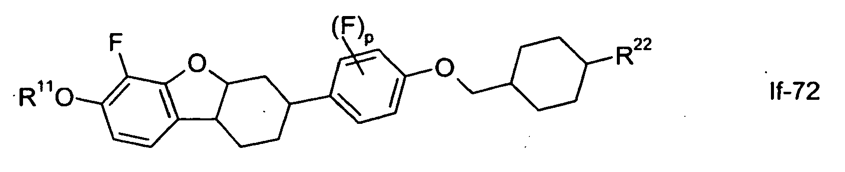 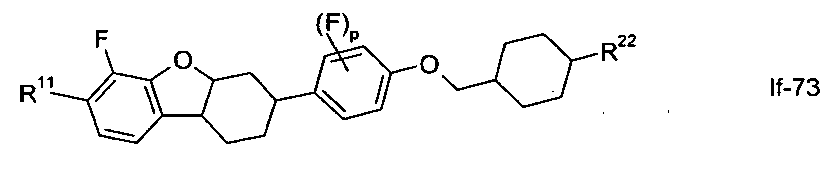 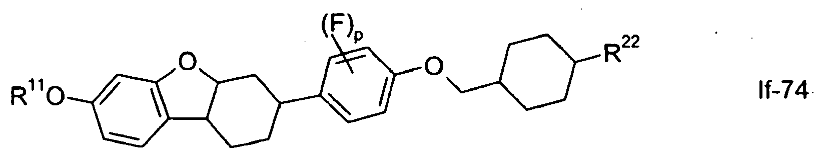 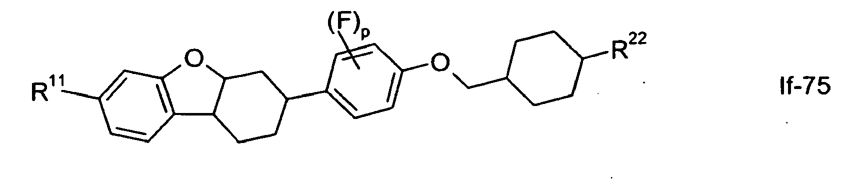 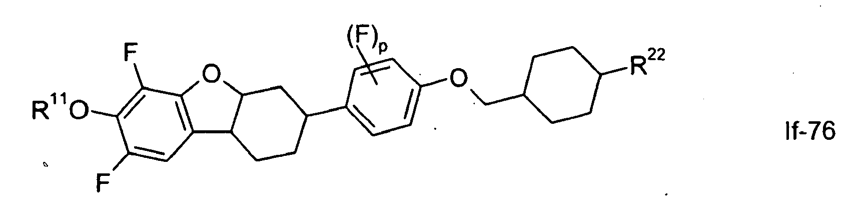 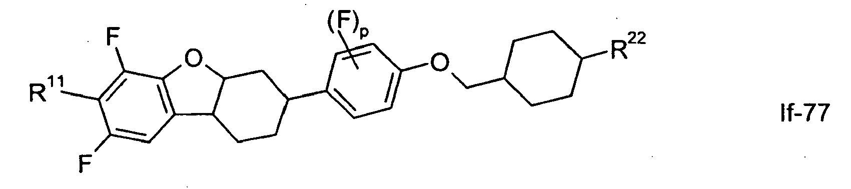 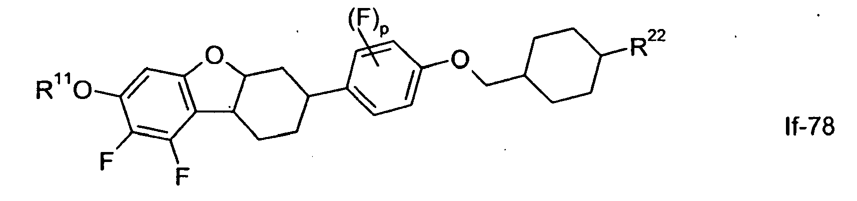 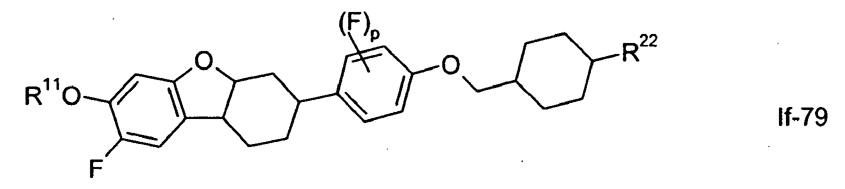 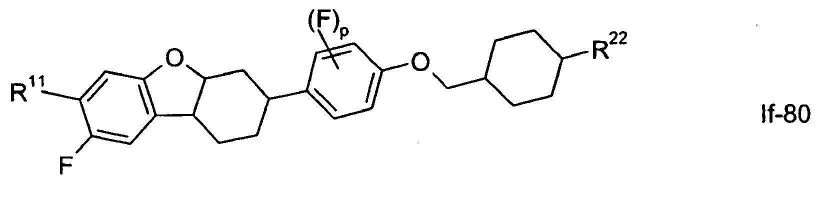 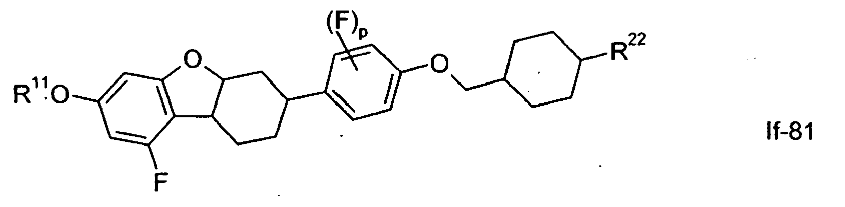 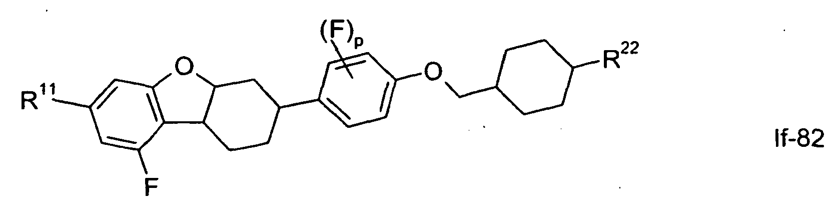 worin
R¹¹, R²² und p die gleichen Bedeutungen wie oben definiert besitzen. Soweit p in den Formeln mehrmals vorkommt, kann es jeweils die gleiche oder verschiedene Bedeutung aufweisen.

Unter den Verbindungen der Formeln If-1 bis If-82 sind die Verbindungen der Formeln mit mindestens einem 1,4-Cyclohexylen-Ring am meisten bevorzugt, insbesondere mit zwei Cyclohexylenringen (If-61 bis If-71) oder mit einem Cyclohexylenring direkt am Grundkörper (If-26 bis If-49). Besonders bevorzugt sind Verbindungen der Formel If-61.

In den Unterformeln der Formeln Ia- bis Ih bedeutet der Molekülteil 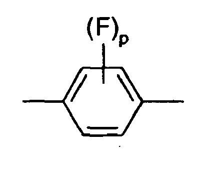 bevorzugt einen Molekülteil der Formeln

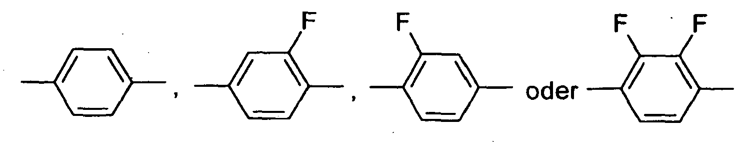

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Der 1,4-substituierte Cyclohexylring der Formel 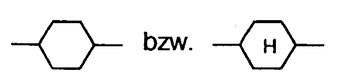 ist in den erfindungsgemäßen Verbindungen und in den übrigen Komponenten für flüssigkristalline Medien bevorzugt trans-konfiguriert, d.h. die beiden Substituenten befindend sich in der thermodynamisch bevorzugten Sesselkonformation beide in äquatorialer Position.

### Synthese der Verbindungen:

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, dass man sie aus dem Reaktionsgemisch nicht isoliert, sondern sofort weiter zu den Verbindungen der allgemeinen Formel I umsetzt.

Die Synthesen verschiedener erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten. Die beschriebenen Reaktionstypen sind als literaturbekannt anzusehen.

Die Verbindungen der Formel I umfassen alle acht Stereoisomere, die aus den möglichen Konfigurationen des Hexahydrobenzofuranrings bezüglich der Positionen 3, 4a und 9b des Ringsystems der allgemeinen Formel 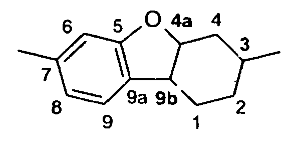 hervorgehen:

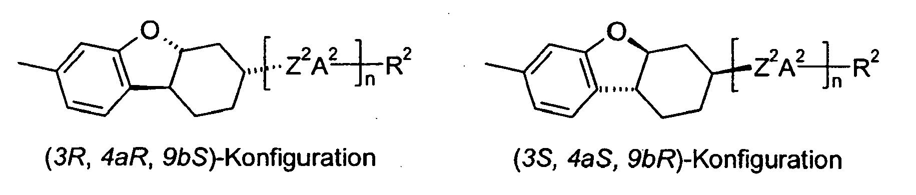

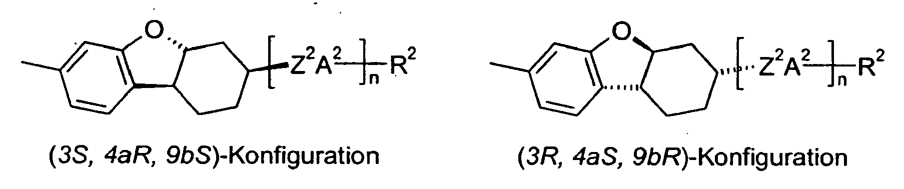

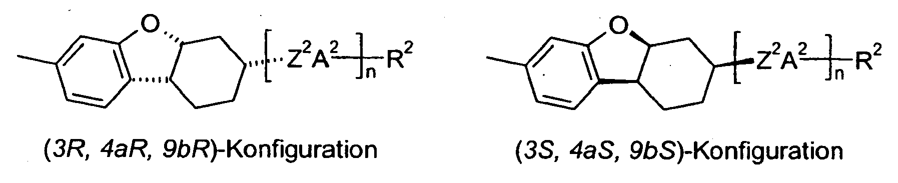

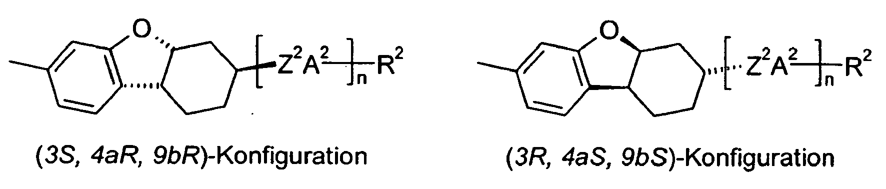

Besonders bevorzugt sind die Stereosiomere in denen eine *trans-*Verknüpfung des *O*-heterocyclischen Ringsystems mit dem Cyclohexanring vorliegt und gleichzeitig der Substituent an C-3 *trans-*ständig zum Substituenten an C-9b angeordnet ist (der Furanring gilt dabei als Substituent des Cyclohexanrings). Dies gilt für die besonders bevorzugten Stereoisomere mit der relativen (*3R**, *4aR* 9bS**)-Konfiguration. Der Stern steht für die relative Konfiguration, die gleich bedeutend mit den beiden spiegelbildlichen, absoluten Konfigurationen ist.

Durch diese Stereochemie erhält das Ringsystem eine flache Geometrie und das gesamte Molekül eine gestrecktere Form.

Ein Teilaspekt der Erfindung sind Verfahren zur Herstellung der erfindungsgemäßen Verbindungen der Formel 1. Die Verfahren unterliegen einer gemeinsamen Synthesestrategie, die sich aus dem Folgenden erklärt.

Ein bevorzugtes Verfahren zur Herstellung von Verbindungen des Typs **1**, die analog zu Verbindungen der Formel I sind, ist in **Schema 1** dargestellt. Die Synthese kann durch die Wahl geeigneter Edukte **3** und **4** an die jeweils gewünschten Verbindungen der Formel **1** angepasst werden. Diese Ausgangsmaterialien sind entweder kommerziell erhältlich, oder sie können, bereits veröffentlichten Verfahren folgend, synthetisiert werden.

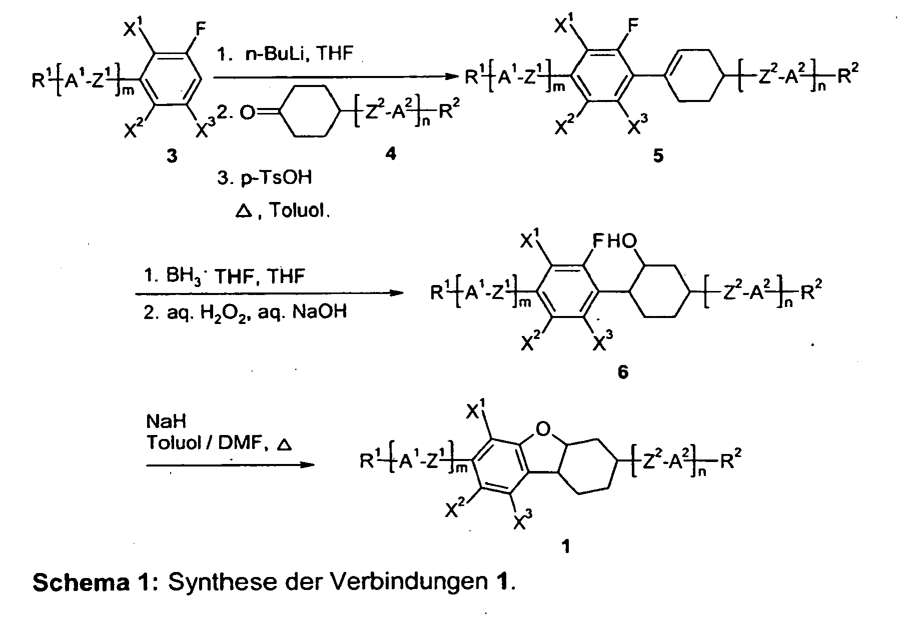

Das Lithiumorganyl der Verbindung **3** addiert an das Cyclohexylketon **4.** Der resultierende Alkohol dehydratisiert bei Behandlung mit *p*-TsOH bereitwillig zum Cyclohexen **5** analog P. Kirsch et al., Angew. Chem. Int. Ed. (1998), 37, 484-489. Hydroborierung-Oxidation der Doppelbindung mit BH₃-THF-Komplex liefert den sekundären Alkohol **6.** Das Alkoholat der Verbindung **6** cyclisiert unter den angegebenen Reaktionsbedingungen zur Zielverbindung **1**. Durch laborübliche Trenn- und Reinigungsverfahren werden die besonders bevorzugten (*3R*,4aR*, 9bS**)-konfigurierten Stereoisomere der Verbindungen der Formel 1 erhalten.

Ebenfalls bevorzugt sind Syntheseverfahren über die Ketozwischenstufe **11,** da Verbindung **11** im Folgenden unterschiedlich funktionalisiert werden kann. Die Synthese des Ketons **11** erfolgt ebenfalls über die in **Schema 1** dargestellte Reaktionssequenz unter Verwendung von 1,4-Cyclohexandion-monoethylenketal **7** als Ausgangsmaterial (vgl. **Schema 2**). Nach der Ringschlussreaktion zu **10** wird die Ketalschutzgruppe im Sauren abgespalten (vgl. Kirsch et al.).

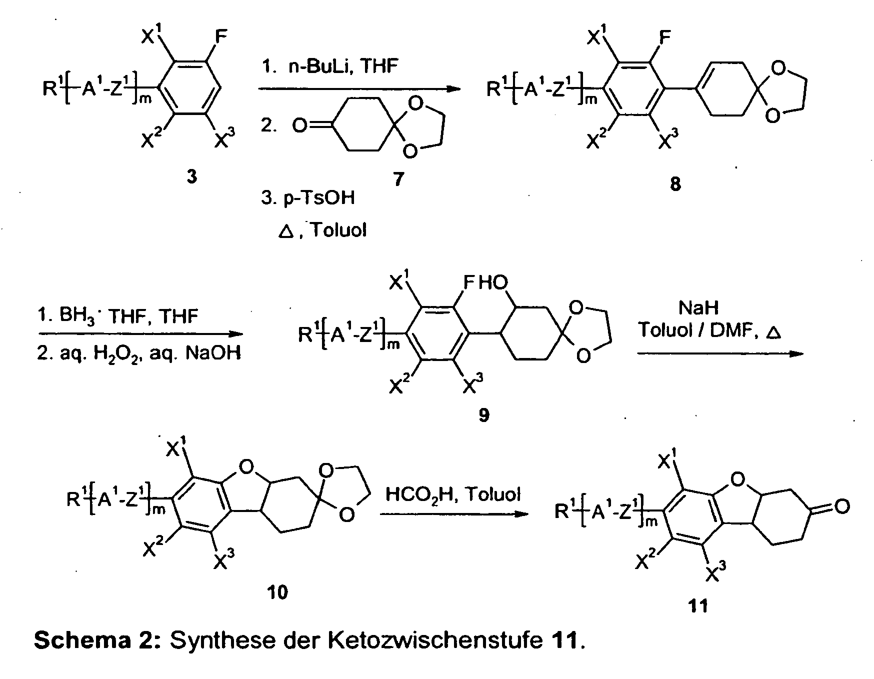

Die weitere Funktionalisierung von **11** kann dann in unterschiedlicher Weise erfolgen (vgl. **Schema 3** und **Schema 4**).

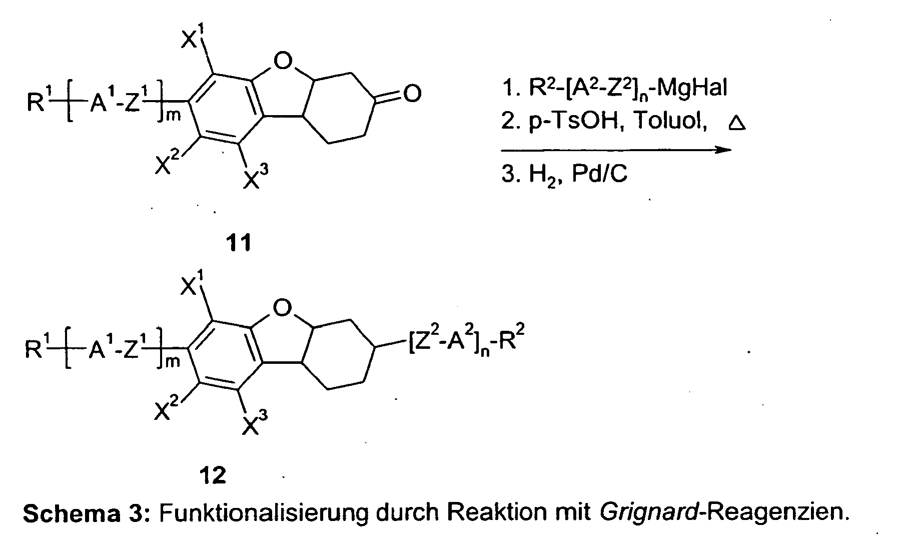

Die Umsetzung von **11** mit einem geeigneten Grignard-Reagenz (vgl. **Schema 3**) oder einem Lithiumorganyl ergibt nach Dehydratisierung des erhaltenen Alkohols und abschließender katalytischer Hydrierung die Zielverbindung **12.** Die bevorzugten (*3R**,*4aR**, *9bS**)-konfigurierten Stereoisomere der Verbindung **12** werden über laborübliche Trennverfahren erhalten.

Weiterhin sind *Wittig*-Olefinierungsreaktionen zur Derivatisierung geeignet (vgl. **Schema 4**). Nach Reaktion mit *Wittig*-Reagentien und anschließender Hydrierung der gebildeten Doppelbindung, wird wiederum ein trennbares Isomerengemisch der Zielverbindung **13** gebildet.

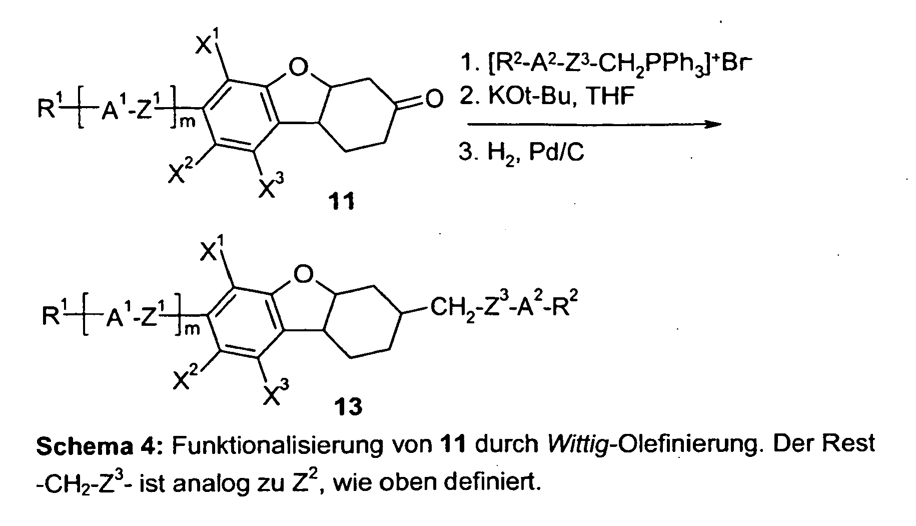

Besonders bevorzugt sind auch Funktionalisierungen über die Zwischenstufe **14,** welche diastereoisomerenrein in drei Schritten aus **11** zugänglich ist (vgl. **Schema 5**).

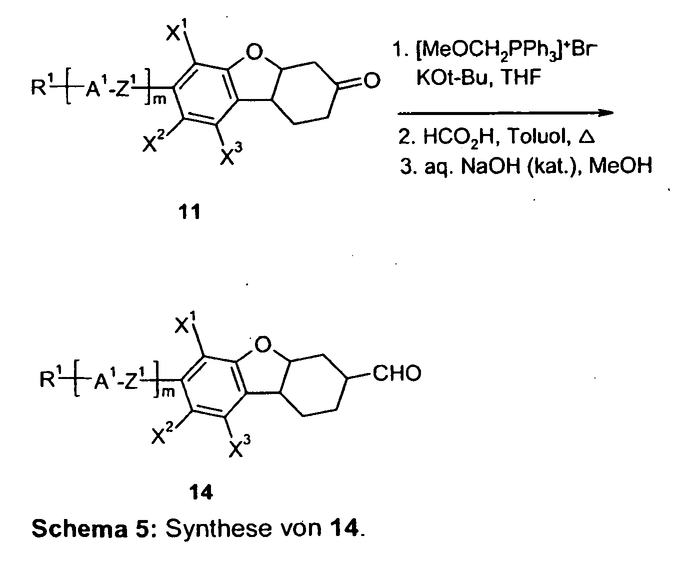

Die Kettenverlängerung ausgehend von **14** kann dann wiederum über die Addition einer Grignard-Verbindung bzw. durch Olefinierung mit einem *Wittig*-Reagenz eingeleitet werden (vgl. **Schema 6**).

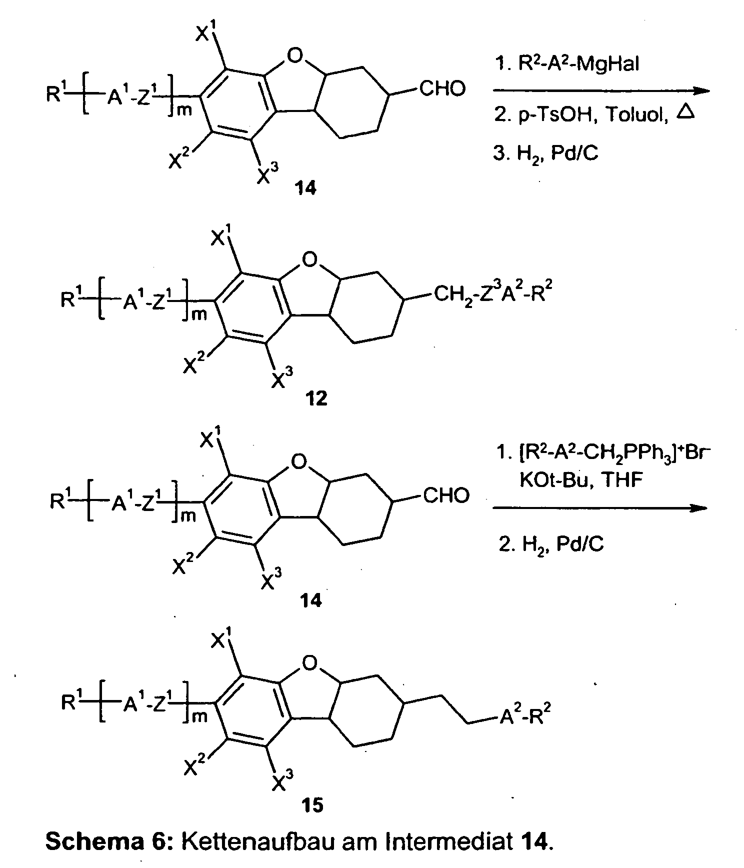

Die dargestellten Reaktionsschemata sind nur als beispielhaft aufzufassen. Der Fachmann kann entsprechende Variationen der vorgestellten Synthesen vornehmen sowie auch andere geeignete Synthesewege beschreiten, um Verbindungen der Formel I zu erhalten. So kann zum Beispiel die Verbindung der Formel **7** eine andere Acetal-Schutzgruppe der Formel >C(OAlkyl)₂ oder >C(cyclo-OCH₂CH₂CH₂O) besitzen.

Ein gemeinsamer Aspekt der Verfahren zur Herstellung von Verbindungen der Formel I ist daher, dass es einen Verfahrenschritt umfasst, worin eine Fluorbenzol-Verbindung in ortho-Stellung mit einer Cyclohexanonverbindung kondensiert wird. Im Speziellen handelt es sich dabei um eine Fluorbenzolverbindung der Formel **3** (Schema 1, 2) sowie um Cyclohexanonverbindungen der Formeln **4** oder **7** gemäß ihrer wie oben verallgemeinerten Definition. Für das Verfahren können ebenso Vorstufen von I Gegenstand der beschriebenen Verfahrensschritte sein, welche erst später an den variablen Substituenten der Ringe derivatisiert werden. Das Verfahren zeichnet sich weiterhin dadurch aus, dass es einen Verfahrensschritt umfasst, wobei eine 1-(2-Halophenyl)cyclohexen-verbindung an der Doppelbindung des Cyclohexens hydroboriert wird. Bei diesen Verbindungen handelt es sich bevorzugt um die Verbindungen der Formeln **5** oder **8**, wobei in **8** die Acetalgruppe wie für Formel **7** variiert werden kann. Das Verfahren beinhaltet weiterhin einen Verfahrensschritt, wobei die gebildete 2-(2-Halophenyl)cyclohexanolverbindung zu einem Tetrahydrodibenzofuranderivat cyclisiert wird.
Die Cyclisierung erfolgt unter der Wikung einer Base, vorzugsweise mittels einer starken Base wie z. B. Natrium- oder Kaliumhydrid. Die Umsetzung erfolgt zwischen 20 und 140°C, je nach Lösungmittel und Reaktionsgeschwindigkeit.
Weitere Einzelheiten zum Verfahren sind den Beispielen zu entnehmen, deren Parameter respräsentativ für das erfindungsgemäße Verfahren sind. Der Fachmann wird in der Lage sein, einzelne Reaktionsbedingungen zu verallgemeinern oder auf den Einzelfall anzupassen.

Bevorzugt handelt es sich bei den Edukten um 2-Fluorbenzolderivate, besonders bevorzugt um 2,3-Difluorderivate. Gegebenfalls wird das unmittelbare Verfahrensprodukt weiter zu den gewünschten flüssigkristallinen oder mesogenen Verbindungen derivatisiert.

Wie bereits erwähnt, können die Verbindungen der allgemeinen Formel I in flüssigkristallinen Medien verwendet werden.

Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

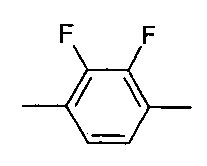

In den Verbindungen der Gruppe B, die mit den Teilformeln (IIb), (IIIb), (IVb), (Vb) und (Vlb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der Teilformeln (IIc), (IIIc), (IVc), (Vc) und (VIc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A:
   0 bis 90 %, vorzugsweise 20 bis 90 %, insbesondere 30 bis 90 %.
Gruppe B:
   0 bis 80 %, vorzugsweise 10 bis 80 %, insbesondere 10 bis 70 %.
Gruppe C:
   0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Weiterhin bevorzugt sind Medien, enthaltend mehr als 40 %, insbesondere 45 bis 90 % an erfindungsgemäßen Verbindungen der Formel I. Die Medien enthalten vorzugsweise eine, zwei, drei, vier oder fünf erfindungsgemäße Verbindungen der Formel I.

Beispiele für die Verbindungen der Formeln (II), (III), (IV), (V) und (VI) sind die nachstehend aufgeführten Verbindungen: 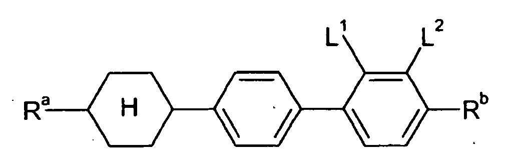 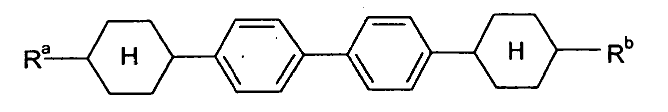 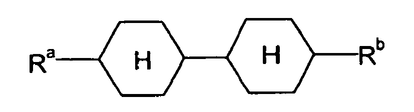 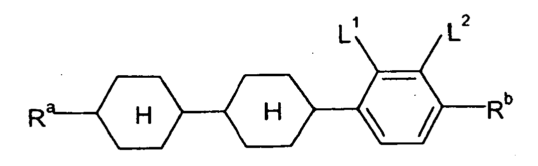 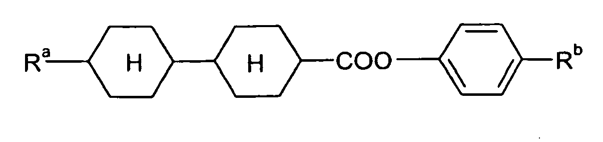 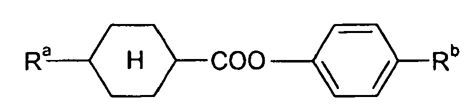 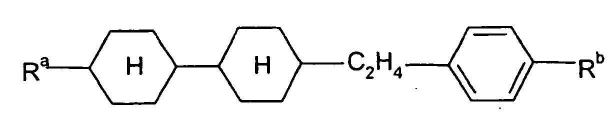 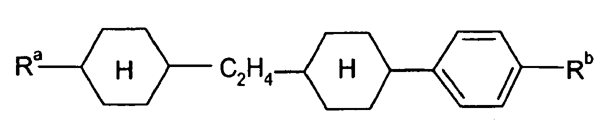 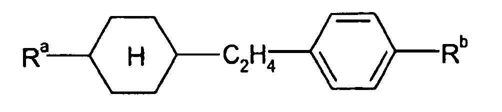 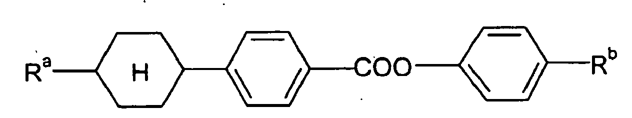 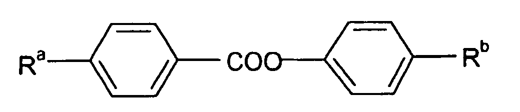 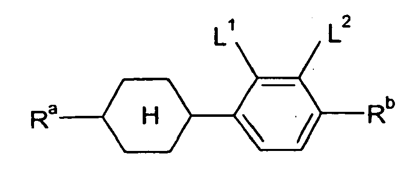 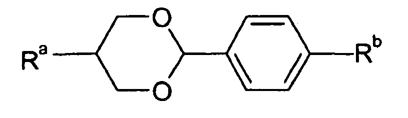 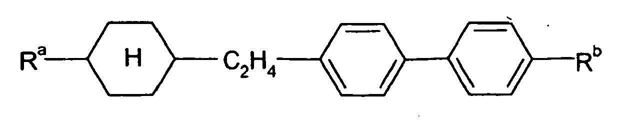 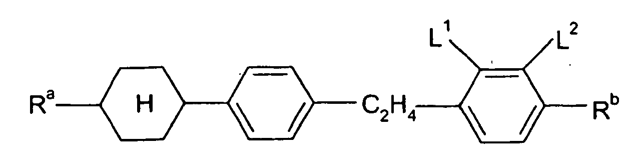 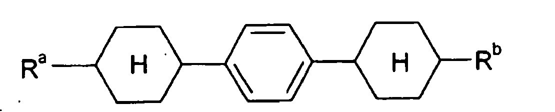 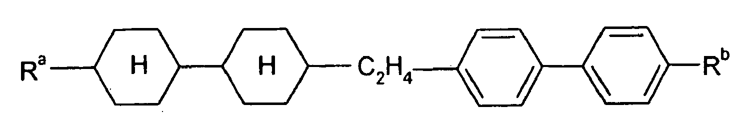 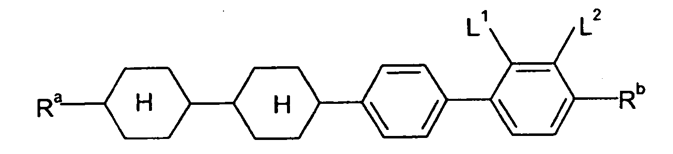 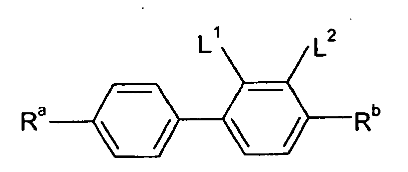 mit R^{a}, R^{b} unabhängig voneinander -CₚH₂ₚ₊₁ oder -OCₚH₂ₚ₊₁ und p = 1, 2, 3, 4, 5, 6, 7 oder 8 sowie L¹, L² unabhängig voneinander -H oder -F, 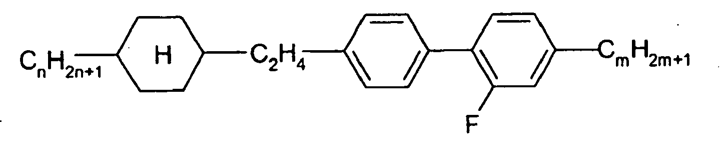 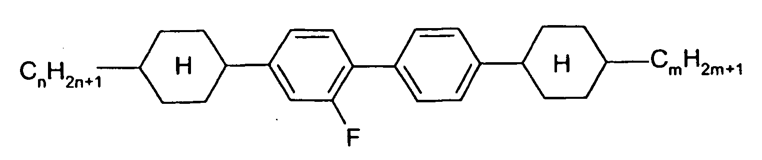 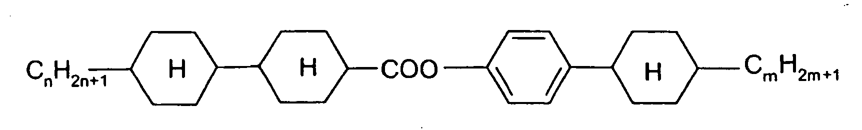 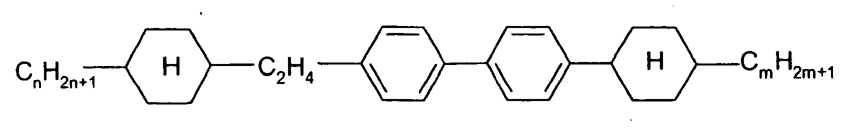 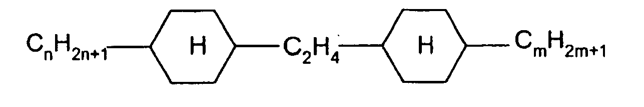 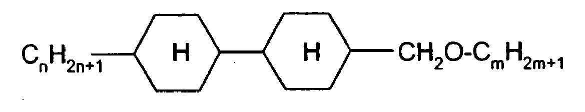 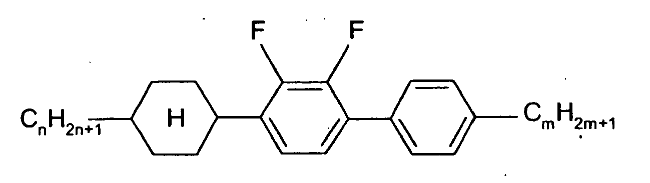 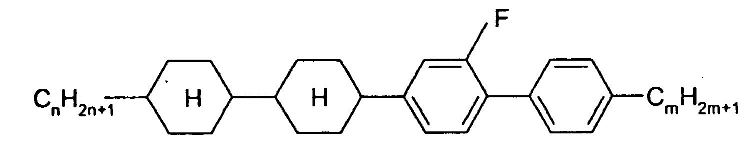 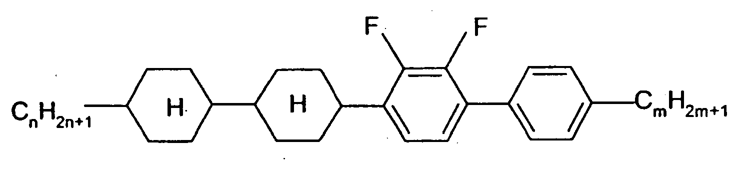 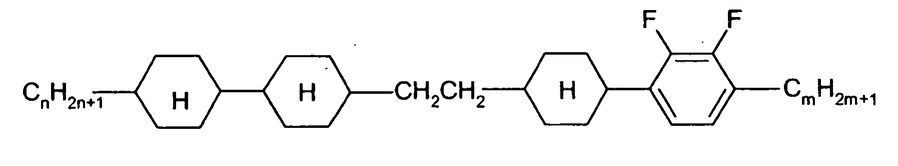 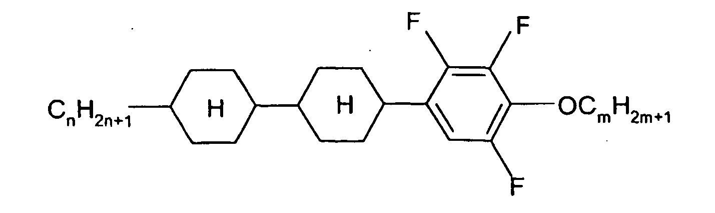 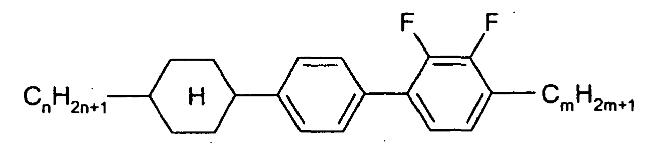 mit m, n unabhängig voneinander 1, 2, 3, 4, 5, 6, 7 oder 8.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Die Erfindung wird im Folgenden anhand von Ausführungsbeispielen näher erläutert, ohne dadurch jedoch eingeschränkt werden zu sollen.

Neben den üblichen und wohlbekannten Abkürzungen werden folgende Abkürzungen verwendet:
K: Kristalline Phase; N: Nematische Phase; Sm: Smektische Phase; I: Isotrope Phase. Die Zahlen zwischen diesen Symbolen geben die Übergangstemperaturen der betreffenden Substanz wieder. Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet Δn die optische Anisotropie (589 nm, 20 °C) und Δε die dielektrische Anisotropie (1 kHz, 20 °C). Die dielektrische Anisotropie Δε wird bei 20°C und 1 kHz bestimmt. Die optische Anisotropie Δn wird bei 20°C und einer Wellenlänge von 589,3 nm bestimmt.

Die Δε- und Δn-Werte, der Klärpunkt (Clp.) sowie die Rotationsviskosität (γ₁) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für Δε) bzw. ZLI-4792 (für Δn, Clp., γ₁) bestehen (Mischungen Fa. Merck KGaA, Darmstadt).

Nachstehend bedeuten die Abkürzungen:
- MTBE: Methyl-t-butylether
- THF: Tetrahydrofuran
- DMF: Dimethylformamid
- i. Vak.: Im Vakuum (ca. 10⁻² bar)
- ges.: gesättigt
- n-BuLi: n-Butyllithium, Lösung in Hexan

### Beispiele

Die Ausgangssubstanzen können nach allgemein zugänglichen Literaturvorschriften oder käuflich erhalten werden. Die beschriebenen Reaktionen sind literaturbekannt.

### 1. ((±)-(3R*, 4aR*, 9bS*)-7-Ethoxy-6-fluor-3-propyl-1,2,3,4,4a,9b-hexahydro-dibenzofuran

### 1.1 8-(4-Ethoxy-2,3-difluor-phenyl)-1,4-dioxa-spiro[4.5]decan-7-ol

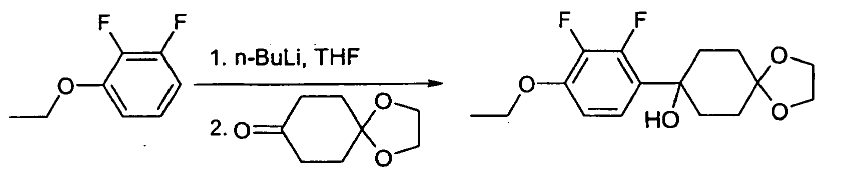

270.2 g (1.70 mol) 2,3-Difluorethoxybenzol werden in 1200 ml THF vorgelegt und bei -70°C mit 1100 ml (1.75 mol) n-BuLi (15% Lsg. in Hexan) versetzt. Nach 1 h bei dieser Temperatur wird eine Lösung von 270.2 g (1.70 mol) 1,4-Cyclohexandion-monoethylenketal in 800 ml THF zudosiert, und der Ansatz wird 1 h gerührt. Die Reaktionsmischung wird auf 0 °C erwärmt und mit 4 N HCl hydrolysiert. Die Lösung wird mit MTBE extrahiert, und die vereinigten organischen Phasen werden mit ges. Natriumchloridlösung gewaschen und mit Natriumsulfat getrocknet. Das nach Entfernen der Lösungsmittel unter vermindertem Druck verbleibende Rohprodukt (609.8 g rotbraunes Öl) wird direkt für die nächste Umsetzung verwendet.

### 1.2 8-(4-Ethoxy-2,3-difluor-phenyl)-1,4-dioxa-spiro[4.5]dec-7-en

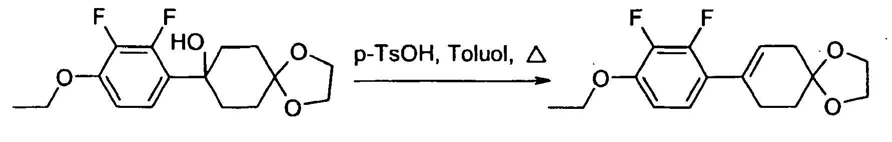

609 g (ca. 1.94 mol) rohes 8-(4-Ethoxy-2,3-difluoro-phenyl)-1,4-dioxa-spiro[4.5]decan-7-ol werden in 2000 ml Toluol zusammen mit 220 ml (3.93 mol) Ethylenglycol unter Zusatz von 36.1 g (0.19 mol) *p-*Toluolsulfonsäure-monohydrat 2 h am Wasserabscheider erhitzt. Nach dem Abkühlen wird der Ansatz nacheinander mit Wasser, ges. Natriumhydrogencarbonatlösung und ges. Natriumchloridlösung gewaschen. Die Lösung wird mit Natriumsulfat getrocknet und vollständig konzentriert. Das Rohprodukt (507 g orange Öl) wird aus Ethanol bei -20 °C kristallisiert. Auf diese Weise wird 8-(4-Ethoxy-2,3-difluor-phenyl)-1,4-dioxa-spiro[4.5]dec-7-en als gelber Feststoff erhalten.

### 1.3 8-(4-Ethoxy-2,3-difluor-phenyl)-1,4-dioxa-spiro[4.5]decan-7-ol

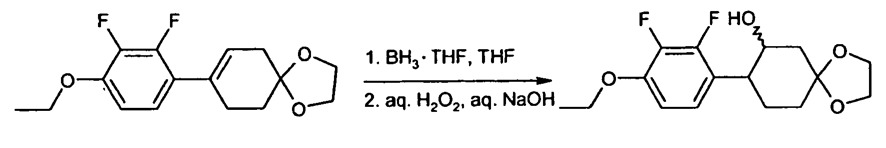

Eine Lösung von 320.0 g (1.08 mol) 8-(4-Ethoxy-2,3-difluor-phenyl)-1,4-dioxa-spiro[4.5]dec-7-en in 3000 ml THF wird bei -7 °C mit 1400 ml (1.40 mol) Boran-THF-Komplex (1 M Lösung) versetzt, und die Reaktionsmischung wird 3 h bei Raumtemperatur gerührt. Der Ansatz wird nacheinander mit 262 ml (4.50 mol) Ethanol, 650 ml (2.6 mol) wässriger Natriumhydroxidlösung (4 M) und 360 ml (4.11 mol) wässriger Wasserstoffperoxid-Lsg. (35 %) versetzt, wobei die Innentemperatur 47 °C nicht übersteigt (Eisbad). Nach beendeter Zugabe wird 2 h zum Rückfluss erwärmt und die Lösung nach dem Abkühlen auf Wasser gegeben und intensiv gerührt. Die organische Phase wird abgetrennt und die wässrige Phase mit MTBE extrahiert. Die vereinigten organischen Phasen werden mit ges. Natriumchloridlösung gewaschen und mit Natriumsulfat getrocknet. Die Lösung wird vollständig konzentriert, und das Rohprodukt (336 g gelbes Öl) wird säulenchromatographisch (SiO₂, Dichlormethan : MTBE = 8 : 2) gereinigt. 8-(4-Ethoxy-2,3-difluor-phenyl)-1,4-dioxa-spiro[4.5]decan-7-ol wird als schwach gelbes Öl erhalten.

### 1.4 (±)-(4aR*, 9bS*)-7-Ethoxy-6-fluor-1,4,4a,9b-tetrahydro-2H-spiro[dibenzo[b,d]furan-3,2'-[1,3]dioxolan]

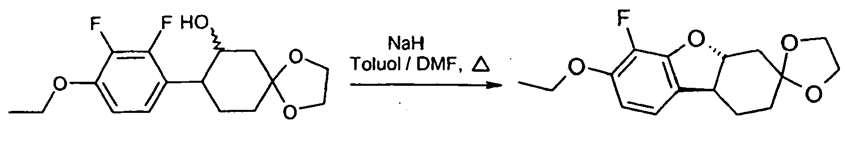

50.0 g (1.25 mol) Natriumhydrid (60% Suspension in Mineralöl) werden wiederholt mit *n*-Pentan gewaschen und in 3000 ml Toluol suspendiert. Die Suspension wird auf 90 °C erhitzt, und eine Lösung von 145.0 g (0.46 mol) 8-(4-Ethoxy-2,3-difluor-phenyl)-1,4-dioxa-spiro[4.5]decan-7-ol in 700 ml DMF wird langsam zudosiert. Der Ansatz wird 30 h bei 90 °C gerührt und nach dem Abkühlen mit Wasser hydrolysiert. Die Mischung wird durch Zugabe 2 N Salzsäure neutralisiert, und die organische Phase wird abgetrennt. Die wässrige Phase wird mit Toluol extrahiert, und die vereinigten organischen Phasen werden mit ges. Natriumchloridlösung gewaschen. Die Lösung wird mit Natriumsulfat getrocknet und unter vermindertem Druck vollständig konzentriert. Das erhaltene Rohprodukt wird bei 5 °C aus Ethanol umkristallisiert. (±)-(*4aR**, *9bS**)-7-Ethoxy-6-fluor-1,4,4a,9b-tetrahydro-2*H*-spiro-[dibenzo[b,d]furan-3,2'-[1,3]dioxolan] wird als farbloser Feststoff isoliert.

### 1.5 (±)-(4aR*, 9bS*)-7-Ethoxy-6-fluor-1,4,4a,9b-tetrahydro-2H-dibenzofuran-3-on

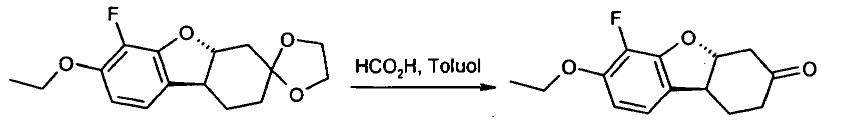

135.0 g (459 mmol) (±)-(*4aR*, 9bS**)-7-Ethoxy-6-fluor-1,4,4a,9b-tetrahydro-2*H*-spiro[dibenzo[b,d]furan-3,2'-[1,3]dioxolan] werden in 1800 ml Toluol gelöst und zusammen mit 550 ml (14.6 mol) Ameisensäure unter Zusatz von 10.0 ml (0.56 mol) Wasser intensiv gerührt. Nach 18 h wird die organische Phase abgetrennt, und die Ameisensäure mit Toluol extrahiert. Die vereinigten organischen Phasen werden nacheinander mit Wasser, ges. Natriumhydrogencarbonatlösung und ges. Natriumchloridlösung gewaschen und mit Natriumsulfat getrocknet. Das nach dem Entfernen des Lösungsmittels verbleibende Rohprodukt wird säulenchromatographisch (SiO₂, Toluol : Ethylacetat = 4 : 1) gereinigt. (±)-(*4aR*, 9bS**)-7-Ethoxy-6-fluor-1,4,4a,9b-tetrahydro-2H-dibenzofuran-3-on wird als farbloser Feststoff isoliert.

### 1.6 (±)-(4aR*, 9bS*)-7-Ethoxy-6-fluor-3-[1-methoxy-methyliden]-1,2,3,4,4a,9b-hexahydro-dibenzofuran

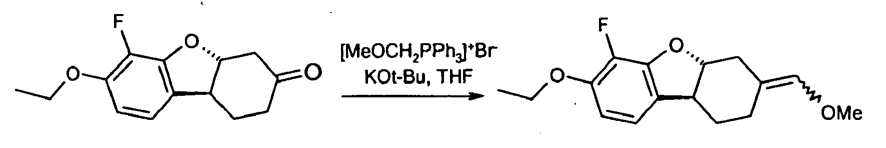

58.3 g (170 mmol) Methoxymethyltriphenylphosphoniumchlorid werden in 500 ml THF vorgelegt und bei 0 °C mit einer Lösung von 19.1 g (170 mmol) Kalium-*tert*-butylat in 200 ml THF versetzt. Nach 30 min bei dieser Temperatur wird 7-Ethoxy-6-fluor-1,4,4a,9b-tetrahydro-2H-dibenzofuran-3-on als Lösung in 300 ml THF zugegeben und der Ansatz 17 h bei Raumtemperatur gerührt. Die Mischung wird bei 0 °C mit Wasser versetzt und mit 2 N Salzsäure angesäuert. Der Ansatz wird mit MTBE extrahiert, und die vereinigten Extrakte werden mit ges. Natriumchloridlösung gewaschen. Die Lösung wird mit Natriumsulfat getrocknet und vollständig konzentriert. Säulenchromatographische (SiO₂, Toluol) Reinigung des Rohproduktes liefert (±)-(*4aR**, *9bS**)-7-Ethoxy-6-fluor-3-[1-methoxy-methyliden]-1,2,3,4,4a,9b-hexahydro-dibenzofuran als farblosen Feststoff.

### 1.7 (±)-(3R*, 4aR*, 9bS*)-7-Ethoxy-6-fluor-1,2,3,4,4a,9b-hexahydro-dibenzofuran-3-carbaldehyd und (±)-(3S*, 4aR*, 9bS*)-7-Ethoxy-6-fluor-1,2,3,4,4a,9b-hexahydro-dibenzofuran-3-carbaldehyd

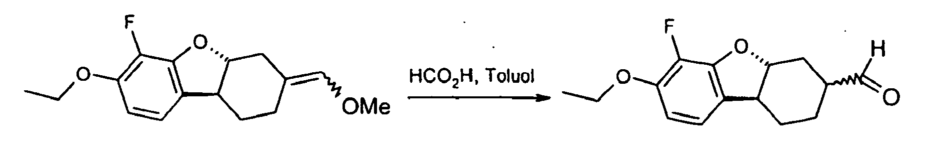

8.0 g (28.7 mmol) (±)-(*4aR**, *9bS**)-7-Ethoxy-6-fluor-3-[1-methoxy-methyliden]-1,2,3,4,4a,9b-hexahydro-dibenzofuran werden in 200 ml Toluol gelöst und zusammen mit 30 ml (0.80 mol) Ameisensäure und 0.5 ml (27.8 mmol) Wasser 18 h bei Raumtemperatur intensiv gerührt. Die organische Phase wird abgetrennt und nacheinander mit Wasser, ges. Natriumhydrogencarbonatlösung und ges. Natriumchloridlösung gewaschen. Die Lösung wird mit Natriumsulfat getrocknet und vollständig konzentriert. Nach säulenchromatographischer Reinigung (SiO₂, Toluol : Ethylacetat = 99 : 1) wird ein Gemisch (64 : 36) aus (±)-(3*R**, *4aR**, *9bS**)-7-Ethoxy-6-fluor-1,2,3,4,4a,9b-hexahydro-dibenzofuran-3-carbaldehyd und (±)-(3*S**, *4aR** *9bS**)-7-Ethoxy-6-fluor-1,2,3,4,4a,9b-hexahydro-dibenzofuran-3-carbaldehyd erhalten.

### 1.8 Isomerisierung zu (±)-(3R*, 4aR* 9bS*)-7-Ethoxy-6-fluor-1,2,3,4,4a,9b-hexahydro-dibenzofuran-3-carbaldehyd

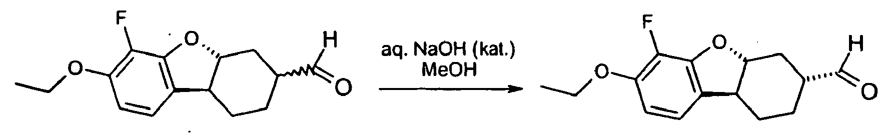

6.70 g (25.2 mmol) eines Gemisches (64 : 36) aus (±)-(3*R**, 4*aR**, 9*bS**)-7-Ethoxy-6-fluor-1,2,3,4,4a,9b-hexahydro-dibenzofuran-3-carbaldehyd und (±)-(3*S**, 4*aR**, *9bS**)-7-Ethoxy-6-fluor-1,2,3,4,4a,9b-hexahydro-dibenzofuran-3-carbaldehyd wird in 170 ml Methanol-THF-Gemisch (5 : 2) gelöst, und 0.37 ml (2.50 mmol) wässrige Natrium-hydroxidlösung (20 %) werden tropfenweise zugegeben. Nach 1 h bei Raumtemperatur wird die Lösung zu Wasser gegeben und mit 2 N Salzsäure angesäuert. Der Ansatz wird mit MTBE extrahiert und die vereinigten organischen Phasen mit ges. Natriumchloridlösung gewaschen. Die Lösung wird mit Natriumsulfat getrocknet und vollständig konzentriert. Das Rohprodukt der Umsetzung wird direkt für die folgenden Stufen verwendet.

### 1.9 (±)-(3R*, 4aR* 9bS*)-7-Ethoxy-6-fluor-3-propenyl)-1,2,3,4,4a,9b-hexahydro-dibenzofuran

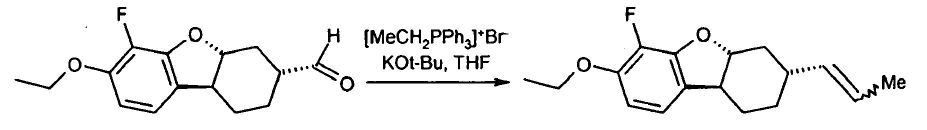

10.0 g (27.0 mmol) Ethyltriphenylphosphoniumbromid werden in 100 ml THF vorgelegt und bei -5 °C mit 2.98 g (26.0 mmol) Kalium-*tert*-butylat in 40 ml THF versetzt. Nach 1 h bei dieser Temperatur werden 6.50 g (24.6 mmol) (±)-(3*R**, *4aR* 9bS**)-7-Ethoxy-6-fluor-1,2,3,4,4a,9b-hexahydro-dibenzofuran-3-carbaldehyd als Lösung in 60 ml THF zugetropft und der Ansatz 2 h bei Raumtemperatur gerührt. Die Reaktionslösung wird mit Wasser hydrolysiert und mit 2 N HCI sauer gestellt. Die Mischung wird mit MTBE extrahiert, und die vereinigten organischen Phasen werden mit Natriumsulfat getrocknet. Das nach Entfernen der Lösungsmittel verbleibende Rohprodukt wird adsorptiv (SiO₂, Toluol) filtriert, und das Filtrat wird vollständig konzentriert. Nach Umkristallisation des Rückstandes aus Methanol wird (±)-(3*R**, *4aR**, *9bS**)-7-Ethoxy-6-fluor-3-propenyl)-1,2,3,4,4a,9b-hexahydro-dibenzofuran als *E*/Z-Isomerengemisch erhalten.

### 1.10 (±)-(3R*, 4aR*, 9bS*)-7-Ethoxy-6-fluor-3-propyl-1,2,3,4,4a,9b-hexahydro-dibenzofuran

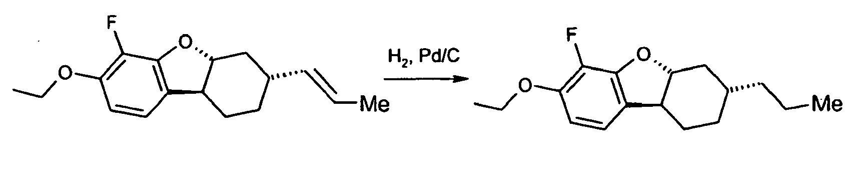

3.50 g (12.7 mmol) (±)-(3*R**, *4aR**, *9bS**)-7-Ethoxy-6-fluor-3-propenyl)-1,2,3,4,4a,9b-hexahydro-dibenzofuran werden in THF unter Zusatz von 1.8 g Pd/C 23 h in einer Wasserstoffatmosphäre hydriert. Nach Beendigung der Wasserstoffaufnahme wird die Reaktionslösung filtriert und vollständig konzentriert. Der Rückstand wird adsorptiv (SiO₂, Toluol: *n*-Heptan = 1 : 1) filtriert, und der erhaltene beige Feststoff wird mehrfach aus Isopropanol bei Raumtemperatur umkristallisiert. Auf diese Weise wird (±)-(3*R**, *4aR**, *9bS**)-7-Ethoxy-6-fluor-3-propyl-1,2,3,4,4a,9b-hexahydro-dibenzofuran als farbloser Feststoff erhalten (Schmelzpunkt 128 °C).
K 128 I
Δε = -6.0
Δ n = 0.102
γ ₁ = 127 mPa·s

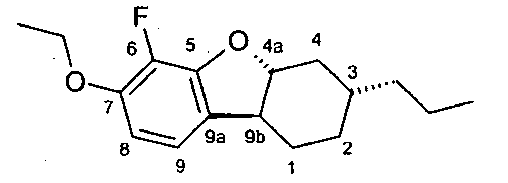

**¹H-NMR** (500 MHz, CHCl₃): δ = 6.73 (d, 1H, *J* = 8.0 Hz, 9-H), 6.45 (dd, 1H, *J* = 8.0 Hz Hz, J = 6.8 Hz, 8-H), 4.06 (dq, 2H, *J* = 7.0 Hz, *J* = 1.5 Hz, O**CH₂**CH₃), 3.95 (ddd, 1H, *J* = 12.6 Hz, *J* = 11.5 Hz, *J* = 3.5 Hz, 4a-H), 2.74 (ddd, 1H, *J* = 12.6 Hz, *J* = 12.6 Hz, *J* = 2.8 Hz, 9b-H), 2.41-2.37 (m, 1H, 4-H), 2.30-2.26 (m, 1H, 1-H), 1.89 (dd, 1H, *J* = 13.8 Hz, *J* = 2.7 Hz, 2-H), 1.58-1.45 (m, 2H, 1-H, 3-H), 1.42 (t, 3H, *J* = 7.0 Hz, OCH₂C**H₃**), 1.39-1.29 (m, 5H, 4-H, **CH₂CH₂**CH₃), 1.06 (ddd, 1H, *J* = 13.8 Hz, *J* = 12.9 Hz, *J* = 4.0 Hz, 2-H), 0.91 (t, 3H, *J* = 6.9 Hz, CH₂CH₂**CH₃**).

**¹⁹F-NMR** (235 MHz, CHCl₃): δ = -157.9 (d, 1F, ⁴*J* = 6.8 Hz).

**MS**: m/z(%) = 278 (100, M⁺), 235 ([M - Pr]⁺, 49).

### 2. (±)-(3R*,4aR*,9bS*)-7-Ethoxy-3-(4-ethoxy-2,3-difluor-phenyl)-6-fluor-1,2,3,4,4a,9b-hexahydro-dibenzofuran

### 2.1 (±)-(4aR*,9bS*)-7-Ethoxy-3-(4-ethoxy-2,3-difluor-phenyl)-6-fluor-1,2,3,4,4a,9b-hexahydrodibenzofuran-3-ol

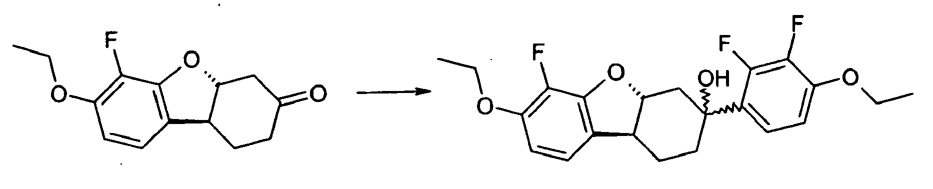

7.94 g (50.2 mmol) 2,3-Difluorethoxybenzol werden in 50 ml THF vorgelegt und bei -70 °C mit 30.5 ml (48.8 mol) n-BuLi (15% Lsg. in Hexan) versetzt. Nach 1 h bei dieser Temperatur wird eine Lösung von 10.0 g (40.0 mmol) (±)-(*4aR**, *9bS**)-7-Ethoxy-6-fluor-1,4,4a,9b-tetrahydro-2H-dibenzofuran-3-on in 150 ml THF zudosiert. Nach 4 h wird mit Wasser hydrolysiert und der Ansatz mit 4 N HCl angesäuert. Die Lösung wird mit MTBE extrahiert, und die vereinigten organischen Phasen werden mit ges. Natriumchloridlösung gewaschen und mit Natriumsulfat getrocknet. Das nach Entfernen der Lösungsmittel unter vermindertem Druck verbleibende Rohprodukt wird in 500 ml Ethanol bei 40 °C digeriert. Nach Filtration wird (±)-(*4aR*, 9bS**)*-*7-Ethoxy-3-(4-ethoxy-2,3-difluor-phenyl)-6-fluor-1,2,3,4,4a,9b-hexahydro-dibenzofuran-3-ol als farbloser Feststoff erhalten.

### 2.2 (±)-(4aR*, 9bS*)-7-Ethoxy-3-(4-ethoxy-2,3-difluor-phenyl)-6-fluor-1,2,4a,9b-tetrahydro-dibenzofuran und (±)-(4aR*, 9bS*)-7-Ethoxy-3-(4-ethoxy-2,3-difluor-phenyl)-6-fluor-1,4,4a,9b-tetrahydro-dibenzofuran

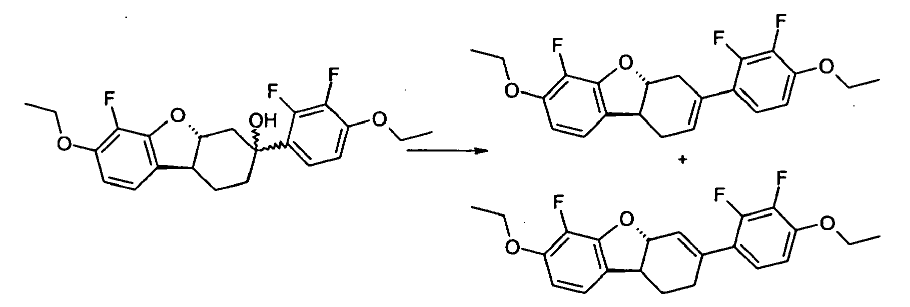

8.5 g (20.8 mmol) (±)-(*4aR** 9*bS**)-7-Ethoxy-3-(4-ethoxy-2,3-difluorphenyl)-6-fluor-1,2,3,4,4a,9b-hexahydro-dibenzofuran-3-ol werden in 200 ml Toluol zusammen mit 396 mg (2.08 mmol) *p*-Toluolsulfonsäure-monohydrat 30 min am Wasserabscheider erhitzt. Nach dem Abkühlen wird der Ansatz adsorptiv (SiO₂, Ethylacetat : *n*-Heptan = 4 : 1) filtriert, und das Filtrat wird vollständig konzentriert. Das so erhaltene Produktgemisch kann direkt für die folgende Umsetzung verwendet werden.

### 2.3 (±)-(3R*, 4aR*, 9bS*)-7-Ethoxy-3-(4-ethoxy-2,3-difluor-phenyl)-6-fluor-1,2,3,4,4a,9b-hexahydro-dibenzofuran

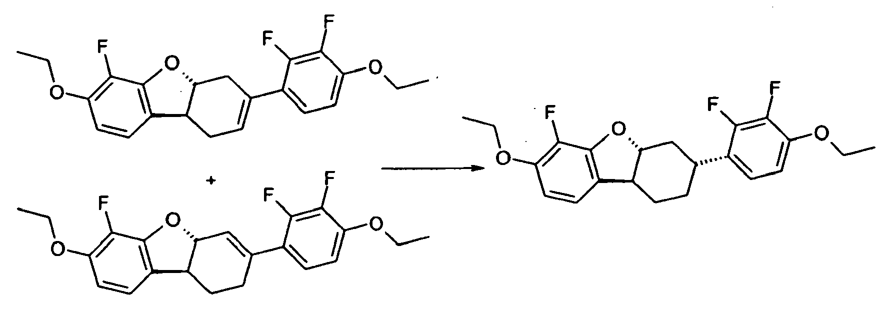

7.4 g (ca. 19.0 mmol) eines Gemisches aus (±)-(*4aR**, *9bS**)-7-Ethoxy-3-(4-ethoxy-2,3-difluor-phenyl)-6-fluor-1,2,4a,9b-tetrahydro-dibenzofuran und (±)-(*4aR**, *9bS**)-7-Ethoxy-3-(4-ethoxy-2,3-difluor-phenyl)-6-fluor-1,4,4a,9b-tetrahydro-dibenzofuran werden in 160 ml Ethylacetat-Ethanol-Gemisch (3 : 1) in Gegenwart von 3.70 g Raney-Nickel und 1.50 g lonenaustauscher (schwach H-acid) unter Wasserstoffdruck (4.4 bar) bei 80 °C hydriert. Nach 18 h wird vom Katalysator abfiltriert, und das Filtrat wird vollständig konzentriert. Das Rohprodukt wird nacheinander aus Isopropanol, *n*-Heptan und Ethanol umkristallisiert. Auf diese Weise wird (±)-(3*R**, *4aR**, *9bS**)-7-Ethoxy-3-(4-ethoxy-2,3-difluor-phenyl)-6-fluor-1,2,3,4,4a,9b-hexahydro-dibenzofuran als farbloser Feststoff (Schmelzpunkt 126 °C) isoliert.
K 126 N(99) I
Δε = -12.0
Δ n = 0.138

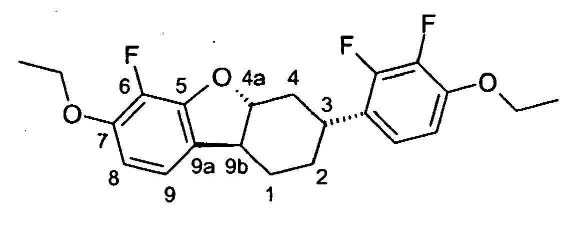

**¹H-NMR** (250 MHz, CHCl₃): δ = 6.94-6.87 (m, 1H, H*_{arom.}*)*,* 6.78 (dm, 1H, J = 8.0 Hz, H*_{arom.}*)*,* 6.75-6.67 (m, 1H, H*_{arom.}*)*,* 6.49 (dd, 1H, J = 8.0 Hz, J = 7.0 Hz, H*ₐᵣₒₘ*.), 4.17-4.04 (m, 5H, O**CH₂**CH₃, 4a-H, O**CH₂**CH₃), 3.11-2.86 (m, 2H, 9b-H, 3-H), 2.54-2.37 (m, 2H, H*_{aliph.}*)*,* 2.20-1.96 (m, 2H, H*_{aliph.}*)*,* 1.63-1.55 (m, 2H, H*_{aliph.}*), 1.44 (t, 3H, J = 7.0 Hz, OCH₂**CH₃**), 1.43 (t, 3H, J = 7.0 Hz, OCH₂**CH₃**).

**¹⁹F-NMR** (235 MHz, CHCl₃): δ = -142.9 (ddd, 1F, J = 13.5 Hz, J = 7.4 Hz, J = 1.2 Hz), -159.2 (ddd, 1F, J = 13.5 Hz, J = 7.4 Hz, J = 1.2 Hz), -159.8 (d, 1F, J = 6.8 Hz).

**MS** (EI): m/z(%) = 392 (39, M⁺), 234 (100).

### 3. (±)-(3R*,4aR*,9bS*)-7-Ethoxy-6-fluor-3-vinyl-1,2,3,4,4a,9b-hexahydro-dibenzofuran

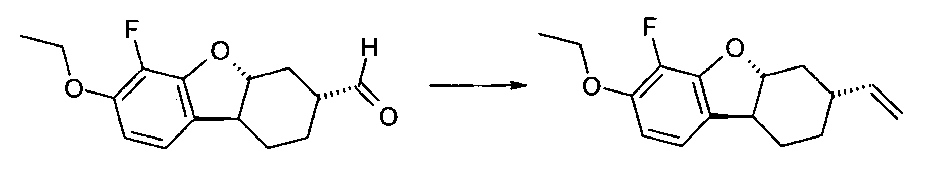

5.0 g (14.0 mmol) Methyltriphenylphosphoniumbromid werden in 50 ml THF vorgelegt und bei -5 °C mit 1.60 g (14.3 mmol) Kalium-*tert*-butylat in 20 ml THF versetzt. Nach 1 h bei dieser Temperatur werden 3.40 g (12.9 mmol) (±)-(3*R**, *4aR**, *9bS**)-7-Ethoxy-6-fluor-1,2,3,4,4a,9b-hexahydro-dibenzofuran-3-carbaldehyd als Lösung in 30 ml THF zugetropft, und der Ansatz wird 16 h bei Raumtemperatur gerührt. Die Reaktionslösung wird mit Wasser hydrolysiert und mit 2 N HCl sauer gestellt. Die Mischung wird mit MTBE extrahiert, und die vereinigten organischen Phasen werden mit ges. Natriumchloridlösung gewaschen und mit Natriumsulfat getrocknet. Das nach Entfernen der Lösungsmittel verbleibende Rohprodukt wird säulenchromatographisch (SiO₂, Toluol) gereinigt. Die weitere Reinigung erfolgte durch mehrfaches Umkristallisieren aus Isopropanol. (±)-(3*R**, *4aR** *9bS**)-7-Ethoxy-6-fluor-3-vinyl-1,2,3,4,4a,9b-hexahydro-dibenzofuran wird als farbloser Feststoff erhalten (Schmelzpunkt 141 °C). K 141 I

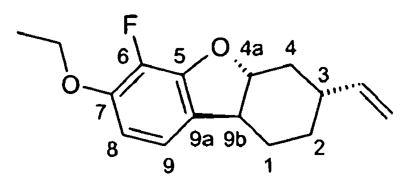

**¹H-NMR** (250 MHz, CHCl₃): δ = 6.75 (dm, 1H, J = 8.0 Hz, 9-H), 6.46 (dd, 1H, J = 8.0 Hz, J = 7.0 Hz, 8-H), 5.92-5.78 (m, 1H, H*_{vinyl}*)*,* 5.11-4.97 (m, 2H, H*_{vinyl}*)*,* 4.07 (d, 2H, J = 7.0 Hz, O**CH**₂CH₃), 4.05-3.95 (m, 1H, 4a-H), 2.83-2.72 (m, 1H, 9b-H), 2.47-2.18 (m, 3H, H*_{aliph.}*), 1.97-1.87 (m, 1H, H*_{aliph.}*), 1.80-1.67 (m, 1H, H*_{aliph.}*)*,* 1.53-1.18 (m, 5H, J = 7.0 Hz, H*_{aliph.,}* OCH₂**CH**₃).

**¹⁹F-NMR** (235 MHz, CHCl₃): δ = -157.6 (d, 1 F, ⁴*J* = 6.8 Hz).
**MS** (EI): m/z(%) = 262 (81, M⁺), 206 (100, [M - Et - Vn]⁺).

### 4. (±)-(3R*, 4aR*, 9bS*)-7-Ethoxy-6-fluor-3-((E)-propenyl)-1,2,3,4,4a,9b-hexahydro-dibenzofuran

### 4.1 Isomerisierung zu (±)-(3R*, 4aR* 9bS*)-7-Ethoxy-6-fluor-3-((E)-propenyl)-1,2,3,4,4a,9b-hexahydro-dibenzofuran

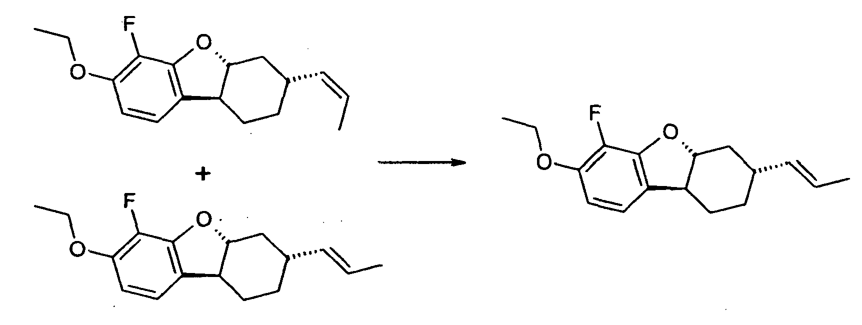

6.0 g (ca. 21.7 mmol) (*E*/Z)-Isomerengemisch von (±)*-*(3*R*, 4aR* 9bS**)*-*7-Ethoxy-6-fluor-3-propenyl-1,2,3,4,4a,9b-hexahydro-dibenzofuran (s. 1.9) werden zusammen mit 1.15 g (7.0 mmol) Benzolsulfinsäure-natriumsalz und 21.4 ml 1 N Salzsäure in 60 ml Toluol zum Rückfluß erhitzt. Nach 1 h wird die Mischung auf Wasser gegeben und die organische Phase abgetrennt. Die wäßrige Phase wird mit MTBE extrahiert, und die vereinigten organischen Phasen werden mit ges. Natriumchloridlösung gewaschen. Die Lösung wird mit Natriumsulfat getrocknet und vollständig konzentriert. Durch mehrfaches Umkristallisieren aus Isopropanol wird (±)-(3*R**, 4a*R** *9bS**)-7-Ethoxy-6-fluor-3-((*E*)-propenyl)-1,2,3,4,4a,9b-hexahydro-dibenzofuran als farbloser Feststoff erhalten (Schmelzpunkt 145°C).
K 1451 I Δε = -6.0
Δ n = 0.110

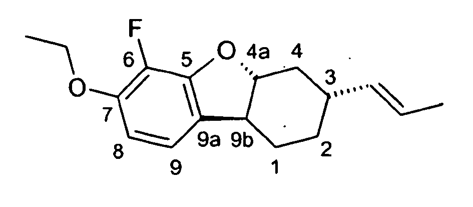

**¹H-NMR** (250 MHz, CHCl₃): δ = 6.74 (dm, 1H, J = 8.0 Hz, 9-H), 6.46 (dd, 1H, J = 8.0 Hz, J = 7.0 Hz, 8-H), 5.54-5.38 (m, 2H, H*_{vinyl}*), 4.07 (q, 2H, J = 7.0 Hz, O**CH₂**CH₃) 4.04-3.92 (m, 1H, 4a-H), 2.81-2.70 (m, 1H, 9b-H), 2.42-2.11 (m, 3H, H*_{aliph.}*), 1.91-1.82 (m, 1H, H*_{aliph.}*), 1.76-1.62 (m, 4H, J = 4.8 Hz, H_{*aliph*.,} CH=CH**CH₃**), 1.52-1.14 (m, 5H, J = 7.0 Hz, H*_{aliph.}*, OCH₂**CH₃**).

**¹⁹F-NMR** (235 MHz, CHCl₃): δ = -159.1 (d, 1F, ⁴*J* = 6.8 Hz).

**MS** (EI): m/z(%) = 276 (72, M⁺), 206 (100, [M **-** Et - C₃H₅]⁺).

### 5. (±)-(3R*,4aR*,9bS*)-7-Ethoxy-6-fluor-3-pentyl-1,2,3,4,4a,9b-hexahydro-dibenzofuran

### 5.1 (±)-(3R*,4aR*9bS*)-7-Ethoxy-6-fluor-3-((E)-pent-1-enyl)-1,2,3,4,4a,9b-hexahydro-dibenzofuran und (±)-(3R*,4aR*,9bS*)-7-Ethoxy-6-fluor-3-((Z)-pent-1-enyl)-1,2,3,4,4a,9b-hexahydro-dibenzofuran

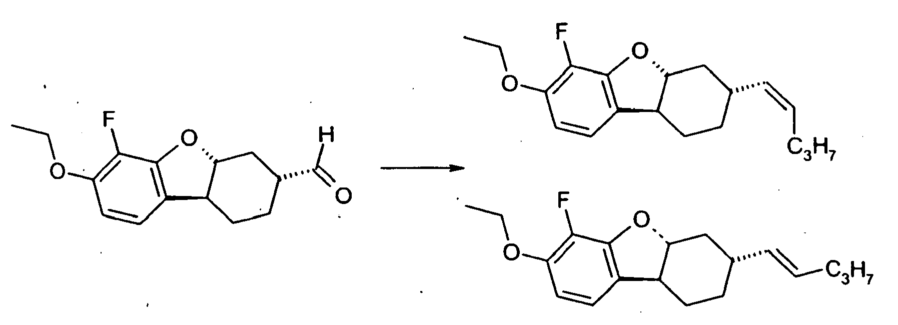

8.98 g (22.5 mmol) Butyltriphenylphosphoniumbromid werden in 100 ml THF vorgelegt und bei -5 °C mit 2.58 g (23.0 mmol) Kalium-*tert*-butylat in 40 ml THF versetzt. Nach 1 h bei dieser Temperatur werden 5.50 g (20.8 mmol)(±)-(3*R**, *4aR** *9bS**)-7-Ethoxy-6-fluor-1,2,3,4,4a,9b-hexahydro-dibenzofuran-3-carbaldehyd als Lösung in 60 ml THF zugetropft, und der Ansatz wird 2 h bei Raumtemperatur gerührt. Die Reaktionslösung wird mit Wasser hydrolysiert und mit 2N HCl sauer gestellt. Die Mischung wird mit MTBE extrahiert, und die vereinigten organischen Phasen werden mit ges. Natriumchloridlösung gewaschen und mit Natriumsulfat getrocknet. Das nach Entfernen der Lösungsmittel verbleibende Rohprodukt wird säulenchromatographisch (SiO₂ Toluol) gereinigt. Das auf diese Weise erhaltene *E*/Z-Isomerengemisch kann direkt für die folgende Umsetzung verwendet werden.

### 5.2 (±)-(3R*, 4aR* 9bS*)-7-Ethoxy-6-fluor-3-pentyl-1,2,3,4,4a,9b-hexahydro-dibenzofuran

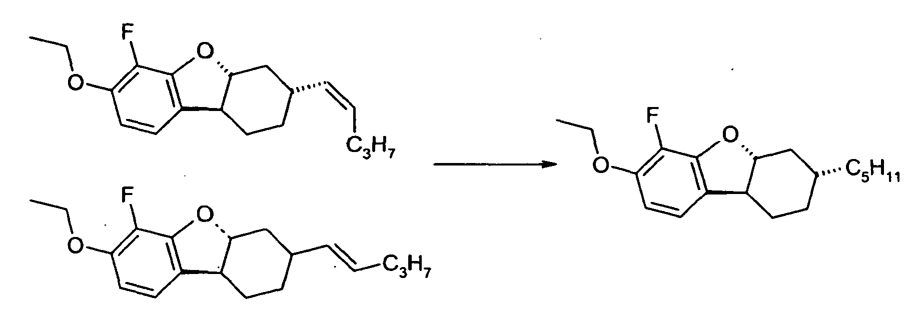

5.1 g (ca. 10.7 mmol) (*E*/Z)-Isomerengemisch von (±)-(3*R**, 4a*R** *9bS**)-7-Ethoxy-6-fluor-3-pent-1-enyl-1,2,3,4,4a,9b-hexahydro-dibenzofuran werden in 50 ml THF unter Zusatz von 5.0 g Pd/C (5% Pd) in einer Wasserstoffatmosphäre hydriert. Nach Beendigung der Wasserstoffaufnahme (37 h) wird die Reaktionslösung filtriert und vollständig konzentriert. Der Rückstand wird adsorptiv (SiO₂, Toluol) filtriert, und der erhaltene beige Feststoff wird mehrfach aus Isopropanol bei Raumtemperatur umkristallisiert. Auf diese Weise wird (±)-(*3R**, *4aR**, *9bS**)-7-Ethoxy-6-fluor-3-pentyl-1,2,3,4,4a,9b-hexahydro-dibenzofuran als farbloser Feststoff (Schmelzpunkt 118 °C) erhalten.
K 118 I
Δε = -5.4
Δ n =0.104
γ ₁ = 172 m Pa·s

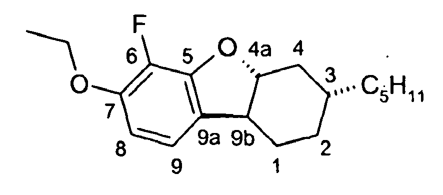

**¹H-NMR** (250 MHz, CHCl₃): δ = 6.74 (dm, 1H, J = 8.0 Hz, 9-H), 6.45 (dd, 1H, J = 8.0 Hz, J = 7.0 Hz, 8-H), 4.07 (q, 2H, J = 7.0 Hz, O**CH₂**CH₃) 4.01-3.90 (m, 1H, 4a-H), 2.80-2.69 (m, 1H, 9b-H), 2.43-2.36 (m, 1H, H*_{aliph.}*), 2.29 (dm, 1H, J = 12.5 Hz, H*_{aliph.}*)*,* 1.90 (dd, 1H, J = 13.1 Hz, J = 2.7 Hz, H*_{aliph.}*), 1.50-1.20 (m, 14 H, H*_{aliph.}*)*,* 1.16-0.98 (m, 1H, H*ₐₗᵢₚₕ*.)*,* 0.90 (t, 3H, J = 7.0 Hz, OCH₂CH₃).

**¹⁹F-NMR** (235 MHz, CHCl₃): δ =-160.1 (d, 1F, ⁴*J*=7.1 Hz).

**MS** (EI): m/z(%) = 306 (100, M⁺), 235 (100, [M -C₅H₁₁]⁺).

### 6. (±)-(3R*,4aR*,9bS*)-7-Butoxy-6-fluor-3-(4-propyl-cyclohexyl)-1,2,3,4,4a,9b-hexahydro-dibenzofuran

### 6.1 4-(4-Butoxy-2,3-difluor-phenyl)-4'-propyl-bicyclohexyl-4-ol

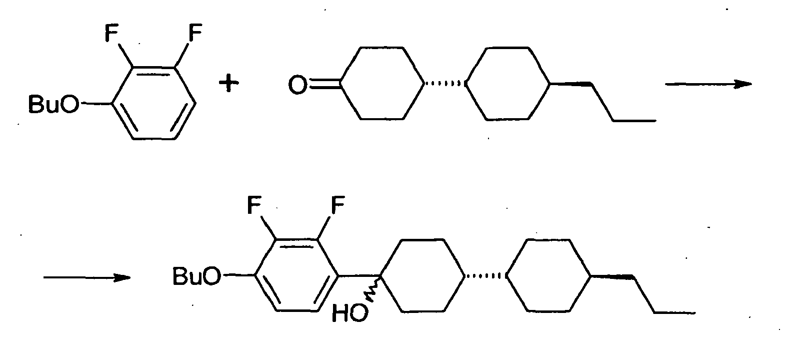

31.0 g (0.17 mol) 1-Butoxy-2,3-difluor-benzol werden in 200 ml THF vorgelegt und bei -70 °C mit 100 ml (0.16 mol) *n*-BuLi (15% Lsg. in Hexan) versetzt. Nach 2 h bei dieser Temperatur wird eine Lösung von 35.6 g (0.16 mol) 4'-Propyl-bicyclohexyl-4-on in 200 ml THF zudosiert, und der Ansatz wird 2.5 h gerührt. Die Reaktionsmischung wird unter Eiskühlung hydrolysiert und mit 2N HCl sauer gestellt. Die Lösung wird mit MTBE extrahiert, und die vereinigten organischen Phasen werden mit ges. Natriumchloridlösung gewaschen und mit Natriumsulfat getrocknet. Die Lösung wird vollständig konzentriert, und das Rohprodukt (66.3 g gelber Feststoff) wird direkt für die folgende Umsetzung verwendet.

### 6.2 4-(4-Butoxy-2,3-difluor-phenyl)-4'-propyl-bicyclohexyl-3-en

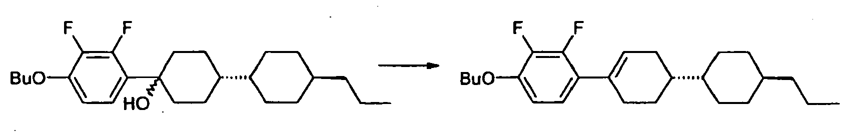

66.3 g (ca. 0.16 mol) rohes 4-(4-Butoxy-2,3-difluor-phenyl)-4'-propyl-bicyclohexyl-4-ol werden in 200 ml Toluol zusammen mit 3.04 g (16.0 mmol) *p*-Toluolsulfonsäure-monohydrat 2 h am Wasserabscheider erhitzt. Nach dem Abkühlen wird der Ansatz nacheinander mit Wasser, ges. Natriumhydrogencarbonatlösung und ges. Natriumchloridlösung gewaschen. Die Lösung wird mit Natriumsulfat getrocknet und. vollständig konzentriert. Das Rohprodukt (60.7 g orange Öl) wird aus Ethanol kristallisiert. Auf diese Weise erhält man 4-(4-Butoxy-2,3-difluor-phenyl)-4'-propyl-bicyclohexyl-3-en als gelben Feststoff.

### 6.3 4-(4-Butoxy-2,3-difluor-phenyl)-4'-propyl-bicyclohexyl-3-ol

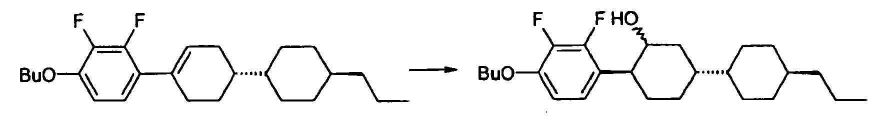

Eine Lösung von 31.4 g (80.4 mmol) 4-(4-Butoxy-2,3-difluor-phenyl)-4'-propyl-bicyclohexyl-3-en in 320 ml THF wird bei -5 °C mit 110 ml (0.11 mol) Boran-THF-Komplex (1 M Lösung) versetzt, und die Reaktionsmischung wird 3 h bei Raumtemperatur gerührt. Der Ansatz wird nacheinander mit 20 ml (0.35 mol) Ethanol, 50 ml (0.2 mol) wässriger Natriumhydroxidlösung (4M) und 28 ml (0.32 mol) wässriger Wasserstoffperoxid-Lsg. (35%) versetzt, wobei die Innentemperatur 47°C nicht übersteigt (Eisbad). Nach beendeter Zugabe wird 2 h zum Rückfluss erwärmt und die Lösung nach dem Abkühlen auf Wasser gegeben und intensiv gerührt. Die organische Phase wird abgetrennt und die wässrige Phase mit MTBE extrahiert. Die vereinigten organischen Phasen werden mit ges. Natriumchloridlösung gewaschen und mit Natriumsulfat getrocknet. Die Lösung wird vollständig konzentriert und das Rohprodukt (32 g) säulenchromatographisch (SiO₂ Toluol → Toluol : Ethylacetat = 8 : 2) gereinigt. 4-(4-Butoxy-2,3-difluor-phenyl)-4'-propyl-bicyclohexyl-3-ol wird als farbloser Feststoff erhalten.

### 6.4 (±)-(3R*,4aR*,9bS*)-7-Butoxy-6-fluor-3-(4-propyl-cyclohexyl)-1,2,3,4,4a,9b-hexahydro-dibenzofuran

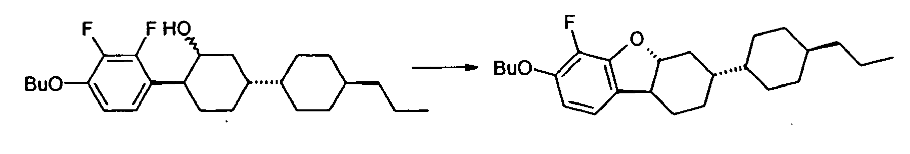

5.40 g (135 mmol) Natriumhydrid (60% Suspension in Mineralöl) werden wiederholt mit n-Pentan gewaschen und in 400 ml Toluol suspendiert. Die Suspension wird auf 90 °C erhitzt, und eine Lösung von 17.5 g (42.8 mmol) 4-(4-Butoxy-2,3-difluor-phenyl)-4'-propyl-bicyclohexyl-3-ol in 100 ml DMF wird langsam zudosiert. Der Ansatz wird 20 h bei 90 °C gerührt und nach dem Abkühlen mit Wasser hydrolysiert. Die Mischung wird durch Zugabe von 2 N Salzsäure neutralisiert, und die organische Phase wird abgetrennt. Die wässrige Phase wird mit Toluol extrahiert, und die vereinigten organischen Phasen werden mit ges. Natriumchloridlösung gewaschen. Die Lösung wird mit Natriumsulfat getrocknet und unter vermindertem Druck vollständig konzentriert. Das erhaltene Rohprodukt wird bei 5 °C aus Ethanol umkristallisiert. (±)-(3*R**, *4aR** *9bS**)-7-Butoxy-6-fluor-3-(4-propyl-cyclohexyl)-1,2,3,4,4a,9b-hexahydro-dibenzofuran wird als farbloser Feststoff (Schmelzpunkt 105 °C) isoliert.
K 105 Sm_{A} 154 N 1661 I
Δε = -5.6
Δ n = 0.114
γ ₁ = 974 mPa·s

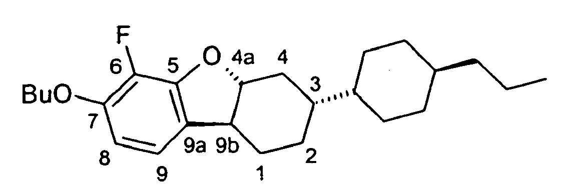

**¹H-NMR** (250 MHz, CHCl₃): δ = 6.73 (dm, 1H, J = 8.0 Hz, 9-H), 6.45 (dd, 1H, J = 8.0 Hz, J = 7.0 Hz, 8-H), 3.99 (t, 3H, J = 6.9 Hz, O**CH₂**CH₂-), 3.98-3.88 (m, 1H, 4a-H), 2.77-2.66 (m, 1H, *H_{aliph.}),* 2.40-2.26 (m, 2H, *H_{aliph.}),* 1.90-1.69 (m, 9H, *H_{aliph.}),* 1.65-1.02 (m, 13H, *H_{aliph.}),* 0.96 (t, 3H, J = 7.4 Hz, Me), 0.88 (t, 3H, J = 7.2 Hz, Me).

**¹⁹F-NMR** (235 MHz, CHCl₃): δ = 158.2 (d, 1F, ⁴*J* = 6.8 Hz).

**MS** (EI): m/z(%) = 388 (100, M⁺).

### 7. (±)-(3R*,4aR*,9bS*)-(±)-(3R*,4aR*,9bS*)-7-Ethoxy-6-fluor-3-(4-vinyl-cyclohexyl)-1,2,3,4,4a,9b-hexahydro-dibenzofuran

### 7.1 4-(1,4-Dioxa-spiro[4.5]dec-8-yl)-1-(4-ethoxy-2,3-difluor-phenyl)-cyclohexanol

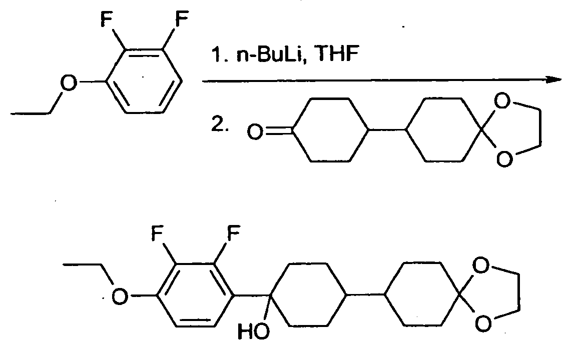

99.5 g (0.63 mol) 2,3-Difluorethoxybenzol werden in 800 ml THF vorgelegt und bei -70 °C mit 384 ml (0.63 mol) *n*-BuLi (15% Lsg. in Hexan) versetzt. Nach 30 min bei dieser Temperatur wird eine Lösung von 150 g (0.63 mol) 4-(1,4-Dioxa-spiro[4.5]dec-8-yl)-cyclohexanon in 700 ml THF zudosiert, und der Ansatz wird 30 min gerührt. Die Reaktionsmischung wird auf 0 °C erwärmt und mit 2 N HCl hydrolysiert. Die Lösung wird mit MTBE extrahiert, und die vereinigten organischen Phasen werden mit ges. Natriumchloridlösung gewaschen und mit Natriumsulfat getrocknet. Das nach Entfernen der Lösungsmittel unter vermindertem Druck verbleibende Rohprodukt wird direkt für die nächste Umsetzung verwendet.

### 7.2 8-[4-(4-Ethoxy-2,3-difluor-phenyl)-cyclohex-3-enyl]-1,4-dioxa-spiro[4.5]decan

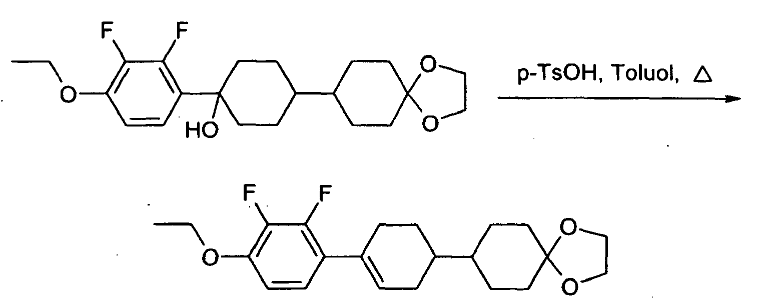

250 g rohes 4-(1,4-Dioxa-spiro[4.5]dec-8-yl)-1-(4-ethoxy-2,3-difluorphenyl)-cyclohexanol werden in 1000 ml Toluol zusammen mit 80.0 ml (1.43 mol) Ethylenglycol unter Zusatz von 12.0 g (0.06 mol) *p*-Toluolsulfonsäure-monohydrat 3 h am Wasserabscheider erhitzt. Nach dem Abkühlen wird der Ansatz nacheinander mit Wasser, ges. Natriumhydrogencarbonatlösung und ges. Natriumchloridlösung gewaschen. Die Lösung wird mit Natriumsulfat getrocknet und vollständig konzentriert. Das Rohprodukt wird aus Acetonitril kristallisiert. Auf diese Weise wird 8-[4-(4-Ethoxy-2,3-difluor-phenyl)-cyclohex-3-enyl]-1,4-dioxa-spiro[4.5]decane als farbloser Feststoff erhalten.

### 7.3 5-(1,4-Dioxa-spiro[4.5]dec-8-yl)-2-(4-ethoxy-2,3-difluor-phenyl)-cyclohexanol

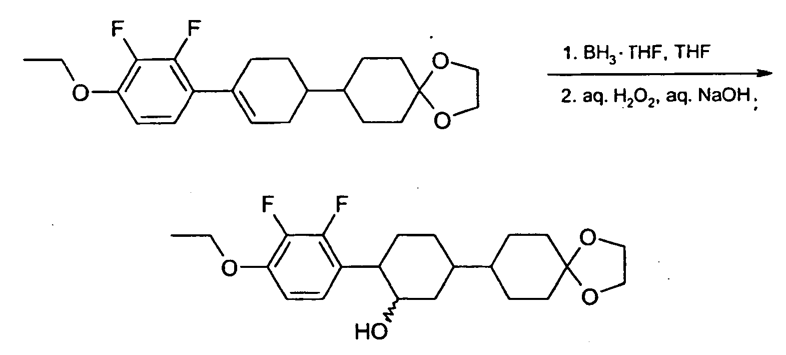

Eine Lösung von 154.0 g (0.41 mol) 8-[4-(4-Ethoxy-2,3-difluor-phenyl)-cyclohex-3-enyl]-1,4-dioxa-spiro[4.5]decane in 1500 ml THF wird bei -5 °C mit 550 ml (0.55 mol) Boran-THF-Komplex (1 M Lösung) versetzt, und die Reaktionsmischung wird 3 h bei Raumtemperatur gerührt. Der Ansatz wird nacheinander mit 99 ml (1.7 mol) Ethanol, 250 ml (1.0 mol) wässriger Natriumhydroxidlösung (16%) und 140 ml (1.6 mol) wässriger Wasserstoffperoxid-Lsg. (35%) versetzt, wobei die Innentemperatur 46 °C nicht übersteigt (Eisbad). Nach beendeter Zugabe wird 2 h zum Rückfluss erwärmt und die Lösung nach dem Abkühlen auf Wasser gegeben und intensiv gerührt. Die organische Phase wird abgetrennt und die wässrige Phase mit MTBE extrahiert. Die vereinigten organischen Phasen werden mit ges. Natriumchloridlösung gewaschen und mit Natriumsulfat getrocknet. Die Lösung wird vollständig konzentriert, und das Rohprodukt wird säulenchromatographisch (SiO₂, Toluol : Ethylacetat = 8 : 2) gereinigt. 5-(1,4-Dioxa-spiro[4.5]dec-8-yl)-2-(4-ethoxy-2,3-difluor-phenyl)-cyclohexanol wird als farbloses, zähes Öl erhalten.

### 7.4 (±)-(3R*,4aR*,9bS*)-3-(1,4-Dioxa-spiro[4.5]dec-8-yl)-7-ethoxy-6-fluor-1,2,3,4,4a,9b-hexahydro-dibenzofuran

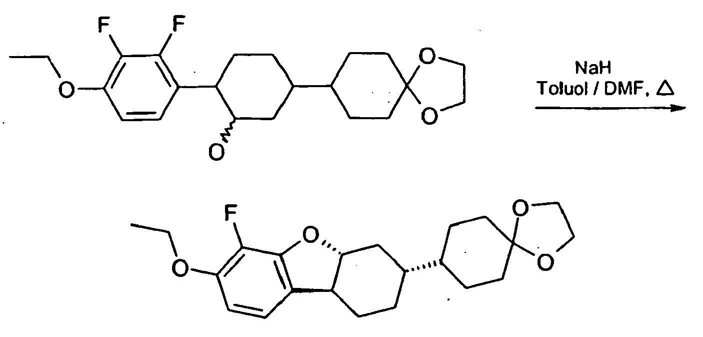

30.0 g (0.75 mol) Natriumhydrid (60% Suspension in Mineralöl) werden wiederholt mit n-Pentan gewaschen und in 2500 ml Toluol suspendiert. Die Suspension wird auf 90°C erhitzt, und eine Lösung von 101.5 g (0.26 mol) 5-(1,4-Dioxa-spiro[4.5]dec-8-yl)-2-(4-ethoxy-2,3-difluor-phenyl)-cyclohexanol in 500 ml DMF wird langsam zudosiert. Der Ansatz wird 20 h bei 90 °C gerührt und nach dem Abkühlen mit Wasser hydrolysiert. Die Mischung wird durch Zugabe 2N Salzsäure neutralisiert, und die organische Phase wird abgetrennt. Die wässrige Phase wird mit Toluol extrahiert, und die vereinigten organischen Phasen werden mit ges. Natriumchloridlösung gewaschen. Die Lösung wird mit Natriumsulfat getrocknet und unter vermindertem Druck vollständig konzentriert. Das erhaltene Rohprodukt wird zunächst säulenchromatographisch (SiO₂, Toluol : Ethylacetat = 8 : 2) gereinigt und dann nacheinander aus Toluol und Toluol : Ethanol (3 : 1) umkristallisiert. (±)-(3*R**,4a*R**,9b*S**)-3-(1,4-Dioxa-spiro[4.5]dec-8-yl)-7-ethoxy-6-fluor-1,2,3,4,4a,9b-hexahydro-dibenzofuran wird als farbloser, kristalliner Feststoff isoliert.

### 7.5 (±)-4-((3R*,4aR*,9bS*)-7-Ethoxy-6-fluor-1,2,3,4,4a,9b-hexahydro-dibenzofuran-3-yl)-cyclohexanon

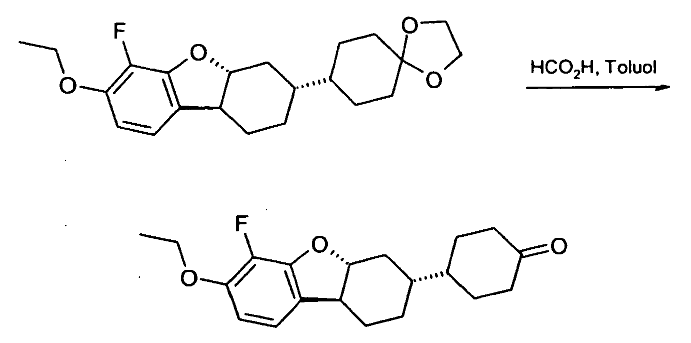

13.3 g (35.3 mmol) (±)-(3*R**,4a*R**,9b*S**)-3-(1,4-Dioxa-spiro[4.5]dec-8-yl)-7-ethoxy-6-fluor-1,2,3,4,4a,9b-hexahydro-dibenzofuran werden in 200 ml Toluol gelöst und zusammen mit 40 ml (1.06 mol) Ameisensäure unter Zusatz von 1.0 ml (55.6 mmol) Wasser intensiv gerührt. Nach 18 h wird die organische Phase abgetrennt, und die Ameisensäure mit Toluol extrahiert. Die vereinigten organischen Phasen werden nacheinander mit Wasser, ges. Natriumhydrogencarbonatlösung und ges. Natriumchloridlösung gewaschen und mit Natriumsulfat getrocknet. Das nach dem Entfernen des Lösungsmittels verbleibende Rohprodukt wird säulenchromatographisch (SiO₂, Toluol : Ethylacetat = 4 : 1) gereinigt. (±)-4-((3*R**,4a*R**,9b*S**)-7-Ethoxy-6-fluor-1,2,3,4,4a,9b-hexahydro-dibenzofuran-3-yl)-cyclohexanon wird als farbloser Feststoff isoliert.

### 7.6 (±)-(3R*,4aR*,9bS*)-7-Ethoxy-6-fluor-3-(4-methoxymethylen-cyclohexyl)-1,2,3,4,4a,9b-hexahydro-dibenzofuran

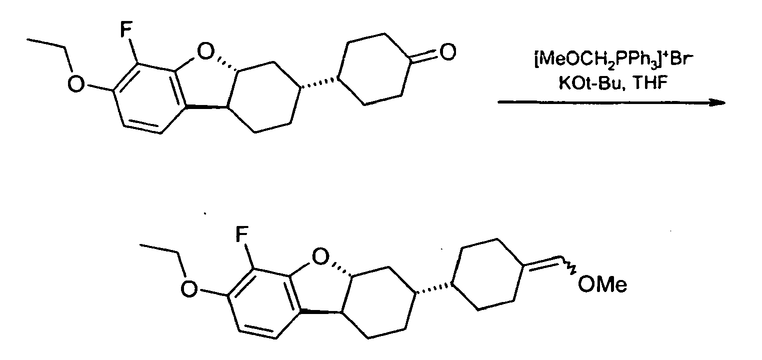

14.1 g (41.3 mmol) Methoxymethyltriphenylphosphoniumchlorid werden in 250 ml THF vorgelegt und bei 0 °C mit einer Lösung von 4.6 g (41.0 mmol) Kalium-*tert* butylat in 100 ml THF versetzt. Nach 30 min bei dieser Temperatur wird (±)-4-((3*R**,4a*R**,9b*S**)-7-Ethoxy-6-fluor-1,2,3,4,4a,9b-hexahydro-dibenzofuran-3-yl)-cyclohexanon als Lösung in 150 ml THF zugegeben und der Ansatz 17 h bei Raumtemperatur gerührt. Die Mischung wird bei 0 °C mit Wasser versetzt und mit 2N Salzsäure angesäuert. Der Ansatz wird mit MTBE extrahiert, und die vereinigten Extrakte werden mit ges. Natriumchloridlösung gewaschen. Die Lösung wird mit Natriumsulfat getrocknet und vollständig konzentriert. Säulenchromatographische (SiO₂, Toluol : Ethylacetat = 95 : 5) Reinigung des Rohproduktes liefert (±)-(3*R**,4a*R**,9b*S**)-7-Ethoxy-6-fluor-3-(4-methoxymethylen-cyclohexyl)-1,2,3,4,4a,9b-hexahydro-dibenzofuran als farblosen Feststoff.

### 7.7 (±)-4-((3R*,4aR*,9bS*)-7-Ethoxy-6-fluor-1,2,3,4,4a,9b-hexahydro-dibenzofuran-3-yl)-cyclohexancarbaldehyd

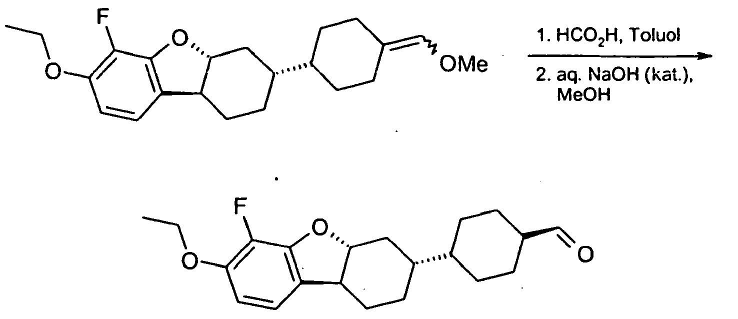

10.0 g (27.7 mmol) (±)-(3*R**,4a*R**,9b*S**)-7-Ethoxy-6-fluor-3-(4-methoxymethylen-cyclohexyl)-1,2,3,4,4a,9b-hexahydro-dibenzofuran werden in 400 ml Toluol gelöst und zusammen mit 30 ml (0.80 mol) Ameisensäure und 1.0 ml (55.6 mmol) Wasser 18 h bei Raumtemperatur intensiv gerührt. Die organische Phase wird abgetrennt und nacheinander mit Wasser, ges. Natriumhydrogencarbonatlösung und ges. Natriumchloridlösung gewaschen. Die Lösung wird mit Natriumsulfat getrocknet und vollständig konzentriert.
Der Rückstand wird in 250 ml Methanol-THF-Gemisch (5 : 2) gelöst, und 0.44 ml (3.0 mmol) wässrige Natriumhydroxidlösung (20%) werden tropfenweise zugegeben. Nach 3 h bei Raumtemperatur wird die Lösung zu Wasser gegeben und mit 2N Salzsäure angesäuert. Der Ansatz wird mit MTBE extrahiert und die vereinigten organischen Phasen mit ges. Natriumchloridlösung gewaschen. Die Lösung wird mit Natriumsulfat getrocknet und vollständig konzentriert. Das Rohprodukt der Umsetzung wird direkt für die folgenden Stufen verwendet.

### 7.8 (±)-(3R*,4aR*,9bS*)-7-Ethoxy-6-fluor-3-(4-vinyl-cyclohexyl)-1,2,3,4,4a,9b-hexahydro-dibenzofuran

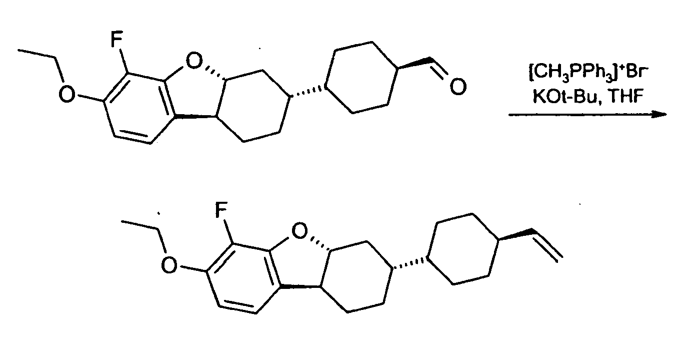

9.65 g (27.0 mmol) Methyltriphenylphosphoniumbromid werden in 100 ml THF vorgelegt und bei -5 °C mit 3.09 g (27.0 mmol) Kalium-*tert*-butylat in 60 ml THF versetzt. Nach 1 h bei dieser Temperatur werden 8.40 g (ca 24 mmol) roher (±)-4-((3*R**,4a*R**,9b*S**)-7-Ethoxy-6-fluor-1,2,3,4,4a,9b-hexahydro-dibenzofuran-3-yl)-cyclohexancarbaldehyd als Lösung in 90 ml THF zugetropft und der Ansatz 3 h bei Raumtemperatur gerührt. Die Reaktionslösung wird mit Wasser hydrolysiert und mit 2N HCl sauer gestellt. Die Mischung wird mit MTBE extrahiert, und die vereinigten organischen Phasen werden mit Natriumsulfat getrocknet. Das nach Entfernen der Lösungsmittel verbleibende Rohprodukt wird adsorptiv (SiO₂ Toluol) filtriert, und das Filtrat wird vollständig konzentriert. Nach mehrfacher Umkristallisation des Rückstandes aus Ethanol wird (±)-(*3R**,4a*R**,9b*S**)-7-Ethoxy-6-fluor-3-(4-vinyl-cyclohexyl)-1,2,3,4,4a,9b-hexahydro-dibenzofuran als farbloser Feststoff erhalten (Schmp. 132 °C).
K 132 N 157 I
Δε = -6.7
Δ n =0.121
γ ₁ = 911 mPa·s

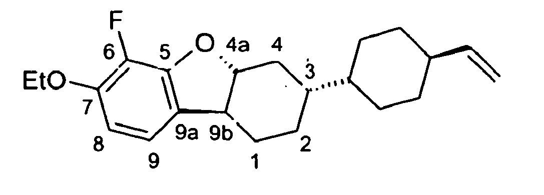

**¹H-NMR** (250 MHz, CHCl₃): δ = 6.74 (dm, 1H, J = 7.8 Hz, 9-H), 6.45 (dd, 1H, J = 8.0 Hz, J = 7.0 Hz, 8-H), 5.84-5.71 (m, 1H, H_{*vinyl*.}), 5.00-4.86 (m, 2H, H_{*vinyl*.}), 4.07 (q, 2H, J = 7.0 Hz, O**CH₂**CH₃), 4.00-3.89 (m, 1H, 4a-H), 2.78-2.67 (m, 1H, H_{*aliph*.}), 2.41-2.27 (m, 2H, H_{*aliph*.}), 1.91-1.74 (m, 7H, H_{*aliph*.}), 1.67-1.63 (m, 1H, *H_{aliph.}),* 1.42 (t, 3H, J = 7.0 Hz, OCH₂**CH₃**), 1.36-1.06 (m, 7H, *H*_{*aliph*.}).

**MS** (EI): m/z(%) = 344 (100, M⁺).

Analog zu den angeführten Beispielen werden unter Verwendung der entsprechenden Vorstufen die folgenden Verbindungen erhalten (Beispiele 1-1014; Tabellen 1 bis 4, Daten Tabelle 5):
Analog zu Beispiel 1: Beispiele 8 bis 62: 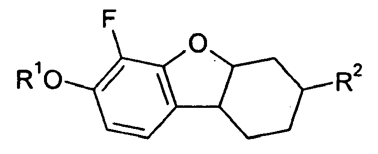
Analog zu Beispiel 1: Beispiele 63 bis 117: 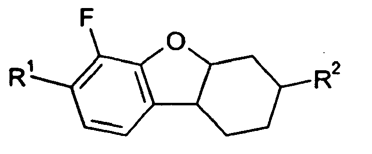
Analog zu Beispiel 3, 4 und 5: Beispiele 118 bis 173: 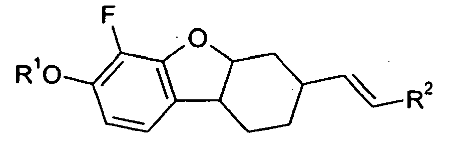
Analog zu Beispiel 3, 4 und 5: Beispiele 173 bis 227:

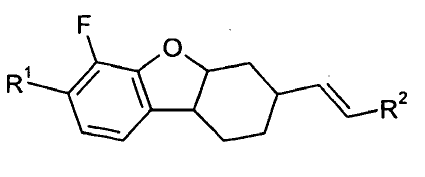

**Tabelle 1**

| **Beisp.** | **Beisp.** | **Beisp.** | **Beisp.** | **R¹** | **R²** |
|---|---|---|---|---|---|
| 8 | 63 | 118 | 173 | CH₃ | H |
| 9 | 64 | 119 | 174 | CH₃ | CH₃ |
| 10 | 65 | 120 | 175 | CH₃ | C₂H₅ |
| 11 | 66 | 121 | 176 | CH₃ | n-C₃H₇ |
| 12 | 67 | 122 | 177 | CH₃ | n-C₄H₉ |
| 13 | 68 | 123 | 178 | CH₃ | n-C₅H₁₁ |
| 14 | 69 | 124 | 179 | CH₃ | n-C₆H₁₃ |
| 15 | 70 | 125 | 180 | CH₃ | n-C₇H₁₅ |
| 16 | 71 | 126 | 181 | C₂H₅ | H |
| 17 | 72 | 127 | 182 | C₂H₅ | CH₃ |
| 18 | 73 | 128 | 183 | C₂H₅ | C₂H₅ |
| 19 | 74 | 129 | 184 | C₂H₅ | n-C₃H₇ |
| 20 | 75 | 130 | 185 | C₂H₅ | n-C₄H₉ |
| 21 | 76 | 131 | 186 | C₂H₅ | n-C₅H₁₁ |
| 22 | 77 | 132 | 187 | C₂H₅ | n-C₆H₁₃ |
| 23 | 78 | 133 | 188 | C₂H₅ | n-C₇H₁₅ |
| 24 | 79 | 134 | 189 | n-C₃H₇ | H |
| 25 | 80 | 135 | 190 | n-C₃H₇ | CH₃ |
| 26 | 81 | 136 | 191 | n-C₃H₇ | C₂H₅ |
| 27 | 82 | 137 | 192 | n-C₃H₇ | n-C₄H₉ |
| 28 | 83 | 138 | 193 | n-C₃H₇ | n-C₅H₁₁ |
| 29 | 84 | 139 | 194 | n-C₃H₇ | n-C₆H₁₃ |
| 30 | 85 | 140 | 195 | n-C₃H₇ | n-C₇H₁₅ |
| 31 | 86 | 141 | 196 | n-C₄H₉ | H |
| 32 | 87 | 142 | 197 | n-C₄H₉ | CH₃ |
| 33 | 88 | 143 | 198 | n-C₄H₉ | C₂H₅ |
| 34 | 89 | 144 | 199 | n-C₄H₉ | n-C₃H₇ |
| 35 | 90 | 145 | 200 | n-C₄H₉ | n-C₄H₉ |
| 36 | 91 | 146 | 201 | n-C₄H₉ | n-C₅H₁₁ |
| 37 | 92 | 147 | 202 | n-C₄H₉ | n-C₆H₁₃ |
| 38 | 93 | 148 | 203 | n-C₄H₉ | n-C₇H₁₅ |
| 39 | 94 | 149 | 204 | n-C₅H₁₁ | H |
| 40 | 95 | 150 | 205 | n-C₅H₁₁ | CH₃ |
| 41 | 96 | 151 | 206 | n-C₅H₁₁ | C₂H₅ |
| 42 | 97 | 152 | 207 | n-C₅H₁₁ | n-C₃H₇ |
| 43 | 98 | 153 | 208 | n-C₅H11 | n-C₄H₉ |
| 44 | 99 | 154 | 209 | n-C₅H₁₁ | n-C₅H₁₁ |
| 45 | 100 | 155 | 210 | n-C₅H₁₁ | n-C₆H₁₃ |
| 46 | 101 | 156 | 211 | n-C₅H₁₁ | n-C₇H₁₅ |
| 47 | 102 | 157 | 212 | n-C₆H₁₃ | H |
| 48 | 103 | 158 | 213 | n-C₆H₁₃ | CH₃ |
| 49 | 104 | 159 | 214 | n-C₆H₁₃ | C₂H₅ |
| 50 | 105 | 160 | 215 | n-C₆H₁₃ | n-C₃H₇ |
| 51 | 106 | 161 | 216 | n-C₆H₁₃ | n-C₄H₉ |
| 52 | 107 | 162 | 217 | n-C₆H₁₃ | n-C₅H₁₁ |
| 53 | 108 | 163 | 218 | n-C₆H₁₃ | n-C₆H₁₃ |
| 54 | 109 | 164 | 219 | n-C₆H₁₃ | n-C₇H₁₅ |
| 55 | 110 | 165 | 220 | n-C₇H₁₅ | H |
| 56 | 111 | 166 | 221 | n-C₇H₁₅ | CH₃ |
| 57 | 112 | 167 | 222 | n-C₇H₁₅ | C₂H₅ |
| 58 | 113 | 168 | 223 | n-C₇H₁₅ | n-C₃H₇ |
| 59 | 114 | 169 | 224 | n-C₇H₁₅ | n-C₄H₉ |
| 60 | 115 | 170 | 225 | n-C₇H₁₅ | n-C₅H₁₁ |
| 61 | 116 | 171 | 226 | n-C₇H₁₅ | n-C₆H₁₃ |
| 62 | 117 | 172 | 227 | n-C₇H₁₅ | n-C₇H₁₅ |

Analog zu Beispiel 2: Beispiele 228 bis 282: 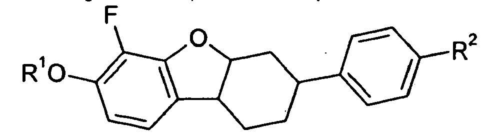
Analog zu Beispiel 2: Beispiele 283 bis 337: 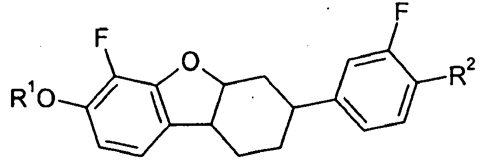
Analog zu Beispiel 2: Beispiele 338 bis 392: 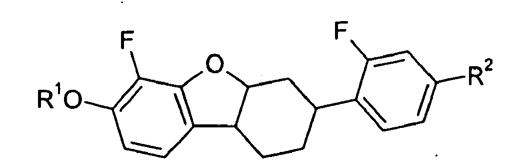
Analog zu Beispiel 2: Beispiele 393 bis 447: 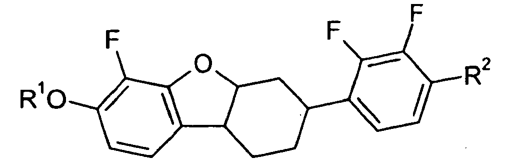
Analog zu Beispiel 2: Beispiele 448 bis 502: 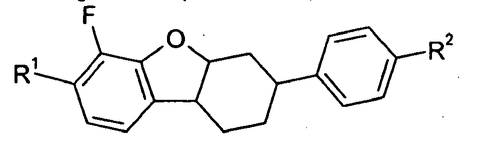
Analog zu Beispiel 2: Beispiele 503 bis 557: 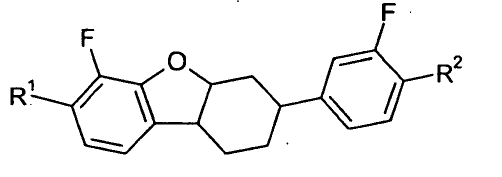
Analog zu Beispiel 2: Beispiele 558 bis 612: 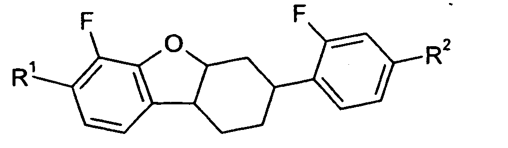
Analog zu Beispiel 2: Beispiele 613 bis 667:

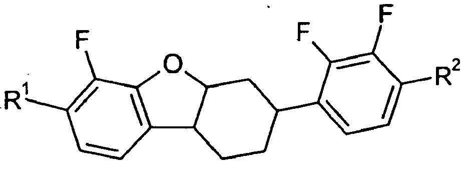

**Tabelle 2**

| **Beisp.** | **Beisp.** | **Beisp.** | **Beisp.** | **Beisp.** | **Beisp.** | **Beisp.** | **Beisp.** | **R¹** | **R²** |
|---|---|---|---|---|---|---|---|---|---|
| 228 | 283 | 338 | 393 | 448 | 503 | 558 | 613 | CH₃ | H |
| 229 | 284 | 339 | 394 | 449 | 504 | 559 | 614 | CH₃ | CH₃ |
| 230 | 285 | 340 | 395 | 450 | 505 | 560 | 615 | CH₃ | C₂H₅ |
| 231 | 286 | 341 | 396 | 451 | 506 | 561 | 616 | CH₃ | n-C₃H₇ |
| 232 | 287 | 342 | 397 | 452 | 507 | 562 | 617 | CH₃ | n-C₄H₉ |
| 233 | 288 | 343 | 398 | 453 | 508 | 563 | 618 | CH₃ | n-C₅H₁₁ |
| 234 | 289 | 344 | 399 | 454 | 509 | 564 | 619 | CH₃ | n-C₆H₁₃ |
| 235 | 290 | 345 | 400 | 455 | 510 | 565 | 620 | CH₃ | n-C₇H₁₅ |
| 236 | 291 | 346 | 401 | 456 | 511 | 566 | 621 | C₂H₅ | H |
| 237 | 292 | 347 | 402 | 457 | 512 | 567 | 622 | C₂H₅ | CH₃ |
| 238 | 293 | 348 | 403 | 458 | 513 | 568 | 623 | C₂H₅ | C₂H₅ |
| 239 | 294 | 349 | 404 | 459 | 514 | 569 | 624 | C₂H₅ | n-C₃H₇ |
| 240 | 295 | 350 | 405 | 460 | 515 | 570 | 625 | C₂H₅ | n-C₄H₉ |
| 241 | 296 | 351 | 406 | 461 | 516 | 571 | 626 | C₂H₅ | n-C₅H₁₁ |
| 242 | 297 | 352 | 407 | 462 | 517 | 572 | 627 | C₂H₅ | n-C₆H₁₃ |
| 243 | 298 | 353 | 408 | 463 | 518 | 573 | 628 | C₂H₅ | n-C₇H₁₅ |
| 244 | 299 | 354 | 409 | 464 | 519 | 574 | 629 | n-C₃H₇ | H |
| 245 | 300 | 355 | 410 | 465 | 520 | 575 | 630 | n-C₃H₇ | CH₃ |
| 246 | 301 | 356 | 411 | 466 | 521 | 576 | 631 | n-C₃H₇ | C₂H₅ |
| 247 | 302 | 357 | 412 | 467 | 522 | 577 | 632 | n-C₃H₇ | n-C₄H₉ |
| 248 | 303 | 358 | 413 | 468 | 523 | 578 | 633 | n-C₃H₇ | n-C₅H₁₁ |
| 249 | 304 | 359 | 414 | 469 | 524 | 579 | 634 | n-C₃H₇ | n-C₆H₁₃ |
| 250 | 305 | 360 | 415 | 470 | 525 | 580 | 635 | n-C₃H₇ | n-C₇H₁₅ |
| 251 | 306 | 361 | 416 | 471 | 526 | 581 | 636 | n-C₄H₉ | H |
| 252 | 307 | 362 | 417 | 472 | 527 | 582 | 637 | n-C₄H₉ | CH₃ |
| 253 | 308 | 363 | 418 | 473 | 528 | 583 | 638 | n-C₄H₉ | C₂H₅ |
| 254 | 309 | 364 | 419 | 474 | 529 | 584 | 639 | n-C₄H₉ | n-C₃H₇ |
| 255 | 310 | 365 | 420 | 475 | 530 | 585 | 640 | n-C₄H₉ | n-C₄H₉ |
| 256 | 311 | 366 | 421 | 476 | 531 | 586 | 641 | n-C₄H₉ | n-C₅H₁₁ |
| 257 | 312 | 367 | 422 | 477 | 532 | 587 | 642 | n-C₄H₉ | n-C₆H₁₃ |
| 258 | 313 | 368 | 423 | 478 | 533 | 588 | 643 | n-C₄H₉ | n-C₇H₁₅ |
| 259 | 314 | 369 | 424 | 479 | 534 | 589 | 644 | n-C₅H₁₁ | H |
| 260 | 315 | 370 | 425 | 480 | 535 | 590 | 645 | n-C₅H₁₁ | CH₃ |
| 261 | 316 | 371 | 426 | 481 | 536 | 591 | 646 | n-C₅H₁₁ | C₂H₅ |
| 262 | 317 | 372 | 427 | 482 | 537 | 592 | 647 | n-C₅H₁₁ | n-C₃H₇ |
| 263 | 318 | 373 | 428 | 483 | 538 | 593 | 648 | n-C₅H₁₁ | n-C₄H₉ |
| 264 | 319 | 374 | 429 | 484 | 539 | 594 | 649 | n-C₅H₁₁ | n-C₅H₁₁ |
| 265 | 320 | 375 | 430 | 485 | 540 | 595 | 650 | n-C₅H₁₁ | n-C₆H₁₃ |
| 266 | 321 | 376 | 431 | 486 | 541 | 596 | 651 | n-C₅H₁₁ | n-C₇H₁₅ |
| 267 | 322 | 377 | 432 | 487 | 542 | 597 | 652 | n-C₆H₁₃ | H |
| 268 | 323 | 378 | 433 | 488 | 543 | 598 | 653 | n-C₆H₁₃ | CH₃ |
| 269 | 324 | 379 | 434 | 489 | 544 | 599 | 654 | n-C₆H₁₃ | C₂H₅ |
| 270 | 325 | 380 | 435 | 490 | 545 | 600 | 655 | n-C₆H₁₃ | n-C₃H₇ |
| 271 | 326 | 381 | 436 | 491 | 546 | 601 | 656 | n-C₆H₁₃ | n-C₄H₉ |
| 272 | 327 | 382 | 437 | 492 | 547 | 602 | 657 | n-C₆H₁₃ | n-C₅H₁₁ |
| 273 | 328 | 383 | 438 | 493 | 548 | 603 | 658 | n-C₆H₁₃ | n-C₆H₁₃ |
| 274 | 329 | 384 | 439 | 494 | 549 | 604 | 659 | n-C₆H₁₃ | n-C₇H₁₅ |
| 275 | 330 | 385 | 440 | 495 | 550 | 605 | 660 | n-C₇H₁₅ | H |
| 276 | 331 | 386 | 441 | 496 | 551 | 606 | 661 | n-C₇H₁₅ | CH₃ |
| 277 | 332 | 387 | 442 | 497 | 552 | 607 | 662 | n-C₇H₁₅ | C₂H₅ |
| 278 | 333 | 388 | 443 | 498 | 553 | 608 | 663 | n-C₇H₁₅ | n-C₃H₇ |
| 279 | 334 | 389 | 444 | 499 | 554 | 609 | 664 | n-C₇H₁₅ | n-C₄H₉ |
| 280 | 335 | 390 | 445 | 500 | 555 | 610 | 665 | n-C₇H₁₅ | n-C₅H₁₁ |
| 281 | 336 | 391 | 446 | 501 | 556 | 611 | 666 | n-C₇H₁₅ | n-C₆H₁₃ |
| 282 | 337 | 392 | 447 | 502 | 557 | 612 | 667 | n-C₇H₁₅ | n-C₇H₁₅ |

Analog zu Beispiel 2: Beispiele 668 bis 715: 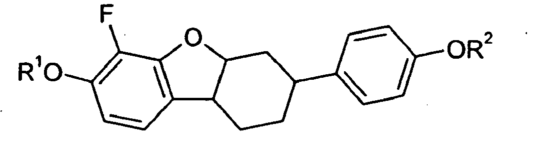
Analog zu Beispiel 2: Beispiele 716 bis 767: 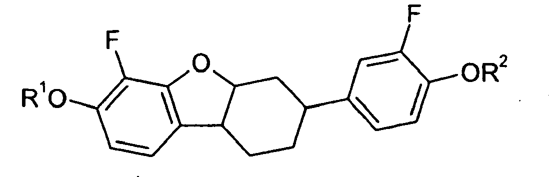
Analog zu Beispiel 2: Beispiele 768 bis 815: 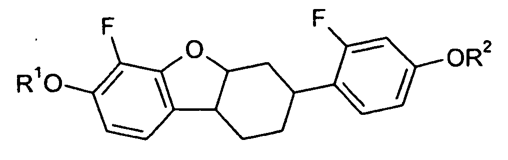
Analog zu Beispiel 2: Beispiele 816 bis 863: 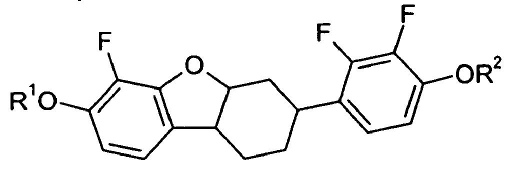
Analog zu Beispiel 2: Beispiele 864 bis 911: 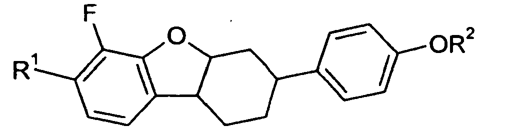
Analog zu Beispiel 2: Beispiele 912 bis 959:

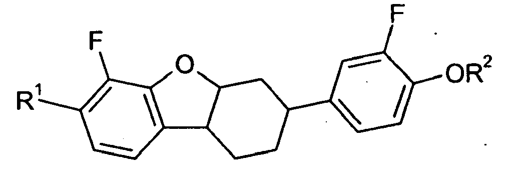

**Tabelle 3**

| **Beisp.** | **Beisp.** | **Beisp.** | **Beisp.** | **Beisp.** | **Beisp.** | **R¹** | **R²** |
|---|---|---|---|---|---|---|---|
| 668 | 716 | 768 | 816 | 864 | 912 | CH₃ | CH₃ |
| 669 | 717 | 769 | 817 | 865 | 913 | CH₃ | C₂H₅ |
| 670 | 718 | 770 | 818 | 866 | 914 | CH₃ | n-C₃H₇ |
| 671 | 719 | 771 | 819 | 867 | 915 | CH₃ | n-C₄H₉ |
| 672 | 720 | 772 | 820 | 868 | 916 | CH₃ | n-C₅H₁₁ |
| 673 | 721 | 773 | 821 | 869 | 917 | CH₃ | n-C₆H₁₃ |
| 674 | 722 | 774 | 822 | 870 | 918 | CH₃ | n-C₇H₁₅ |
| 675 | 723 | 775 | 823 | 871 | 919 | C₂H₅ | CH₃ |
| 676 | 724 | 776 | 824 | 872 | 920 | C₂H₅ | C₂H₅ |
| 677 | 725 | 777 | 825 | 873 | 921 | C₂H₅ | n-C₃H₇ |
| 678 | 726 | 778 | 826 | 874 | 922 | C₂H₅ | n-C₄H₉ |
| 679 | 727 | 779 | 827 | 875 | 923 | C₂H₅ | n-C₅H₁₁ |
| 680 | 728 | 780 | 828 | 876 | 924 | C₂H₅ | n-C₆H₁₃ |
| 681 | 729 | 781 | 829 | 877 | 925 | C₂H₅ | n-C₇H₁₅ |
| 682 | 730 | 782 | 830 | 878 | 926 | n-C₃H₇ | CH₃ |
| 683 | 731 | 783 | 831 | 879 | 927 | n-C₃H₇ | C₂H₅ |
| 684 | 732 | 784 | 832 | 880 | 928 | n-C₃H₇ | n-C₄H₉ |
| 685 | 733 | 785 | 833 | 881 | 929 | n-C₃H₇ | n-C₅H₁₁ |
| 686 | 734 | 786 | 834 | 882 | 930 | n-C₃H₇ | n-C₆H₁₃ |
| 687 | 735 | 787 | 835 | 883 | 931 | n-C₃H₇ | n-C₇H₁₅ |
| 688 | 736 | 788 | 836 | 884 | 932 | n-C₄H₉ | CH₃ |
| 689 | 737 | 789 | 837 | 885 | 933 | n-C₄H₉ | C₂H₅ |
| 690 | 738 | 790 | 838 | 886 | 934 | n-C₄H₉ | n-C₃H₇ |
| 691 | 739 | 791 | 839 | 887 | 935 | n-C₄H₉ | n-C₄H₉ |
| 692 | 740 | 792 | 840 | 888 | 936 | n-C₄H₉ | n-C₅H₁₁ |
| 693 | 741 | 793 | 841 | 889 | 937 | n-C₄H₉ | n-C₆H₁₃ |
| 694 | 742 | 794 | 842 | 890 | 938 | n-C₄H₉ | n-C₇H₁₅ |
| 695 | 743 | 795 | 843 | 891 | 939 | n-C₅H₁₁ | CH₃ |
| 696 | 744 | 796 | 844 | 892 | 940 | n-C₅H₁₁ | C₂H₅ |
| 697 | 745 | 797 | 845 | 893 | 941 | n-C₅H₁₁ | n-C₃H₇ |
| 698 | 746 | 798 | 846 | 894 | 942 | n-C₅H₁₁ | n-C₄H₉ |
| 699 | 747 | 799 | 847 | 895 | 943 | n-C₅H₁₁ | n-C₅H₁₁ |
| 700 | 748 | 800 | 848 | 896 | 944 | n-C₅H₁₁ | n-C₆H₁₃ |
| 701 | 749 | 801 | 849 | 897 | 945 | n-C₅H₁₁ | n-C₇H₁₅ |
| 702 | 750 | 802 | 850 | 898 | 946 | n-C₆H₁₃ | CH₃ |
| 703 | 751 | 803 | 851 | 899 | 947 | n-C₆H₁₃ | C₂H₅ |
| 704 | 752 | 804 | 852 | 900 | 948 | n-C₆H₁₃ | n-C₃H₇ |
| 705 | 753 | 805 | 853 | 901 | 949 | n-C₆H₁₃ | n-C₄H₉ |
| 706 | 754 | 806 | 854 | 902 | 950 | n-C₆H₁₃ | n-C₅H₁₁ |
| 707 | 755 | 807 | 855 | 903 | 951 | n-C₆H₁₃ | n-C₆H₁₃ |
| 708 | 756 | 808 | 856 | 904 | 952 | n-C₆H₁₃ | n-C₇H₁₅ |
| 709 | 757 | 809 | 857 | 905 | 953 | n-C₇H₁₅ | CH₃ |
| 710 | 758 | 810 | 858 | 906 | 954 | n-C₇H₁₅ | C₂H₅ |
| 711 | 759 | 811 | 859 | 907 | 955 | n-C₇H₁₅ | n-C₃H₇ |
| 712 | 760 | 812 | 860 | 908 | 956 | n-C₇H₁₅ | n-C₄H₉ |
| 713 | 761 | 813 | 861 | 909 | 957 | n-C₇H₁₅ | n-C₅H₁₁ |
| 714 | 762 | 814 | 862 | 910 | 958 | n-C₇H₁₅ | n-C₆H₁₃ |
| 715 | 763 | 815 | 863 | 911 | 959 | n-C₇H₁₅ | n-C₇H₁₅ |

Analog zu Beispiel 6: Beispiele 960 bis 1014: 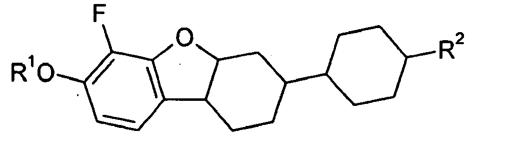
Analog zu Beispiel 6: Beispiele 1015 bis 1069: 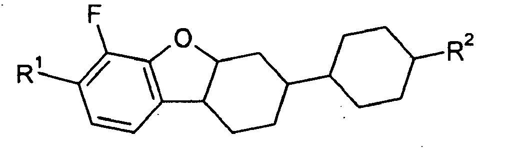
Analog zu Beispiel 7: Beispiele 1070 bis 1124: 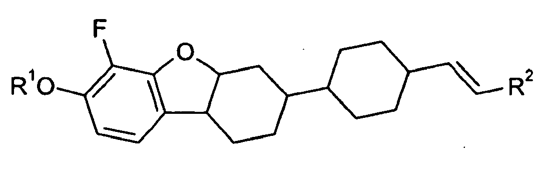
Analog zu Beispiel 7: Beispiele 1125 bis 1179:

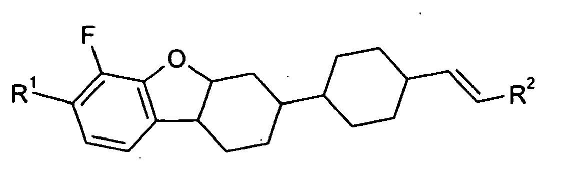

**Tabelle 4**

| **Beisp.** | **Beisp.** | **Beisp.** | **Beisp.** | **R¹** | **R²** |
|---|---|---|---|---|---|
| 960 | 1015 | 1070 | 1125 | CH₃ | H |
| 961 | 1016 | 1071 | 1126 | CH₃ | CH₃ |
| 962 | 1017 | 1072 | 1127 | CH₃ | C₂H₅ |
| 963 | 1018 | 1073 | 1128 | CH₃ | n-C₃H₇ |
| 964 | 1019 | 1074 | 1129 | CH₃ | n-C₄H₉ |
| 965 | 1020 | 1075 | 1130 | CH₃ | n-C₅H₁₁ |
| 966 | 1021 | 1076 | 1131 | CH₃ | n-C₆H₁₃ |
| 967 | 1022 | 1077 | 1132 | CH₃ | n-C₇H₁₅ |
| 968 | 1023 | 1078 | 1133 | C₂H₅ | H |
| 969 | 1024 | 1079 | 1134 | C₂H₅ | CH₃ |
| 970 | 1025 | 1080 | 1135 | C₂H₅ | C₂H₅ |
| 971 | 1026 | 1081 | 1136 | C₂H₅ | n-C₃H₇ |
| 972 | 1027 | 1082 | 1137 | C₂H₅ | n-C₄H₉ |
| 973 | 1028 | 1083 | 1138 | C₂H₅ | n-C₅H₁₁ |
| 974 | 1029 | 1084 | 1139 | C₂H₅ | n-C₆H₁₃ |
| 975 | 1030 | 1085 | 1140 | C₂H₅ | n-C₇H₁₅ |
| 976 | 1031 | 1086 | 1141 | n-C₃H₇ | H |
| 977 | 1032 | 1087 | 1142 | n-C₃H₇ | CH₃ |
| 978 | 1033 | 1088 | 1143 | n-C₃H₇ | C₂H₅ |
| 979 | 1034 | 1089 | 1144 | n-C₃H₇ | n-C₄H₉ |
| 980 | 1035 | 1090 | 1145 | n-C₃H₇ | n-C₅H₁₁ |
| 981 | 1036 | 1091 | 1146 | n-C₃H₇ | n-C₆H₁₃ |
| 982 | 1037 | 1092 | 1147 | n-C₃H₇ | n-C₇H₁₅ |
| 983 | 1038 | 1093 | 1148 | n-C₄H₉ | H |
| 984 | 1039 | 1094 | 1149 | n-C₄H₉ | CH₃ |
| 985 | 1040 | 1095 | 1150 | n-C₄H₉ | C₂H₅ |
| 986 | 1041 | 1096 | 1151 | n-C₄H₉ | n-C₃H₇ |
| 987 | 1042 | 1097 | 1152 | n-C₄H₉ | n-C₄H₉ |
| 988 | 1043 | 1098 | 1153 | n-C₄H₉ | n-C₅H₁₁ |
| 989 | 1044 | 1099 | 1154 | n-C₄H₉ | n-C₆H₁₃ |
| 990 | 1045 | 1100 | 1155 | n-C₄H₉ | n-C₇H₁₅ |
| 991 | 1046 | 1101 | 1156 | n-C₅H₁₁ | H |
| 992 | 1047 | 1102 | 1157 | n-C₅H₁₁ | CH₃ |
| 993 | 1048 | 1103 | 1158 | n-C₅H₁₁ | C₂H₅ |
| 994 | 1049 | 1104 | 1159 | n-C₅H₁₁ | n-C₃H₇ |
| 995 | 1050 | 1105 | 1160 | n-C₅H₁₁ | n-C₄H₉ |
| 996 | 1051 | 1106 | 1161 | n-C₅H₁₁ | n-C₅H₁₁ |
| 997 | 1052 | 1107 | 1162 | n-C₅H₁₁ | n-C₆H₁₃ |
| 998 | 1053 | 1108 | 1163 | n-C₅H₁₁ | n-C₇H₁₅ |
| 999 | 1054 | 1109 | 1164 | n-C₆H₁₃ | H |
| 1000 | 1055 | 1110 | 1165 | n-C₆H₁₃ | CH₃ |
| 1001 | 1056 | 1111 | 1166 | n-C₆H₁₃ | C₂H₅ |
| 1002 | 1057 | 1112 | 1167 | n-C₆H₁₃ | n-C₃H₇ |
| 1003 | 1058 | 1113 | 1168 | n-C₆H₁₃ | n-C₄H₉ |
| 1004 | 1059 | 1114 | 1169 | n-C₆H₁₃ | n-C₅H₁₁ |
| 1005 | 1060 | 1115 | 1170 | n-C₆H₁₃ | n-C₆H₁₃ |
| 1006 | 1061 | 1116 | 1171 | n-C₆H₁₃ | n-C₇H₁₅ |
| 1007 | 1062 | 1117 | 1172 | n-C₇H₁₅ | H |
| 1008 | 1063 | 1118 | 1173 | n-C₇H₁₅ | CH₃ |
| 1009 | 1064 | 1119 | 1174 | n-C₇H₁₅ | C₂H₅ |
| 1010 | 1065 | 1120 | 1175 | n-C₇H₁₅ | n-C₃H₇ |
| 1011 | 1066 | 1121 | 1176 | n-C₇H₁₅ | n-C₄H₉ |
| 1012 | 1067 | 1122 | 1177 | n-C₇H₁₅ | n-C₅H₁₁ |
| 1013 | 1068 | 1123 | 1178 | n-C₇H₁₅ | n-C₆H₁₃ |
| 1014 | 1069 | 1124 | 1179 | n-C₇H₁₅ | n-C₇H₁₅ |

Werte zu einzelnen Verbindungen aus allen Tabellen 1-4:

**Tabelle 5**

| **Beispiel-Nr.** | **Δε** | **Δn** | **γ₁** | **Phasensequenz** |
|---|---|---|---|---|
| 34 | -5.1 | 0.088 | 132 | K 114 I |
| 36 | -5.0 | 0.090 | 171 | K 116 I |
| 99 | -1.4 | 0.060 | 130 | K 97 I |
| 141 | -5.4 | 0.098 | 102 | K 120 I |
| 142 | -5.4 | 0.094 | 149 | K 124 I |
| 144 | -4.8 | 0.100 | 164 | K 125 I |
| 204 | -1.9 | 0.062 | 59 | K 78 I |

## Patentansprüche

1. Verbindungen der allgemeinen Formel I: 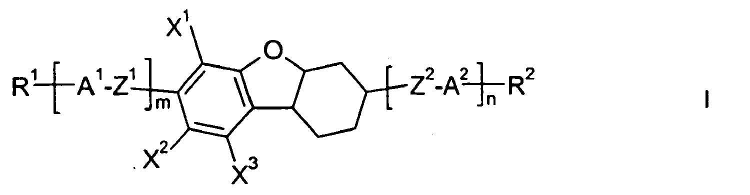 worin
m und n jeweils unabhängig voneinander 0, 1 oder 2 sind,
X¹, X² und X³ jeweils unabhängig voneinander H, Halogen, CN oder CF₃ bedeuten,
A¹ und A² jeweils unabhängig voneinander 1,4-Phenylen, worin =CH- ein- oder zweimal durch =N- ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substitu- iert sein kann,
1,4-Cyclohexylen, 1,4-Cyclohexenylen oder 1,4- Cyclohexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F und/oder -Cl substituiert sein können,
Bicyclo[1.1.1]pentan-1,3-diyl, Bicyclo[2.2.2]octan-1,4- diyl, Spiro[3.3]heptan-2,6-diyl, Tetrahydropyran-2,5- diyl oder 1,3-Dioxan-2,5-diyl,
bedeutet;
Z¹ und Z² jeweils unabhängig voneinander eine Einfach- bindung, -CF₂O-, -OCF₂-, -CH₂CH₂-, -CF₂CF₂-, -CF₂CH₂-, -CH₂CF₂-, -CHF-CHF-, -(CO)O-, -O(CO)-, -CH₂O-, -OCH₂-, -CF=CH-, -CH=CF-, -CF=CF-, -CH=CH- oder -C≡C- bedeuten;
R¹ und R² unabhängig voneinander, Wasserstoff, einen unsubstituierten, einen einfach mit -CN oder -CF₃ oder einen einfach oder mehrfach mit -F, -Cl, -Br und/oder -I substituierten Alkanyl-, Alkoxy-, Alkenyl- oder Alkinylrest mit 1 bis 15 bzw. 2-bis 15 C-Atomen, wobei in diesen Resten auch eine oder mehrere CH₂- Gruppen jeweils unabhängig voneinander durch -O-, -S-, -SO₂-, -CO-, -(CO)O-, -O(CO)- oder -O-CO-O- so ersetzt sein können, dass Heteroatome nicht direkt verknüpft sind, -F, -Cl, -Br, -I, -CN, -SCN, -NCS oder -SF₅ bedeuten;
wobei
A¹, A², Z¹, Z² jeweils die gleiche oder unterschiedliche Bedeutungen haben können, wenn m beziehungsweise n größer als 1 sind, und
wobei
für den Fall, dass zugleich n = 0 und m = 0 ist und X¹, X² und X³ ungleich F sind, R¹ und R² dann nicht gleichzeitig H bedeuten.

2. Verbindungen nach Anspruch 1, **dadurch gekennzeichnet, dass** einer oder mehrere der Reste X¹, X² und X³ ein F bedeuten.

3. Verbindungen nach Anspruch 1, **dadurch gekennzeichnet, dass** der Substituent X¹ F, Cl, CN oder CF₃ bedeutet und X² und X³ Wasserstoff bedeuten.

4. Verbindungen nach einem oder mehreren der Ansprüche 1 bis 3, **dadurch gekennzeichnet, dass** X¹ F bedeutet.

5. Verbindungen nach einem oder mehreren der Ansprüche 1 bis 4,
**dadurch gekennzeichnet, dass**
Z¹ und Z² unabhängig voneinander eine Einfachbindung, -CF₂O-, -OCF₂-, -CF₂CF₂-, -CH=CH-, -CF=CH-, -CH=CF- oder -CF=CF- bedeuten.

6. Verbindungen nach einem oder mehreren der Ansprüche 1 bis 5,
**dadurch gekennzeichnet, dass** A¹ und A² unabhängig voneinander einen Ring der Formeln 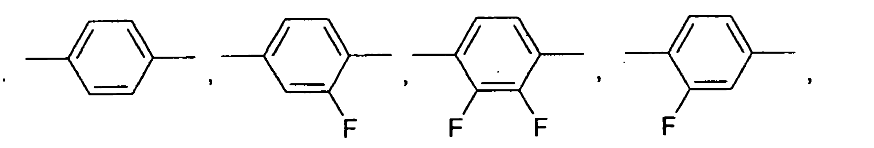 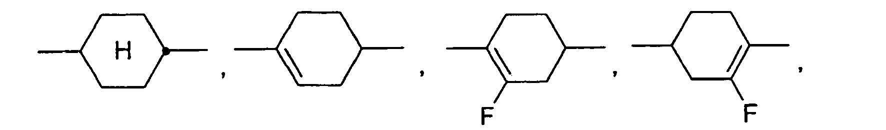 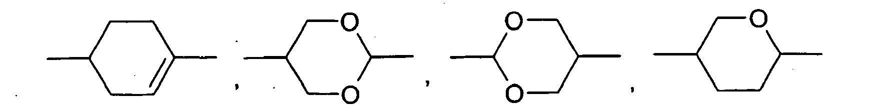 oder 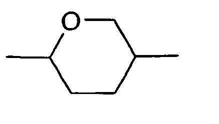 bedeuten.

7. Verbindungen nach einem oder mehreren der Ansprüche 1 bis 6, **dadurch gekennzeichnet, dass**
R¹ und R² jeweils unabhängig voneinander ein Alkanylrest, Alkoxyrest oder Alkenylrest mit 1 bis 7 bzw. 2 bis 7 Kohlenstoffatomen sind, wobei jeder dieser Reste unsubstituiert oder mit Halogen einfach oder mehrfach substituiert ist, oder Fluor oder Wasserstoff bedeutet.

8. Verbindungen nach einem oder mehreren der Ansprüche 1 bis 7, **dadurch gekennzeichnet, dass**
m und n beide null sind, und
R¹ und R² jeweils unabhängig voneinander ein unverzweigter Alkanylrest, Alkoxyrest oder Alkenylrest mit 1 bis 7 bzw. 2 bis 7 Kohlenstoffatomen sind.

9. Verbindungen nach einem oder mehreren der Ansprüche 1 bis 7, **dadurch gekennzeichnet, dass** m + n = 1 ist und
A¹, A² unabhängig voneinander 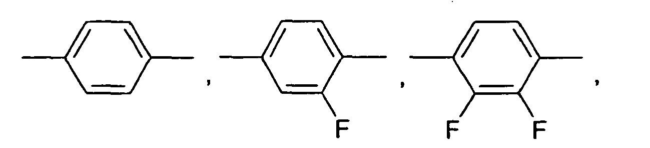 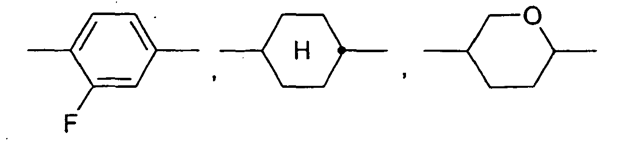
oder 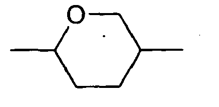 bedeuten.

10. Verbindungen nach einem oder mehreren der Ansprüche 1 bis 9, **dadurch gekennzeichnet, dass** m = 0 ist, und
R¹ ein Alkoxyrest oder Alkenyloxyrest mit 2 bis 7 Kohlenstoffatomen ist.

11. Verwendung einer oder mehrerer Verbindungen nach einem oder mehreren der vorangehenden Ansprüche in flüssigkristallinen Medien.

12. Flüssigkristallines Medium enthaltend mindestens zwei Verbindungen, **dadurch gekennzeichnet, dass** es mindestens eine Verbindung nach einem oder mehreren der Ansprüche 1 bis 10 enthält.

13. Elektrooptisches Anzeigeelement, enthaltend ein flüssigkristallines Medium nach Anspruch 12.

14. Verfahren zur Herstellung von Verbindungen nach einem oder mehreren der Ansprüche 1 bis 10, **dadurch gekennzeichnet, dass** es einen Verfahrensschritt umfasst, wobei eine entsprechend substituierte 2-(2-Halophenyl)cyclohexanolverbindung zu einem Tetrahydrodibenzofuranderivat cyclisiert wird.

## Claims

1. Compounds of the general formula I: 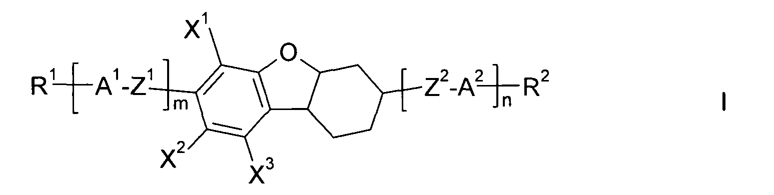 in which
m and n are each, independently of one another, 0, 1 or 2,
X¹, X² and X³ each, independently of one another, denote H, halo- gen, CN or CF₃,
A¹ and A² each, independently of one another, denote 1,4- phenylene, in which =CH- may be replaced once or twice by =N- and which may be unsubstituted or mono- to tetrasubstituted, independently of one an- other, by -CN, -F, -Cl, -Br, -I, unsubstituted or mono- or polyfluorine- and/or -chlorine-substituted C₁-C₆- alkanyl, unsubstituted or mono- or polyfluorine- and/or -chlorine-substituted C₁-C₆-alkoxy, 1,4-cyclohexylene, 1,4-cyclohexenylene or 1,4-cyclo- hexadienylene, in which -CH₂- may be replaced once or twice, independently of one another, by -O- or -S- in such a way that heteroatoms are not linked direct- ly, and which may be unsubstituted or mono- or poly- substituted by -F and/or -Cl,
bicyclo[1.1.1]pentane-1,3-diyl, bicyclo[2.2.2]octane- 1,4-diyl, spiro[3.3]heptane-2,6-diyl, tetrahydropyran- 2,5-diyl or 1,3-dioxane-2,5-diyl;
Z¹ and Z² each, independently of one another, denote a single bond, -CF₂O-, -OCF₂-, -CH₂CH₂-, -CF₂CF₂-, -CF₂CH₂-, -CH₂CF₂-, -CHF-CHF-, -(CO)O-, -O(CO)-, -CH₂O-, -OCH₂-, -CF=CH-, -CH=CF-, -CF=CF-, -CH=CH- or -C≡C-;
R¹ and R², independently of one another, denote hydrogen, an alkanyl, alkoxy, alkenyl or alkynyl radical having 1 to 15 or 2 to 15 C atoms respectively which is unsubsti- tuted, monosubstituted by -CN or -CF₃ or mono- or polysubstituted by -F, -Cl, -Br and/or -I, where one or more CH₂ groups in these radicals may also each, independently of one another, be replaced by -O-, -S-, -SO₂-, -CO-, -(CO)O-, -O(CO)- or -O-CO-O- in such a way that heteroatoms are not linked directly, -F, -Cl, -Br, -I, -CN, -SCN, -NCS or -SF₅;
where
A¹, A², Z¹, Z² may each have identical or different meanings if m or n respectively is greater than 1, and
where
in the case where simultaneously n = 0 and m = 0 and X¹, X² and X³ are not equal to F, R¹ and R² then do not simultaneously denote H.

2. Compounds according to Claim 1, **characterised in that** one or more of the radicals X¹, X² and X³ denote F.

3. Compounds according to Claim 1, **characterised in that** the substituent X¹ denotes F, Cl, CN or CF₃ and X² and X³ denote hydrogen.

4. Compounds according to one or more of Claims 1 to 3, **characterised in that** X¹ denotes F.

5. Compounds according to one or more of Claims 1 to 4, **characterised in that**
Z¹ and Z², independently of one another, denote a single bond, -CF₂O-, -OCF₂-, -CF₂CF₂-, -CH=CH-, -CF=CH-, -CH=CF- or -CF=CF-.

6. Compounds according to one or more of Claims 1 to 5, **characterised in that** A¹ and A², independently of one another, denote a ring of the formula 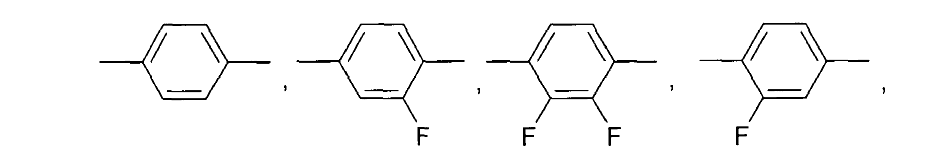 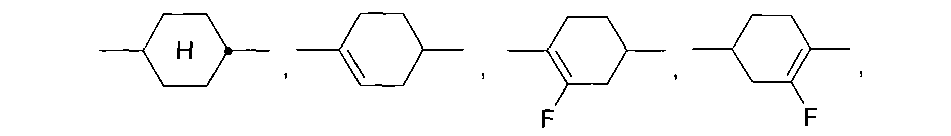 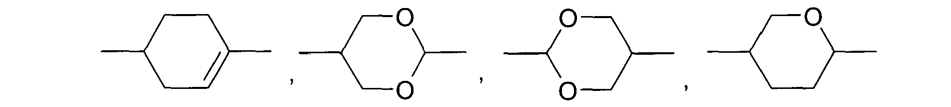 or

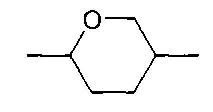

7. Compounds according to one or more of Claims 1 to 6, **characterised in that**
R¹ and R² are each, independently of one another, an alkanyl radi- cal, alkoxy radical or alkenyl radical having 1 to 7 or 2 to 7 carbon atoms respectively, where each of these radi- cals is unsubstituted or mono- or polysubstituted by halogen, or denotes fluorine or hydrogen.

8. Compounds according to one or more of Claims 1 to 7, **characterised in that**
m and n are both zero, and
R¹ and R² are each, independently of one another, an unbranched alkanyl radical, alkoxy radical or alkenyl radical having 1 to 7 or 2 to 7 carbon atoms respectively.

9. Compounds according to one or more of Claims 1 to 7, **characterised in that** m + n = 1, and
A¹, A², independently of one another, denote 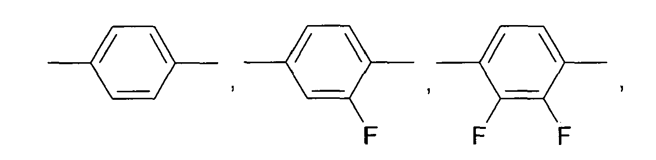 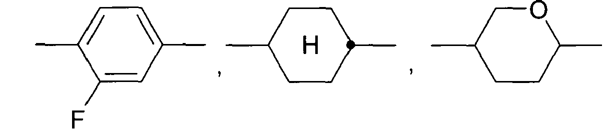
or

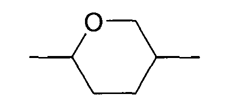

10. Compounds according to one or more of Claims 1 to 9, **characterised in that** m = 0, and
R¹ is an alkoxy radical or alkenyloxy radical having 2 to 7 carbon atoms.

11. Use of one or more compounds according to one or more of the preceding claims in liquid-crystalline media.

12. Liquid-crystalline medium comprising at least two compounds, **characterised in that** it comprises at least one compound according to one or more of Claims 1 to 10.

13. Electro-optical display element containing a liquid-crystalline medium according to Claim 12.

14. Process for the preparation of compounds according to one or more of Claims 1 to 10, **characterised in that** it includes a process step in which an appropriately substituted 2-(2-halophenyl)cyclohexanol compound is cyclised to give a tetrahydrodibenzofuran derivative.

## Revendications

1. Composés de la formule générale I : 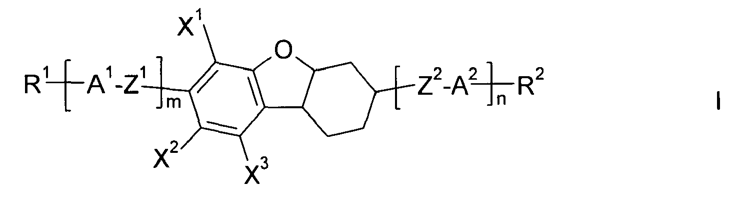 dans laquelle
m et n sont chacun, indépendamment l'un de l'autre, 0, 1 ou 2,
X¹, X² et X³ chacun, indépendamment l'un de l'autre, représen- tent H, halogène, CN ou CF₃,
A¹ et A² chacun, indépendamment l'un de l'autre, représen- tent 1,4-phénylène, dans lequel =CH- peut être rem- placé une fois ou deux fois par =N- et qui peut être non substitué ou mono- à tétrasubstitué, indé-pen- damment l'un de l'autre, par -CN, -F, -CI, -Br, -I, C₁-C₆-alkanyle non substitué ou mono- ou polysubs- titué avec fluor et/ou chlore, C₁-C₆-alcoxy non subs- titué ou mono- ou polysubstitué avec fluor et/ou chlore,
1,4-cyclohexylène, 1,4-cyclohexénylène ou 1,4-cyclo- hexadiénylène, dans lequel -CH₂- peut être remplacé une fois ou deux fois, indépendamment l'un de l'autre, par -O- ou -S- de telle sorte que des hétéro- atomes ne soient pas liés directement, et qui peut être non substitué ou mono- ou polysubstitué par -F et/ou -CI,
bicyclo[1.1.1]pentane-1,3-diyle, bicyclo[2.2.2]octane- 1,4-diyle, spiro[3.3]heptane-2,6-diyle, tétrahydro- pyranne-2,5-diyle ou 1,3-dioxane-2,5-diyle ;
Z¹ et Z² chacun, indépendamment l'un de l'autre, représen- tent une liaison simple, -CF₂O-, -OCF₂-, -CH₂CH₂-, -CF₂CF₂-, -CF₂CH₂-, -CH₂CF₂-, -CHF-CHF-, -(CO)O-, -O(CO)-, -CH₂O-, -OCH₂-, -CF=CH-, -CH=CF-, -CF=CF-, -CH=CH- ou -C≡C- ;
R¹ et R², indépendamment l'un de l'autre, représentent hydro- gène, un radical alkanyle, alcoxy, alkényle ou alky- nyle ayant respectivement 1 à 15 ou 2 à 15 atomes de C qui est non substitué, nonosubstitué par -CN ou -CF₃ ou mono- ou polysubstitué par -F, -Cl, -Br et/ou -I, dans lequel un ou plusieurs groupes CH₂ dans ces radicaux peuvent également chacun, indépendam- ment l'un de l'autre, être remplacés par -O-, -S-, -SO₂-, -CO-, -(CO)O-, -O(CO)- ou -O-CO-O- de telle sorte que des hétéroatomes ne soient pas liés directement, -F, -CI, -Br, -I, -CN, -SCN, -NCS ou -SF₅;
dans lequel
A¹, A², Z¹, Z² peuvent chacun avoir des significations identiques ou différentes si m ou n est respectivement supérieur à 1, et
dans lequel
dans le cas où simultanément n = 0 et m = 0 et X¹, X² et X³ ne sont pas égaux à F, alors R¹ et R² ne représentent pas simultanément H.

2. Composés selon la revendication 1, **caractérisés en ce que** un ou plusieurs des radicaux X¹, X² et X³ représentent F.

3. Composés selon la revendication 1, **caractérisés en ce que** le substituant X¹ représente F, Cl, CN ou CF₃ et X² et X³ représentent hydrogène.

4. Composés selon une ou plusieurs des revendications 1 à 3, **caractérisés en ce que** X¹ représente F.

5. Composés selon une ou plusieurs des revendications 1 à 4, **caractérisés en ce que**
Z¹ et Z², indépendamment l'un de l'autre, représentent une liai- son simple, -CF₂O-, -OCF₂-, -CF₂CF₂-, -CH=CH-, -CF=CH-, -CH=CF- ou -CF=CF-.

6. Composés selon une ou plusieurs des revendications 1 à 5, **caractérisés en ce que** A¹ et A², indépendamment l'un de l'autre, représentent un cycle de la formule 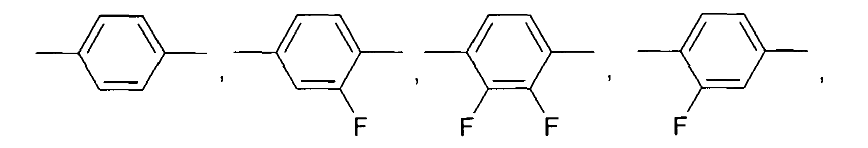 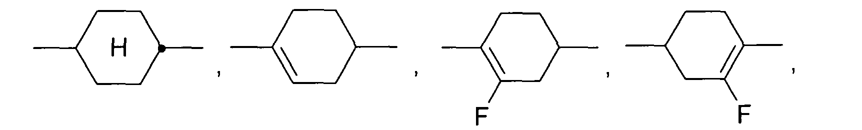 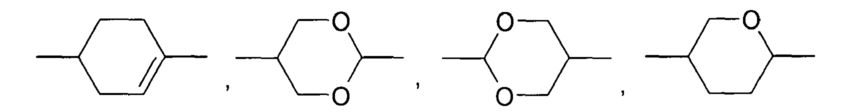 ou

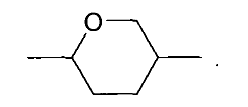

7. Composés selon une ou plusieurs des revendications 1 à 6, **caractérisés en ce que**
R¹ et R² sont chacun, indépendamment l'un de l'autre, un radical alkanyle, un radical alcoxy ou un radical alkényle ayant respectivement 1 à 7 ou 2 à 7 atomes de carbone, dans lesquels chacun de ces radicaux est non substitué ou mono- ou polysubstitué par halogène, ou représente fluor ou hydrogène.

8. Composés selon une ou plusieurs des revendications 1 à 7, **caractérisés en ce que**
m et n sont tous deux zéro, et
R¹ et R² sont chacun, indépendamment l'un de l'autre, un radical alkanyle, radical alcoxy ou radical alkényle non ramifié ayant respectivement 1 à 7 ou 2 à 7 atomes de car- bone.

9. Composés selon une ou plusieurs des revendications 1 à 7, **caractérisés en ce que** m + n = 1, et
A¹, A², indépendamment l'un de l'autre, représentent 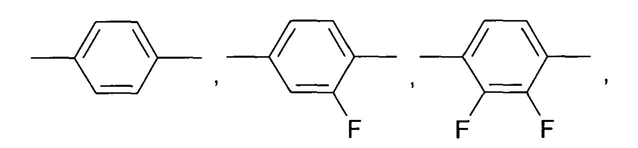 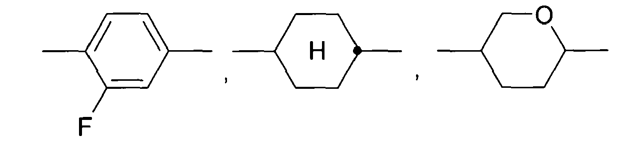
ou

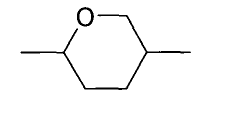

10. Composés selon une ou plusieurs des revendications 1 à 9, **caractérisés en ce que** m = 0, et
R¹ est un radical alcoxy ou un radical alkényloxy ayant 2 à 7 atomes de carbone.

11. Utilisation d'un ou plusieurs composés selon une ou plusieurs des revendications précédentes dans des milieux cristallins liquides.

12. Milieu cristallin liquide comprenant au moins deux composés, **caractérisé en ce qu'**il comprend au moins un composé selon une ou plusieurs des revendications 1 à 10.

13. Elément d'affichage électro-optique contenant un milieu cristallin liquide selon la revendication 12.

14. Procédé pour la préparation de composés selon une ou plusieurs des revendications 1 à 10, **caractérisé en ce qu'**il inclut une étape de procédé dans laquelle un composé 2-(2-halophényl)cyclohexanol approximativement substitué est cyclisé pour donner un dérivé de tétrahydrodibenzofuranne.
